# EUROPEAN PATENT APPLICATION

(11) **EP 3 725 764 A1**
(43) Date of publication of application: **21.10.2020**
(21) Application number: 18888168.4
(22) Date of filing: 05.12.2018
(51) Int. Cl.: C07C 69/734, C09K 19/12, C09K 19/14, C09K 19/20, C09K 19/30, C09K 19/34, C09K 19/54, G02F 1/1337

(54) **POLYMERIZABLE COMPOUND HAVING METHOXYMETHYL ACRYLIC GROUP, LIQUID CRYSTAL COMPOSITION, AND LIQUID CRYSTAL DISPLAY DEVICE**

(30) Priority: 12.12.2017 JP 2017237430
(71) Applicant: JNC Corporation, Chiyoda-ku Tokyo 100-8105 (JP); JNC Petrochemical Corporation, Tokyo 100-0004 (JP)
(72) Inventor: MORI Ayako, Ichihara-shi, Chiba 290-8551 (JP); TANAKA Hiroyuki, Ichihara-shi, Chiba 290-8551 (JP)
(74) Representative: Becker, Eberhard
(86) International application number: PCT/JP2018/044680
(87) International publication number: WO 2019/116979

(57) **Abstract**

The problem is to provide a polymerizable compound having suitable polymerization reactivity, a high conversion ratio, low polymerization starting temperature, and high solubility in a liquid crystal composition; a liquid crystal composition containing the compound; and a liquid crystal display device including the composition.

A means thereof includes a compound represented by formula (1) and a liquid crystal composition containing the compound. 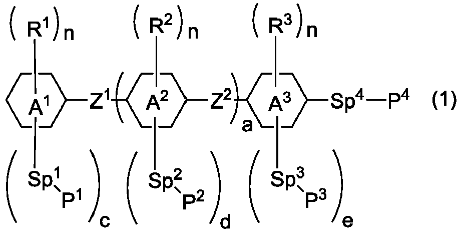 In formula (1), R¹, R² and R³ are independently alkyl or the like; n is 0 or the like; ring A¹ is phenyl or the like, and ring A² and ring A³ are independently 1,4-phenylene or the like; Z¹ and Z² are independently a single bond or the like; Sp¹ to Sp⁴ are a single bond or the like; a is 1 or the like; c, d and e are independently 0 or 1, and a sum of c, d and e is 1 or the like; and P¹ to P⁴ are a polymerizable group, and at least one of P¹ to P⁴ is a polymerizable group represented by formula (P-1).

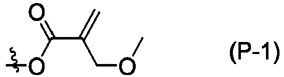

## Description

### Technical Field

The invention relates to a polymerizable compound, a polymerizable composition containing the polymerizable compound and a liquid crystal composition, a liquid crystal composite prepared from the polymerizable composition, and a liquid crystal display device. More specifically, the invention relates to a polymerizable compound having a methoxymethyl acrylic group, a liquid crystal composition that contains the same, and a liquid crystal device that includes the composition.

### Background Art

In a liquid crystal display device, a classification based on an operating mode for liquid crystal molecules includes a phase change (PC) mode, a twisted nematic (TN) mode, a super twisted nematic (STN) mode, an electrically controlled birefringence (ECB) mode, an optically compensated bend (OCB) mode, an in-plane switching (IPS) mode, a vertical alignment (VA) mode, a fringe field switching (FFS) mode and a field-induced photo-reactive alignment (FPA) mode. A classification based on a driving mode in the device includes a passive matrix (PM) and an active matrix (AM). The PM is classified into static, multiplex and so forth, and the AM is classified into a thin film transistor (TFT), a metal insulator metal (MIM) and so forth.

The device is sealed with a liquid crystal composition. Physical properties of the composition relate to characteristics in the device. Specific examples of the physical properties in the composition include stability to heat or light, a temperature range of a nematic phase, viscosity, optical anisotropy, dielectric anisotropy, specific resistance and an elastic constant. The composition is prepared by mixing a great number of liquid crystal compounds. Physical properties required for a compound include high stability to environment such as water, air, heat and light, a wide temperature range of a liquid crystal phase, small viscosity, suitable optical anisotropy, large dielectric anisotropy, a suitable elastic constant and good compatibility with other liquid crystal compounds. A compound having high maximum temperature of the nematic phase is preferred. A compound having low minimum temperature in the liquid crystal phase such as the nematic phase and a smectic phase is preferred. A compound having small viscosity contributes to a short response time in the device. A suitable value of the optical anisotropy depends on a type of the operating mode in a device. A compound having large positive or negative dielectric anisotropy is preferred for driving the device at low voltage. A compound having good compatibility with other liquid crystal compounds is preferred for preparing the composition. The device may be occasionally used at a temperature below freezing point, and therefore a compound having good compatibility at low temperature is preferred.

A liquid crystal display device having a mode in which a polymer is combined with a liquid crystal composition is known. The mode is, for example, a polymer sustained alignment (PSA) mode or a polymer stabilized (PS) mode. In the liquid crystal display device having the mode, a liquid crystal composition to which a polymerizable compound is added is injected into the display device. The polymerizable compound is polymerized by irradiation with ultraviolet light while voltage is applied between electrodes, and thus the polymer is formed in the liquid crystal composition. A liquid crystal display device in which a response time is shortened and image persistence is improved can be obtained by the method.

The method can be applied to a liquid crystal display device having various operating modes, and a PS-TN mode, a PS-IPS mode, a PS-FFS mode, a PSA-VA mode, a PSA-OCB mode and so forth are known. A polymerizable compound used in a device having such a mode is considered to have high capability of aligning liquid crystal molecules, but does not necessarily have high solubility in the liquid crystal composition. Attempt to improve solubility in the liquid crystal composition has been performed so far, but polymerization reactivity tends to be decreased as the solubility is improved. Accordingly, development of a polymerizable compound having a suitable balance between the solubility and the polymerization reactivity has been desired.

A large number of polymerizable compounds have been prepared so far. Development of a new polymerizable compound has been still continued. The reason is that a new compound is expected to have good physical properties that are not found in conventional compounds. The reason is that the new compound may occasionally give a suitable balance to at least two of physical properties of the composition.

On page 40 of Patent literature No. 1, compound (A) is described:

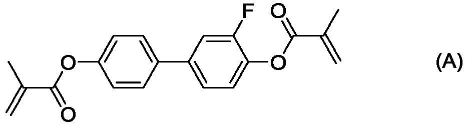

On page 119 of Patent literature No. 2, compound (B) is described:

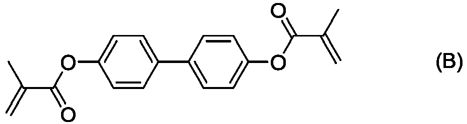

### Citation List

### Patent Literature

Patent literature No. 1: WO 2012/086504 A.
Patent literature No. 2: WO 2011/160765 A.

### Summary of Invention

### Technical Problem

A first object of the invention is to provide a polymerizable compound having suitable polymerization reactivity, a high conversion ratio, low polymerization starting temperature, and a high solubility in a liquid crystal composition. A second object is to provide a liquid crystal composite satisfying at least one of physical properties such as high maximum temperature of a nematic phase, low minimum temperature of the nematic phase, small viscosity, suitable optical anisotropy, large dielectric anisotropy, a suitable elastic constant, large specific resistance and a suitable pretilt. The object is to provide a liquid crystal composite having a suitable balance regarding at least two of the physical properties. A third object is to provide a liquid crystal display device has a wide temperature range in which the device can be used, a short response time, a high voltage holding ratio, a low threshold voltage, a large contrast ratio and a long service life.

### Solution to Problem

The invention concerns a compound represented by formula (1), a liquid crystal composition containing the compound, and a liquid crystal display device including the composition: 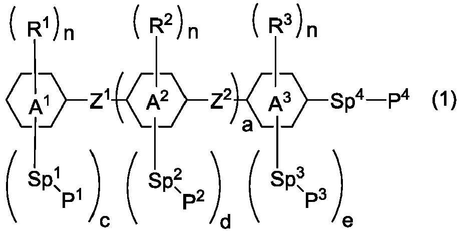 wherein, in formula (1),
R¹, R² and R³ are independently alkyl having 1 to 10 carbons, and in the alkyl, at least one piece of -CH₂- may be replaced by -O- or -NH-;
n is independently 0, 1, 2, 3 or 4;
ring A¹ is independently cyclohexyl, cyclohexenyl, phenyl, 1-naphthyl, 2-naphthyl, tetrahydropyran-2-yl, tetrahydropyran-3-yl, 1,3-dioxane-2-yl, 1,3-dioxane-3-yl, pyrimidine-2-yl, pyrimidine-5-yl, pyridine-2-yl or pyridine-3-yl, ring A² and ring A³ are independently 1,4-cyclohexylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, tetrahydropyran-2,5-diyl, 1,3-dioxane-2,5-diyl, pyrimidine-2,5-diyl or pyridine-2,5-diyl, and
in the rings, at least one hydrogen may be replaced by fluorine or chlorine;
Z¹ and Z² are independently a single bond or alkylene having 1 to 10 carbons, and in the alkylene, at least one piece of -CH₂- may be replaced by -O-, -COO-, -OCO- or -OCOO-, and at least one piece of -(CH₂)₂- may be replaced by -CH=CH- or -C≡C-, and in the groups, at least one hydrogen may be replaced by fluorine or chlorine;
Sp¹, Sp², Sp³ and Sp⁴ are independently a single bond or alkylene having 1 to 10 carbons, and in the alkylene, at least one piece of -CH₂-may be replaced by -O-, -COO-, -OCO- or -OCOO-, and at least one piece of -(CH₂)₂- may be replaced by -CH=CH- or -C≡C-, and in the groups, at least one hydrogen may be replaced by fluorine or chlorine;
a is 0, 1, 2, 3 or 4;
c, d and e are independently 0, 1, 2, 3 or 4, and a sum of c, d and e is 1, 2, 3 or 4; and
P¹, P², P³ and P⁴ are a polymerizable group, and at least one of P¹, P², P³ and P⁴ is a polymerizable group represented by formula (P-1).

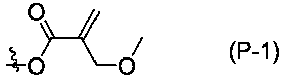

### Advantageous Effects of Invention

A first object of the invention is a polymerizable compound that has high polymerization reactivity, a high conversion ratio, low polymerization starting temperature, and a high solubility in a liquid crystal composition. A second advantage is a liquid crystal composite satisfies at least one of physical properties such as high maximum temperature of a nematic phase, low minimum temperature of the nematic phase, small viscosity, suitable optical anisotropy, large dielectric anisotropy, a suitable elastic constant, large specific resistance and a suitable pretilt. The object is a liquid crystal composite that has a suitable balance regarding at least two of the physical properties. A third object is a liquid crystal display device that has a wide temperature range in which the device can be used, a short response time, a high voltage holding ratio, a low threshold voltage, a large contrast ratio and a long service life.

### Description of Embodiments

Usage of terms herein is as described below. Terms "liquid crystal compound," "liquid crystal composition" and "liquid crystal display device" may be occasionally abbreviated as "compound," "composition" and "device," respectively. "Liquid crystal compound" is a generic term for a compound having a liquid crystal phase such as a nematic phase and a smectic phase, and a compound having no liquid crystal phase but to be added for the purpose of adjusting physical properties of a composition, such as maximum temperature, minimum temperature, viscosity and dielectric anisotropy. The compound has a six-membered ring such as 1,4-cyclohexylene and 1,4-phenylene, and has rod-like molecular structure. "Liquid crystal display device" is a generic term for a liquid crystal display panel and a liquid crystal display module. "Polymerizable compound" is a compound to be added for the purpose of forming a polymer in the composition. "Polymerizable composition" is a mixture of the polymerizable compound, the liquid crystal composition, an additive or the like. "Liquid crystal composite" is a composite formed by polymerization of the polymerizable composition.

The liquid crystal composition is prepared by mixing a plurality of liquid crystal compounds. An additive is added to the composition for the purpose of further adjusting the physical properties. The additive such as the polymerizable compound, a polymerization initiator, a polymerization inhibitor, an optically active compound, an antioxidant, an ultraviolet light absorber, a light stabilizer, a heat stabilizer, a dye and an antifoaming agent is added thereto when necessary. The liquid crystal compound and the additive are mixed in such a procedure. A proportion (content) of the liquid crystal compound is expressed in terms of weight percent (% by weight) based on the weight of the liquid crystal composition containing no additive, even after the additive has been added. A proportion (amount of addition) of the additive is expressed in terms of weight percent (% by weight) based on the weight of the liquid crystal composition containing no additive. Weight parts per million (ppm) may be occasionally used. A proportion of the polymerization initiator and the polymerization inhibitor is exceptionally expressed based on the weight of the polymerizable compound.

"Clearing point" is a transition temperature between the liquid crystal phase and an isotropic phase in the liquid crystal compound. "Minimum temperature of the liquid crystal phase" is a transition temperature between a solid and the liquid crystal phase (the smectic phase, the nematic phase or the like) in the liquid crystal compound. "Maximum temperature of the nematic phase" is a transition temperature between the nematic phase and the isotropic phase in a mixture of the liquid crystal compound and a base liquid crystal or in the liquid crystal composition, and may be occasionally abbreviated as "maximum temperature." "Minimum temperature of the nematic phase" may be occasionally abbreviated as "minimum temperature." An expression "increase the dielectric anisotropy" means that a value of dielectric anisotropy positively increases in a composition having positive dielectric anisotropy, and the value of dielectric anisotropy negatively increases in a composition having negative dielectric anisotropy. An expression "having a large voltage holding ratio" means that the device has a large voltage holding ratio at room temperature and also at a temperature close to the maximum temperature in an initial stage, and the device has the large voltage holding ratio at room temperature and also at a temperature close to the maximum temperature even after the device has been used for a long period of time. In the composition or the device, the characteristics may be occasionally examined before and after an aging test (including an acceleration deterioration test).

A compound represented by formula (1) may be occasionally abbreviated as compound (1). At least one compound selected from the group of compounds represented by formula (1) may be occasionally abbreviated as compound (1). "Compound (1)" means one compound, a mixture of two compounds or a mixture of three or more compounds represented by formula (1). A same rule applies also to any other compound represented by any other formula. In formulas (1) to (15), a symbol of A¹, B¹, C¹ or the like surrounded by a hexagonal shape corresponds to ring A¹, ring B¹ and ring C¹, respectively. The hexagonal shape represents a six-membered ring such as cyclohexane or benzene. The hexagonal shape may occasionally represent a fused ring such as naphthalene or a bridged ring such as adamantane.

A compound represented by formula (1) means that -Sp⁴-P⁴ is replaced to a para position with respect to bonding group Z². In the compound, when a is 0, a same rule applies also to bonding group Z¹.

A symbol of terminal group R¹¹ is used in a plurality of compounds in chemical formulas of component compounds. In the compounds, two groups represented by two pieces of arbitrary R¹¹ may be identical or different. For example, in one case, R¹¹ of compound (2) is ethyl and R¹¹ of compound (3) is ethyl. In another case, R¹¹ of compound (2) is ethyl and R¹¹ of compound (3) is propyl. A same rule applies also to a symbol of R¹², R¹³, Z¹¹ or the like. In compound (8), when i is 2, two of ring D¹ exists. In the compound, two groups represented by two of ring D¹ may be identical or different. A same rule applies also to two of arbitrary ring D¹ when i is larger than 2. A same rule applies also to other symbols.

An expression "at least one piece of 'A'" means that the number of 'A' is arbitrary. An expression "at least one piece of 'A' may be replaced by 'B'" means that, when the number of 'A' is 1, a position of 'A' is arbitrary, and also when the number of 'A' is 2 or more, positions thereof can be selected without restriction. A same rule applies also to an expression "at least one piece of 'A' is replaced by 'B'." An expression "at least one piece of 'A' may be replaced by 'B', 'C' or 'D'" includes a case where arbitrary 'A' is replaced by 'B', a case where arbitrary 'A' is replaced by 'C', and a case where arbitrary 'A' is replaced by 'D', and also a case where a plurality of pieces of 'A' are replaced by at least two pieces of 'B', 'C' and/or 'D.' For example, "alkyl in which at least one piece of -CH₂- may be replaced by -O- or -CH=CH-" includes alkyl, alkoxy, alkoxyalkyl, alkenyl, alkoxyalkenyl and alkenyloxyalkyl. In addition, a case where replacement of two consecutive pieces of -CH₂- by -O- results in forming -O-O- is not preferred. In the alkyl or the like, a case where replacement of -CH₂- of a methyl part (-CH₂-H) by -O- results in forming -O-H is not preferred, either.

An expression "R¹¹ and R¹² are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine" may be occasionally used. In the expression, "in the groups" may be interpreted according to wording. In the expression, "the groups" means alkyl, alkenyl, alkoxy, alkenyloxy or the like. More specifically, "the groups" represents all of the groups described before the term "in the groups." The common interpretation is applied also to terms of "in the monovalent groups" or "in the divalent groups." For example, "the monovalent groups" represents all of the groups described before the term "in the monovalent groups."

Halogen means fluorine, chlorine, bromine and iodine. Preferred halogen is fluorine and chlorine. Further preferred halogen is fluorine. Alkyl of a liquid crystal compound has a straight-chain or a branched-chain. In general, straight-chain alkyl is preferred to branched-chain alkyl. A same rule applies also to a terminal group such as alkoxy and alkenyl. With regard to a configuration of 1,4-cyclohexylene, trans is preferred to cis for increasing the maximum temperature. Then, 2-fluoro-1,4-phenylene means two divalent groups described below. In a chemical formula, fluorine may be leftward (L) or rightward (R). A same rule applies also to an asymmetrical divalent group formed by removing two hydrogens from a ring, such as tetrahydropyran-2,5-diyl.

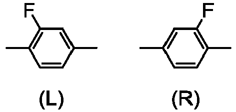

The invention includes items described below.
Item 1. A compound, represented by formula (1): 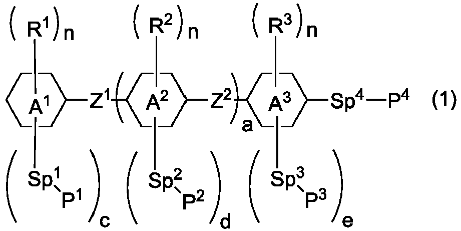 wherein, in formula (1),
   R¹, R² and R³ are independently alkyl having 1 to 10 carbons, and in the alkyl, at least one piece of -CH₂- may be replaced by -O- or -NH-;
   n is independently 0, 1, 2, 3 or 4;
   ring A¹ is cyclohexyl, cyclohexenyl, phenyl, 1-naphthyl, 2-naphthyl, tetrahydropyran-2-yl, tetrahydropyran-3-yl, 1,3-dioxane-2-yl, 1,3-dioxane-3-yl, pyrimidine-2-yl, pyrimidine-5-yl, pyridine-2-yl or pyridine-3-yl, ring A² and ring A³ are independently 1,4-cyclohexylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, tetrahydropyran-2,5-diyl, 1,3-dioxane-2,5-diyl, pyrimidine-2,5-diyl or pyridine-2,5-diyl, and
   in the rings, at least one hydrogen may be replaced by fluorine or chlorine;
   Z¹ and Z² are independently a single bond or alkylene having 1 to 10 carbons, and in the alkylene, at least one piece of -CH₂- may be replaced by -O-, -COO-, -OCO- or -OCOO-, and at least one piece of -(CH₂)₂- may be replaced by -CH=CH- or -C≡C-, and in the groups, at least one hydrogen may be replaced by fluorine or chlorine;
   Sp¹, Sp², Sp³ and Sp⁴ are independently a single bond or alkylene having 1 to 10 carbons, and in the alkylene, at least one piece of -CH₂-may be replaced by -O-, -COO-, -OCO- or -OCOO-, and at least one piece of -(CH₂)₂- may be replaced by -CH=CH- or -C≡C-, and in the groups, at least one hydrogen may be replaced by fluorine or chlorine;
   a is 0, 1, 2, 3 or 4;
   c, d and e are independently 0, 1, 2, 3 or 4, and a sum of c, d and e is 1, 2, 3 or 4; and
   P¹, P², P³ and P⁴ are a polymerizable group, and at least one of P¹, P², P³ and P⁴ is a polymerizable group represented by formula (P-1). 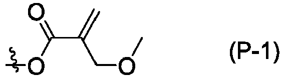
Item 2. The compound according to item 1,
   wherein, in formula (1),
   ring A¹ is cyclohexyl, cyclohexenyl, phenyl, 1-naphthyl, 2-naphthyl, tetrahydropyran-2-yl, tetrahydropyran-3-yl, 1,3-dioxane-2-yl or 1,3-dioxane-3-yl, ring A² and ring A³ are independently 1,4-cyclohexylene, 1,4-cyclohexenylene, 1,4-phenylene, naphthalene-1,5-diyl, naphthalene-2,6-diyl, tetrahydropyran-2,5-diyl or 1,3-dioxane-2,5-diyl, and
   in the rings, at least one hydrogen may be replaced by fluorine or chlorine.
Item 3. The compound according to item 1,
   wherein, in formula (1),
   Sp¹, Sp², Sp³ and Sp⁴ are independently a single bond or alkylene having 1 to 7 carbons, and in the alkylene, at least one piece of -CH₂-may be replaced by -O-, and at least one piece of - (CH₂)₂- may be replaced by -CH=CH-, and in the groups, at least one hydrogen may be replaced by fluorine or chlorine.
Item 4. The compound according to any one of items 1 to 3, represented by any one of formula (1-1) to formula (1-3): 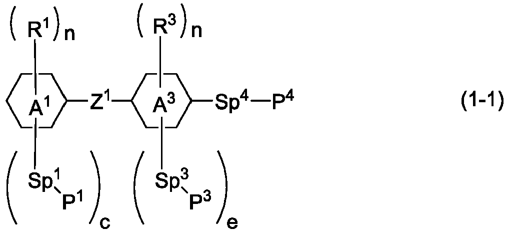 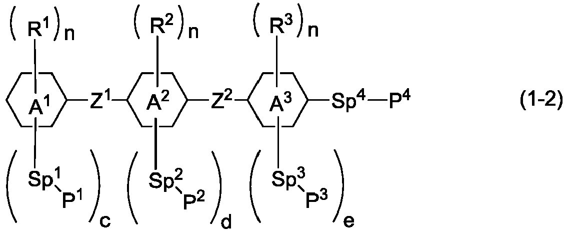 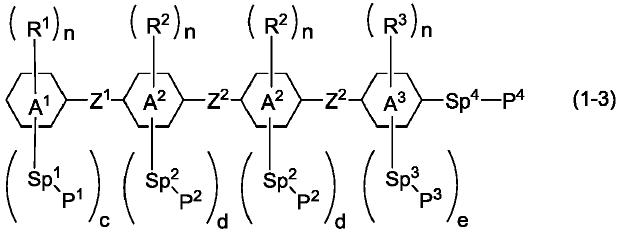 wherein, in formula (1-1) to formula (1-3),
   R¹, R² and R³ are independently alkyl having 1 to 7 carbons, and in the alkyl, at least one piece of -CH₂- may be replaced by -O-;
   n is independently 0, 1, 2, 3 or 4;
   ring A¹ is cyclohexyl, cyclohexenyl, phenyl, 1-naphthyl, 2-naphthyl, tetrahydropyran-2-yl, tetrahydropyran-3-yl, 1,3-dioxane-2-yl or 1,3-dioxane-3-yl, ring A² and ring A³ are independently 1,4-cyclohexylene, 1,4-cyclohexenylene, 1,4-phenylene, naphthalene-1,5-diyl, naphthalene-2,6-diyl, tetrahydropyran-2,5-diyl or 1,3-dioxane-2,5-diyl, and
   in the rings, at least one hydrogen may be replaced by fluorine or chlorine;
   Z¹ and Z² are independently a single bond, alkylene having 1 to 4 carbons, -CH₂O-, -OCH₂-, -COO-, -OCO-, -CH₂CH₂-, -CH=CH-, -C≡C-, -CH=CH-COO-, -OCO-CH=CH-, -CO-CH=CH- or -CH=CH-CO-;
   Sp¹, Sp², Sp³ and Sp⁴ are independently a single bond or alkylene having 1 to 7 carbons, and in the alkylene, at least one piece of -CH₂-may be replaced by -O-, and at least one piece of - (CH₂)₂- may be replaced by -CH=CH-, and in the groups, at least one hydrogen may be replaced by fluorine or chlorine;
   c, d and e are independently 0, 1, 2, 3 or 4, and a sum of c, d and e is 1, 2, 3 or 4; and
   P¹, P², P³ and P⁴ are independently a group selected from the group of polymerizable groups represented by formula (P-1) to formula (P-6), and at least one of P¹, P², P³ and P⁴ is a polymerizable group represented by formula (P-1); 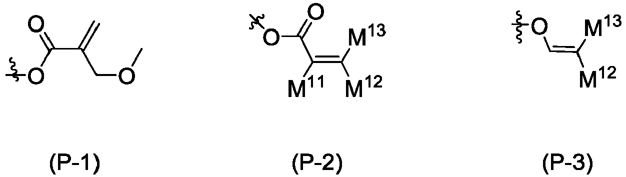 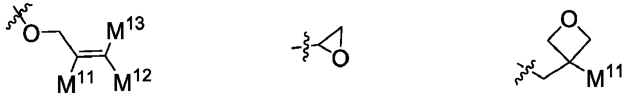 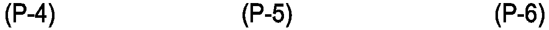 wherein, in formula (P-2) to formula (P-6), M¹¹, M¹² and M¹³ are independently hydrogen, fluorine, alkyl having 1 to 5 carbons, or alkyl having 1 to 5 carbons in which at least one hydrogen is replaced by halogen.
Item 5. The compound according to any one of items 1 to 4, wherein, in formula (1-1) to formula (1-3),
   ring A¹ is cyclohexyl or phenyl, ring A² and ring A³ are independently 1,4-cyclohexylene or 1,4-phenylene, and
   in the rings, at least one hydrogen may be replaced by fluorine or chlorine. 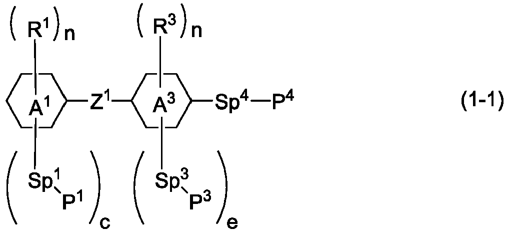 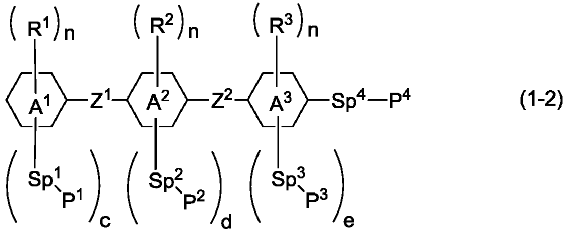 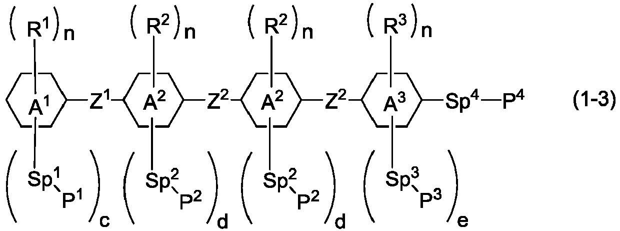
Item 6. The compound according to any one of items 1 to 5, represented by any one of formula (1-4) to formula (1-12): 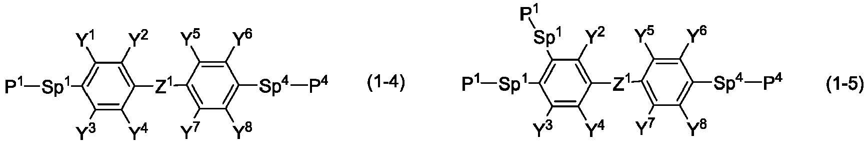 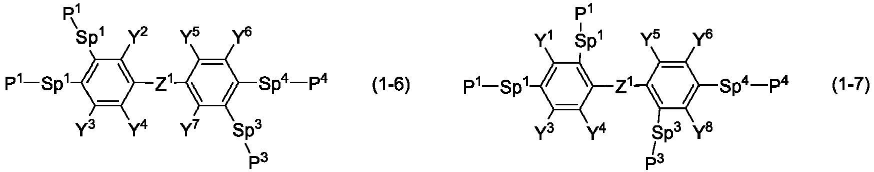 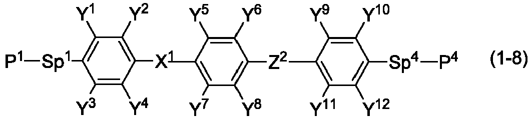 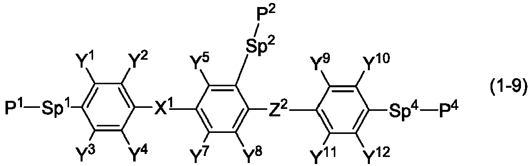 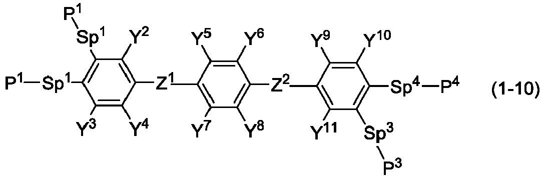 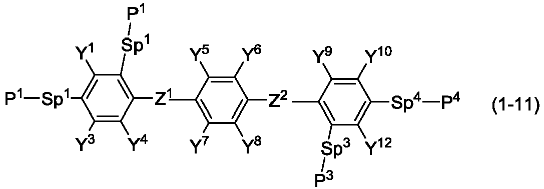 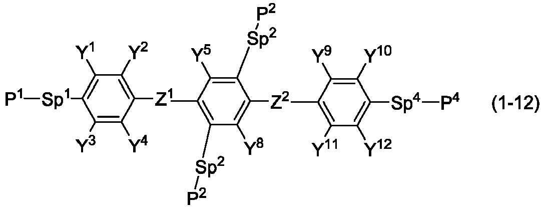 wherein, in formula (1-4) to formula (1-12),
   Y¹, Y², Y³, Y⁴, Y⁵, Y⁶, Y⁷, Y⁸, Y⁹, Y¹⁰, Y¹¹ and Y¹² are independently hydrogen, fluorine, methyl, ethyl, propyl, butyl, methoxy, ethoxy, propyloxy or butoxy;
   Sp¹, Sp², Sp³ and Sp⁴ are independently a single bond or alkylene having 1 to 5 carbons, and in the alkylene, at least one piece of -CH₂-may be replaced by -O-, and at least one piece of - (CH₂)₂- may be replaced by -CH=CH-;
   Z¹ and Z² are independently a single bond, alkylene having 1 to 4 carbons, -CH₂O-, -OCH₂-, -COO-, -OCO-, -CH₂CH₂-, -CH=CH-, -CH=CH-COO-or -OCO-CH=CH-; and
   P¹, P², P³ and P⁴ are independently a group selected from the group of polymerizable groups represented by formula (P-1) to formula (P-4), and at least one of P¹, P², P³ and P⁴ is a polymerizable group represented by formula (P-1); 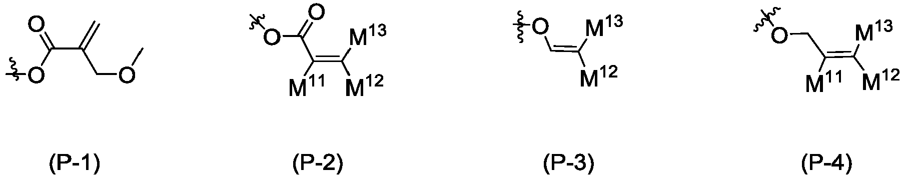 wherein, in formula (P-2) to formula (P-4), M¹¹, M¹² and M¹³ are independently hydrogen, fluorine, alkyl having 1 to 5 carbons, or alkyl having 1 to 5 carbons in which at least one hydrogen is replaced by halogen.
Item 7. The compound according to any one of items 1 to 6, represented by any one of formula (1-13) to formula (1-24): 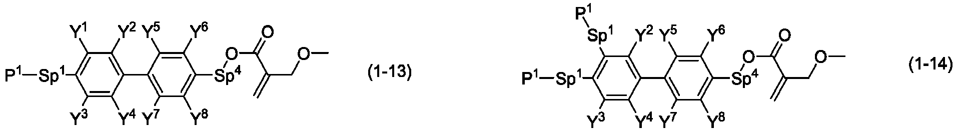 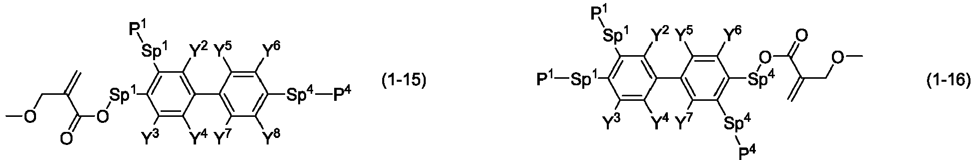 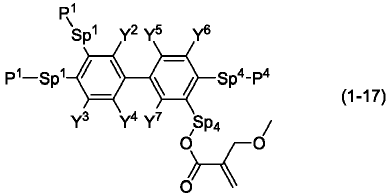 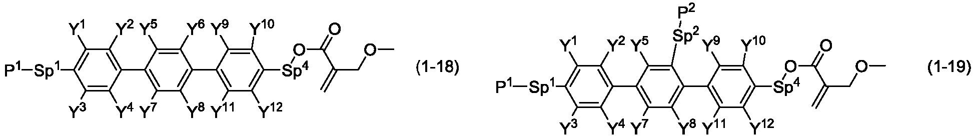 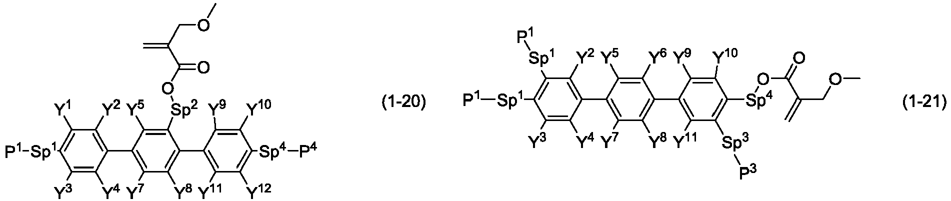 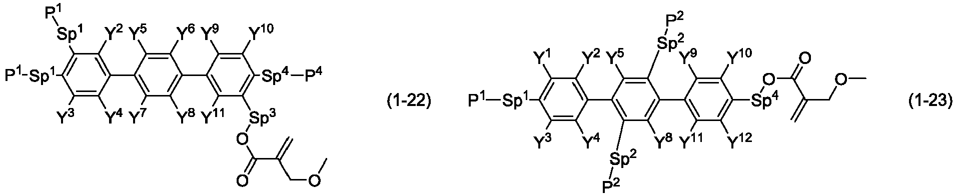 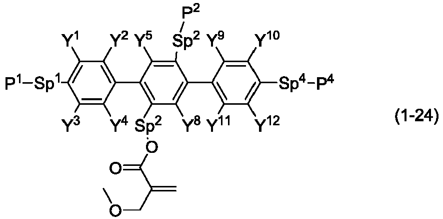 wherein, in formula (1-13) to formula (1-24),
   Y¹, Y², Y³, Y⁴, Y⁵, Y⁶, Y⁷, Y⁸, Y⁹, Y¹⁰, Y¹¹ and Y¹² are independently hydrogen, fluorine, methyl, ethyl, propyl, butyl, methoxy, ethoxy, propyloxy or butoxy;
   Sp¹, Sp², Sp³ and Sp⁴ are independently a single bond or alkylene having 1 to 5 carbons, and in the alkylene, at least one piece of -CH₂-may be replaced by -O-, and at least one piece of - (CH₂)₂- may be replaced by -CH=CH-; and
   P¹, P², P³ and P⁴ are independently acryloyloxy, methacryloyloxy, or a polymerizable group represented by formula (P-1). 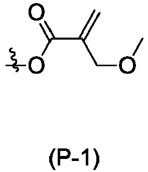
Item 8. A liquid crystal composition, containing at least one compound according to any one of items 1 to 7 as component A.
Item 9. The liquid crystal composition according to item 8, further containing at least one compound selected from the group of compounds represented by formulas (2) to (4) as component B: 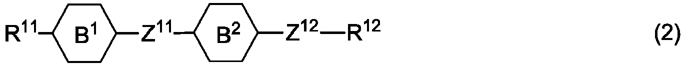 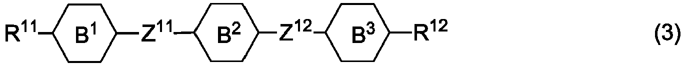 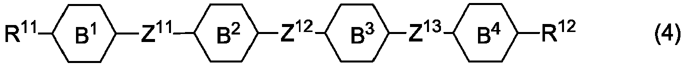 wherein, in formulas (2) to (4),
   R¹¹ and R¹² are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
   ring B¹, ring B², ring B³ and ring B⁴ are independently 1,4-cyclohexylene, 1,4-phenylene, 2-fluoro-1,4-phenylene, 2,5-difluoro-1,4-phenylene or pyrimidine-2,5-diyl; and
   Z¹¹, Z¹² and Z¹³ are independently a single bond, -COO-, -CH₂CH₂-, -CH=CH- or -C≡C-.
Item 10. The liquid crystal composition according to item 8 or 9, further containing at least one compound selected from the group of compounds represented by formulas (5) to (7) as component C: 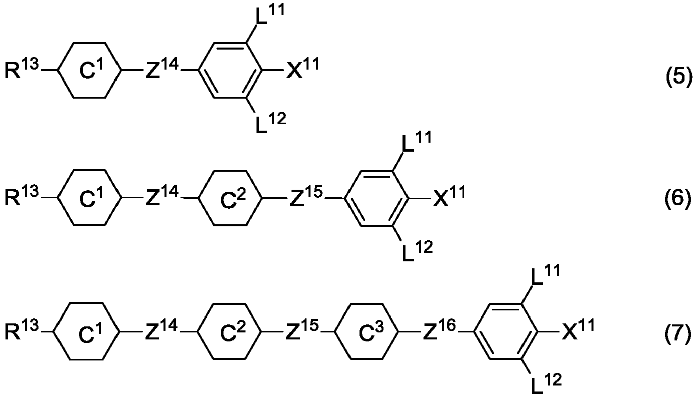 wherein, in formulas (5) to (7),
   R¹³ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
   X¹¹ is fluorine, chlorine, -OCF₃, -OCHF₂, -CF₃, -CHF₂, -CH₂F, -OCF₂CHF₂ or -OCF₂CHFCF₃;
   ring C¹, ring C² and ring C³ are independently 1, 4-cyclohexylene, 1,4-phenylene, 1,4-phenylene in which at least one hydrogen is replaced by fluorine, tetrahydropyran-2,5-diyl, 1,3-dioxane-2,5-diyl or pyrimidine-2,5-diyl;
   Z¹⁴, Z¹⁵ and Z¹⁶ are independently a single bond, -COO-, -OCO-, -CH₂O-, -OCH₂-, -CH₂CH₂-, -CH=CH-, -C≡C- or -(CH₂)₄-; and
   L¹¹ and L¹² are independently hydrogen or fluorine.
Item 11. The liquid crystal composition according to any one of items 8 to 10, further containing at least one compound selected from the group of compounds represented by formula (8) as component D: 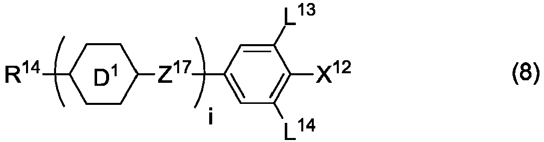 wherein, in formula (8),
   R¹⁴ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
   X¹² is -C≡N or -C≡C-C≡N;
   ring D¹ is 1,4-cyclohexylene, 1,4-phenylene, 1,4-phenylene in which at least one hydrogen is replaced by fluorine, tetrahydropyran-2,5-diyl, 1,3-dioxane-2,5-diyl or pyrimidine-2,5-diyl;
   Z¹⁷ is a single bond, -COO-, -OCO-, -CH₂O-, -OCH₂-, -CH₂CH₂- or -C≡C-;
   L¹³ and L¹⁴ are independently hydrogen or fluorine; and
   i is 1, 2, 3 or 4.
Item 12. The liquid crystal composition according to any one of items 8 to 11, further containing at least one compound selected from the group of polymerizable compounds represented by formula (16) as component F: 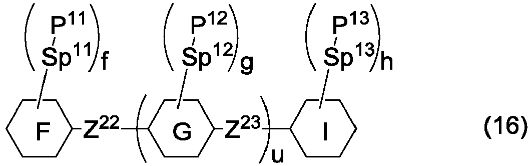 wherein, in formula (16),
   ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
   ring G is 1,4-cyclohexylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, phenanthrene-2,7-diyl, tetrahydropyran-2,5-diyl, 1,3-dioxane-2,5-diyl, pyrimidine-2,5-diyl or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
   Z²² and Z²³ are independently a single bond or alkylene having 1 to 10 carbons, and in the alkylene, at least one piece of -CH₂- may be replaced by -O-, -CO-, -COO- or -OCO-, and at least one piece of -CH₂CH₂- may be replaced by -CH=CH-, -C(CH₃)=CH-, -CH=C(CH₃)- or -C(CH₃)=C(CH₃)-, and in the groups, at least one hydrogen may be replaced by fluorine or chlorine;
   Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine;
   u is 0, 1 or 2;
   f, g and h are independently 0, 1, 2, 3 or 4, and a sum of f, g and h is 1 or more; and
   P¹¹, P¹² and P¹³ are independently a group selected from the group of polymerizable groups represented by formula (P-2) to formula (P-6) ; 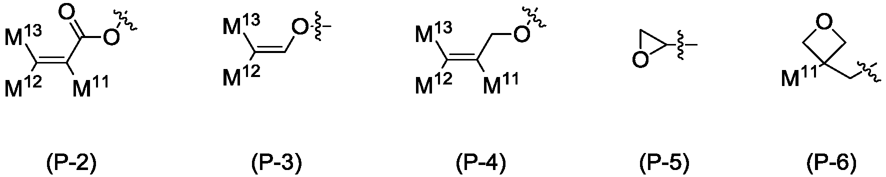 wherein, in formula (P-2) to formula (P-6), M¹¹, M¹² and M¹³ are independently hydrogen, fluorine, alkyl having 1 to 5 carbons, or alkyl having 1 to 5 carbons in which at least one hydrogen is replaced by halogen.
Item 13. The liquid crystal composition according to item 12, wherein component F is at least one compound selected from the group of polymerizable compounds represented by formula (16-1) to formula (16-7) : 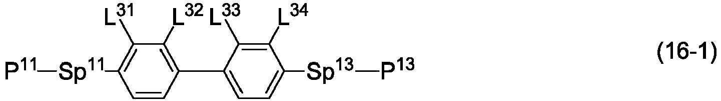 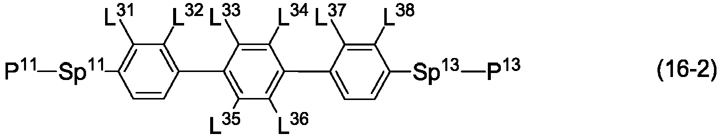 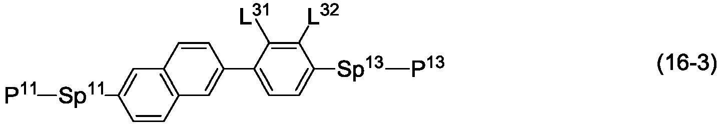 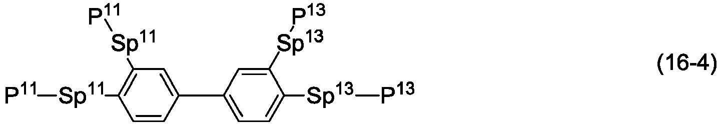 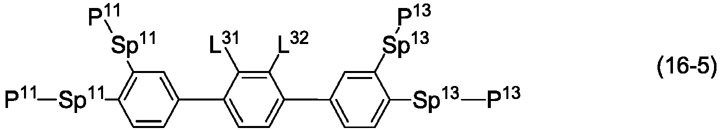 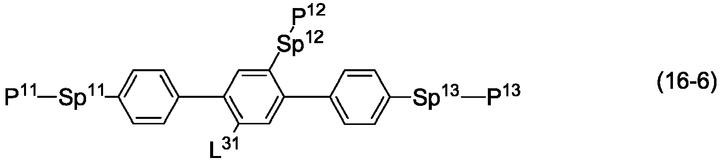 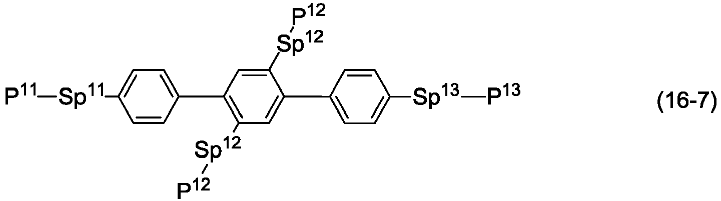 wherein, in formula (16-1) to formula (16-7), P¹¹, P¹² and P¹³ are independently a group selected from the group of polymerizable groups represented by formula (P-2) to formula (P-4), in which M¹¹, M¹² and M¹³ are independently hydrogen, fluorine, alkyl having 1 to 5 carbons, or alkyl having 1 to 5 carbons in which at least one hydrogen is replaced by halogen; 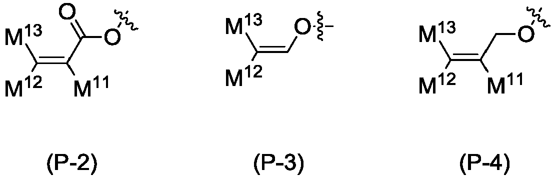 wherein, L³¹, L³², L³³, L³⁴, L³⁵, L³⁶, L³⁷ and L³⁸ are independently hydrogen, fluorine or methyl; and Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine.
Item 14. The liquid crystal composition according to any one of items 8 to 13, further containing at least one of polymerizable compounds different from the compounds represented by formula (1) and formula (16), a polymerization initiator, a polymerization inhibitor, an optically active compound, an antioxidant, an ultraviolet light absorber, a light stabilizer, a heat stabilizer and an antifoaming agent.
Item 15. A liquid crystal display device, including at least one liquid crystal composition according to any one of items 8 to 14.

The invention further includes the following items: (a) the liquid crystal composition, further containing at least two of additives such as a polymerizable compound, a polymerization initiator, a polymerization inhibitor, an optically active compound, an antioxidant, an ultraviolet light absorber, a light stabilizer, a heat stabilizer and an antifoaming agent; (b) a polymerizable composition prepared by adding a polymerizable compound different from compound (1) or compound (16) to the liquid crystal composition; (c) a polymerizable composition prepared by adding compound (1) and compound (16) to the liquid crystal composition; (d) a liquid crystal composite prepared by polymerizing the polymerizable composition; (e) a polymer sustained alignment mode device including the liquid crystal composite; and (f) a device that has a polymer sustained alignment mode, and is prepared by using a polymerizable composition prepared by adding compound (1), compound (16) and a polymerizable compound different from compound (1) or compound (16) to the liquid crystal composition.

An aspect of compound (1), a synthetic method of compound (1), the liquid crystal composition and the liquid crystal display device will be described in the order.

### 1. Aspect of compound (1)

Compound (1) of the invention has a structure of a mesogen moiety which is formed of at least one ring and a methoxymethyl acrylic group. Compound (1) has a feature of having low polymerization starting temperature in comparison with a similar compound (see Comparative Example 1). Preferred examples of compound (1) will be described. Preferred examples of a symbol of R¹, Z¹, Sp¹, P¹, A¹, n, a or the like in compound (1) are applied also to a subordinate formula of formula (1) for compound (1). In compound (1), physical properties can be arbitrarily adjusted by suitably combining the groups. Compound (1) may contain a larger amount of isotope such as ²H (deuterium) and ¹³C than the amount of natural abundance because no significant difference exists in the physical properties of the compound. In addition, symbols in compound (1) are defined according to item 1.

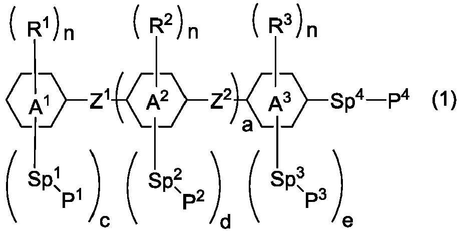

In formula (1), R¹, R² and R³ are independently alkyl having 1 to 10 carbons, and in the alkyl, at least one piece of -CH₂- may be replaced by -O- or -NH-.

Examples of R¹ to R³ include alkyl, alkoxy, alkoxyalkyl and alkoxyalkoxy. Specific examples of preferred R¹ to R³ include alkyl, alkoxy, alkoxyalkyl or alkoxyalkoxy. Specific examples of further preferred R¹ to R³ include alkyl, alkoxy or alkoxyalkyl. Specific examples of particularly preferred R¹ to R³ include alkyl or alkoxy. Specific examples of most preferred R¹ to R³ include methyl, ethyl, propyl, butyl, methoxy, ethoxy, propyloxy or butoxy.

Preferred alkyl is -CH₃, -C₂H₅, -C₃H₇, -C₄H₉, -C₅H₁₁, -C₆H₁₃ or -C₇H₁₅.

Preferred alkoxy is -OCH₃, -OC₂H₅, -OC₃H₇, -OC₄H₉, -OC₅H₁₁, -OC₆H₁₃ or -OC₇H₁₅.

Then, n is independently 0, 1, 2, 3 or 4, and preferred n is 0, 1 or 2. Particularly preferred n is 0 or 1.

Preferred alkoxyalkyl is -CH₂OCH₃, -CH₂OC₂H₅, -CH₂OC₃H₇, - (CH₂)₂-OCH₃, - (CH₂)₂-OC₂H₅, - (CH₂)₂-OC₃H₇, - (CH₂)₃-OCH₃, - (CH₂)₄-OCH₃ or - (CH₂)₅-OCH₃.

In formula (1), ring A¹ is cyclohexyl, cyclohexenyl, phenyl, 1-naphthyl, 2-naphthyl, tetrahydropyran-2-yl, tetrahydropyran-3-yl, 1,3-dioxane-2-yl, 1,3-dioxane-3-yl, pyrimidine-2-yl, pyrimidine-5-yl, pyridine-2-yl or pyridine-3-yl, ring A² and ring A³ are independently 1,4-cyclohexylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, tetrahydropyran-2,5-diyl, 1,3-dioxane-2,5-diyl, pyrimidine-2,5-diyl or pyridine-2,5-diyl, and in the rings, at least one hydrogen may be replaced by fluorine or chlorine.

Preferred ring A¹ is cyclohexyl, cyclohexenyl, phenyl, 1-naphthyl, 2-naphthyl, tetrahydropyran-2-yl, tetrahydropyran-3-yl, 1,3-dioxane-2-yl or 1,3-dioxane-3-yl, preferred ring A² and ring A³ are independently 1,4-cyclohexylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, tetrahydropyran-2,5-diyl or 1,3-dioxane-2,5-diyl, and in the rings, at least one hydrogen may be replaced by fluorine.

Further preferred ring A¹ is cyclohexyl, phenyl, 1-naphthyl, 2-naphthyl or fluorophenyl, and further preferred ring A² and ring A³ are independently 1,4-cyclohexylene, 1,4-phenylene, 2-fluoro-1,4-phenylene, or 2,3-difluoro-1,4-phenylene, naphthalene-1,5-diyl, or naphthalene-2,6-diyl.

When at least one of ring A² or ring A³ is 1, 4-cyclohexylene or ring A¹ is cyclohexyl, compound (1) has a high clearing point and small viscosity. When at least one of ring A² or ring A³ is 1,4-phenylene or ring A¹ is phenyl, compound (1) has large optical anisotropy and comparatively large reactivity. When at least one of ring A² or ring A³ is 2-fluoro-1,4-phenylene or 2,3-difluoro-1,4-phenylene, or ring A¹ is fluorophenyl, compound (1) has large optical anisotropy, a low polymerization starting temperature, and good compatibility to liquid crystal compound.

In formula (1), Z¹ and Z² are independently a single bond or alkylene having 1 to 10 carbons, and in the alkylene, at least one piece of -CH₂-may be replaced by -O-, -COO-, -OCO- or -OCOO-, and at least one piece of -(CH₂)₂- may be replaced by -CH=CH- or -C≡C-, and in the groups, at least one hydrogen may be replaced by fluorine or chlorine.

Preferred Z¹ and Z² are a single bond, alkylene having 1 to 4 carbons, -COO-, -OCH₂-, -CF₂O-, -CH₂CH₂-, -C=C-, -C≡C-, -CH=CH=COO-, -OCO-CH=CH-, -CO-CH=CH- or -CH=CH-CO-. Particularly preferred Z¹ and Z² are a single bond, -COO-, -CH₂CH₂-, -C-C- or -C≡C-. Most preferred Z¹ and Z² are a single bond.

When Z¹ or Z² is a single bond, compound (1) has high chemical stability. When Z¹ or Z² is -OCH₂- or -CH₂CH₂-, compound (1) has good compatibility with other liquid crystal compounds. When Z¹ or Z² is -C-C- or -C≡C, compound (1) has high reactivity.

In formula (1), Sp¹, Sp², Sp³ and Sp⁴ are independently a single bond or alkylene having 1 to 10 carbons, and in the alkylene, at least one piece of -CH₂- may be replaced by -O-, -COO-, -OCO- or -OCOO-, and at least one piece of -(CH₂)₂- may be replaced by -CH=CH- or -C≡C-, and in the groups, at least one hydrogen may be replaced by fluorine or chlorine.

Preferred Sp¹, Sp², Sp³ and Sp⁴ are a single bond, -CH₂-, -CH₂O-, -OCH₂-, -COO-, -OCO-, -CH₂CH₂-, -CH=CH-, -C≡C-, -(CH₂)₃-, -CH₂CH₂O-, -OCH₂CH₂-, -CH=CH-O-, -O-CH=CH-, -C≡C-O-, -O-C≡C-, -(CH₂)₄-, -(CH₂)₃-O-, -O-(CH₂)₃-, -(CH₂)₄-, -(CH₂)₄O- or -O(CH₂)₄-.

Further preferred Sp¹, Sp², Sp³ and Sp⁴ are a single bond, -CH₂-, -CH₂O-, -OCH₂-, -COO-, -OCO-, -CH=CH-, -C≡C-, -(CH₂)₃-, -CH₂CH₂O-, -OCH₂CH₂-, -CH=CH-O- or -O-CH=CH-.

Particularly preferred Sp¹, Sp², Sp³ and Sp⁴ are a single bond, -CH=CH-O-, -O-CH=CH-, - (CH₂)₃-, -CH₂CH₂O- or -OCH₂CH₂-. Most preferred examples include a single bond. A configuration of a double bond of -CH=CH- may be a cis form or may be a trans form.

When Sp¹, Sp², Sp³ or Sp⁴ is a single bond, compound (1) has high reactivity. When Sp¹, Sp², Sp³ or Sp⁴ is -(CH₂)₃-, compound (1) has significantly good compatibility with liquid crystal compounds and a low polymerization starting temperature. When Sp¹, Sp², Sp³ or Sp⁴ is -CH₂CH₂O- or -OCH₂CH₂-, compound (1) has high reactivity and comparatively good compatibility with liquid crystal compounds. When Sp¹, Sp², Sp³ or Sp⁴ is -CH=CH-O- or -O-CH=CH-, compound (1) has significantly high reactivity.

In formula (1), a is 0, 1, 2, 3 or 4.

Preferred a is 0, 1 or 2. Particularly preferred a is 0 or 1.

When a is 0, compound (1) has good compatibility. When a is 1, compound (1) has high reactivity and good compatibility with a liquid crystal compound. When a is 2, compound (1) has high chemical stability.

In formula (1), c, d and e are independently 0, 1, 2, 3 or 4, and a sum of c, d and e is 1, 2, 3 or 4.

Preferred c, d and e are 0 or 1, and a sum of c, d and e is 1 or 2.

When a sum of c, d and e is 1, chemical stability is high, and compatibility with the liquid crystal compound is good. When a sum of c, d and e is 2, compatibility with the liquid crystal compound is good, reactivity is high, and a polymerization starting temperature is low.

Specific examples of preferred compound (1) include compounds (1-1) to (1-3) described in item 5. Specific examples of further preferred compound (1) include compounds (1-4) to (1-12) described in item 6, and compound (1-4-1), (1-5-1), (1-6-1), (1-7-1), (1-8-1), (1-9-1), (1-10-1), (1-11-1) and (1-12-1) described below. Specific examples of most preferred compound (1) include compounds (1-13) to (1-24) described in item 7.

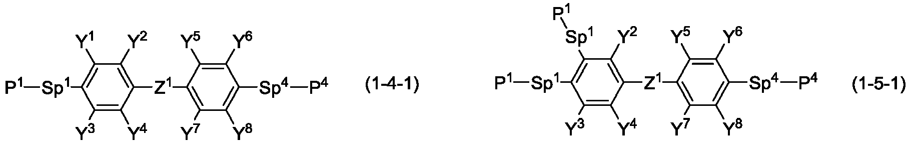

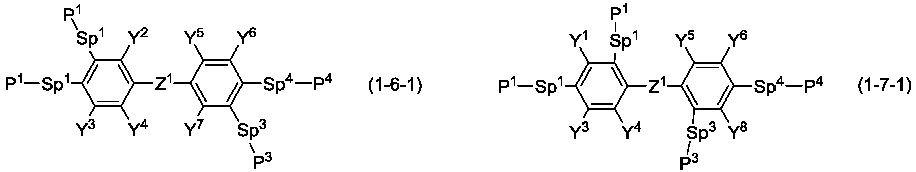

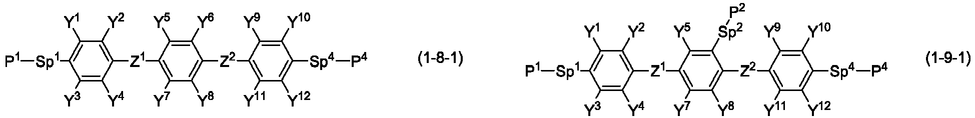

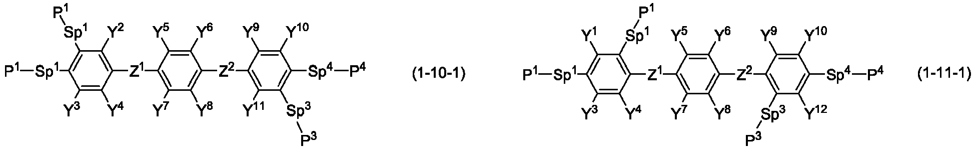

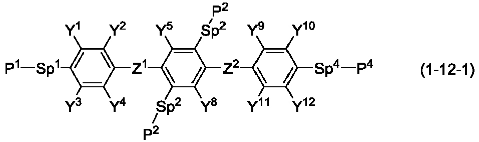

Compound (1-11) is preferred from a viewpoint of high stability to heat and light, and good compatibility. Compounds (1-12) to (1-15) are preferred from a viewpoint of high reactivity. Compounds (1-16) to (1-18) are preferred from a viewpoint of high stability to heat and light, and high reactivity. Compounds (1-19) to (1-24) are preferred from a viewpoint of high reactivity.

### 2. Synthesis of compound (1)

The synthetic method of compound (1) will be described. Compound (1) can be prepared by suitably combining methods in organic synthetic chemistry. A method for introducing a required terminal group, ring and bonding group into a starting material is described in books such as "Organic Syntheses" (John Wiley & Sons, Inc.), "Organic Reactions" (John Wiley & Sons, Inc.), "Comprehensive Organic Synthesis" (Pergamon Press) and "New Experimental Chemistry Course (Shin Jikken Kagaku Koza in Japanese)" (Maruzen Co., Ltd.).

### 2-1. Formation of bonding group Z

First, a scheme is shown with regard to a method of forming bonding groups Z¹ to Z³. Next, reactions described in the scheme will be described in methods (1) to (11). In the scheme, MSG¹ (or MSG²) is a monovalent organic group having at least one ring. The monovalent organic groups represented by a plurality of MSG¹ (or MSG²) used in the scheme may be identical or different. Compounds (1A) to (1J) correspond to compound (1).

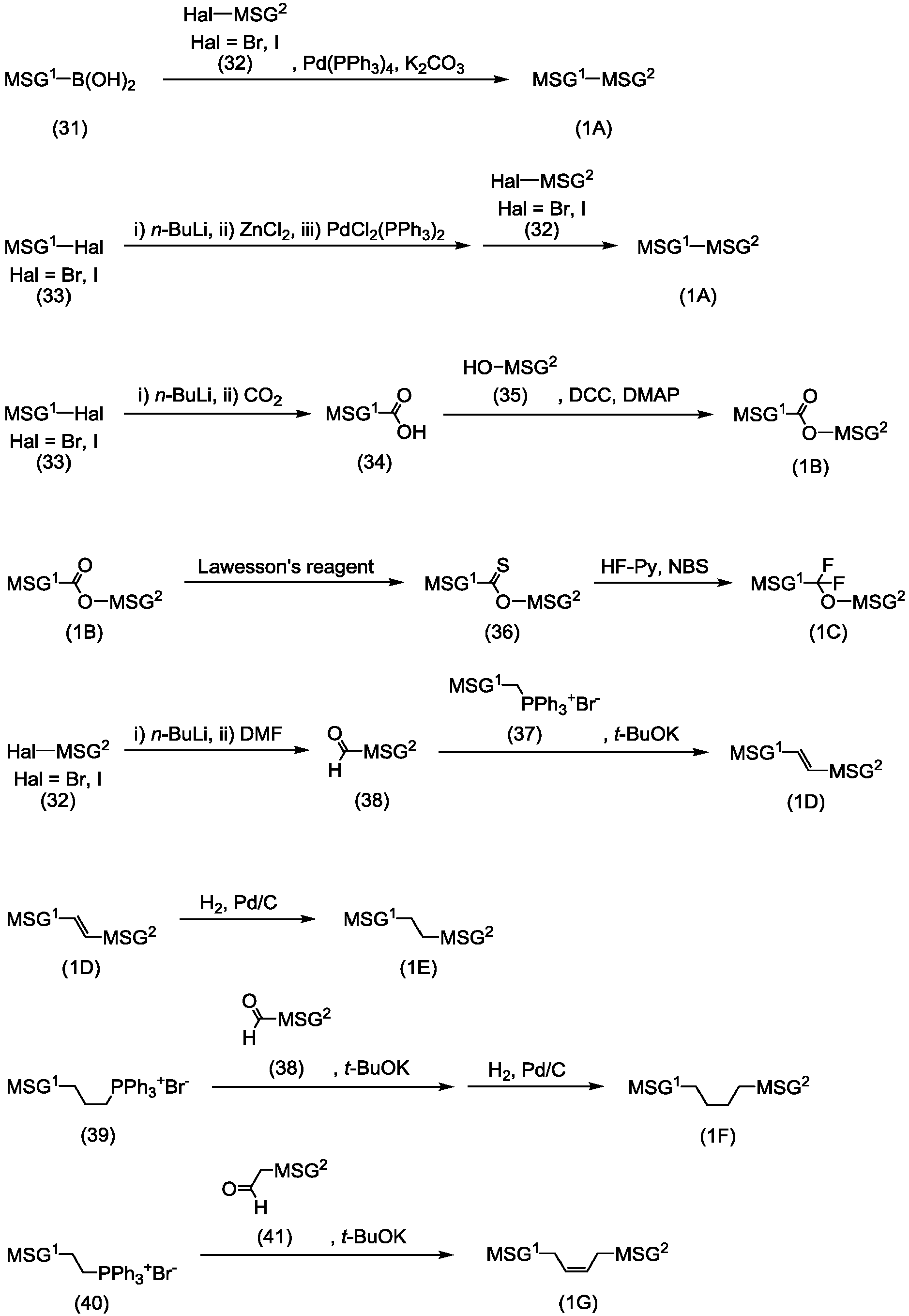

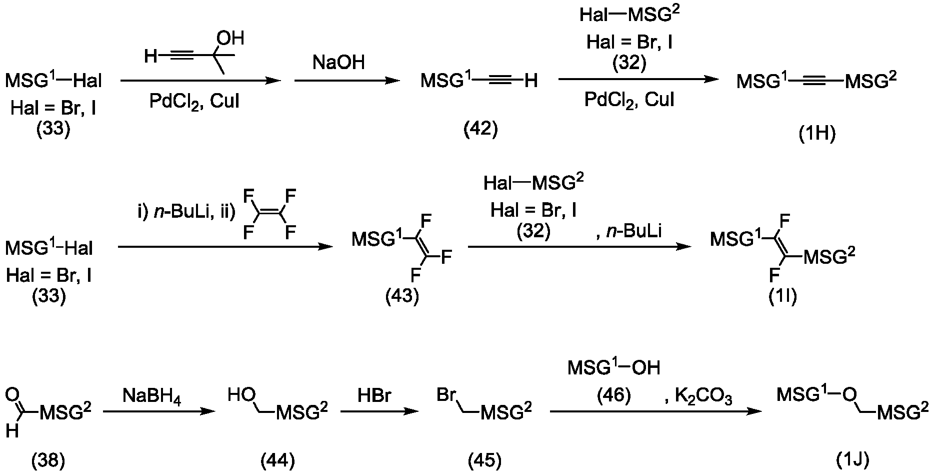

### (1) Formation of a single bond

Compound (1A) is prepared by allowing aryl boronic acid (31) prepared according to a publicly-known method to react with halide (32) in the presence of a carbonate and a catalyst such as tetrakis(triphenylphosphine)palladium. Compound (1A) is also prepared by allowing halide (33) prepared according to a publicly-known method to react with n-butyllithium and subsequently with zinc chloride, and further with halide (32) in the presence of a catalyst such as dichlorobis(triphenylphosphine)palladium.

### (2) Formation of -COO-

Carboxylic acid (34) is obtained by allowing halide (33) to react with n-butyllithium and subsequently with carbon dioxide. Compound (1B) is prepared by dehydration of compound (35) prepared according to a publicly-known method and carboxylic acid (34) in the presence of 1,3-dicyclohexylcarbodiimide (DCC) and 4-dimethylaminopyridine (DMAP) .

### (3) Formation of -CF₂O-

Thionoester (36) is obtained by treating compound (1B) with a thiation reagent such as Lawesson' s reagent. Compound (1C) is prepared by fluorinating thionoester (36) with a hydrogen fluoride-pyridine complex and N-bromosuccinimide (NBS). Refer to M. Kuroboshi et al., Chem. Lett., 1992, 827. Compound (1C) is also prepared by fluorinating thionoester (36) with (diethylamino) sulfur trifluoride (DAST) . Refer to W. H. Bunnelle et al., J. Org. Chem. 1990, 55, 768. The bonding group can also be formed according to the method described in Peer. Kirsch et al., Angew. Chem. Int. Ed. 2001, 40, 1480.

### (4) Formation of -CH=CH-

Aldehyde (38) is obtained by treating halide (32) with n-butyllithium and then allowing the treated halide to react with formamide such as N,N-dimethylformamide (DMF). Phosphorus ylide is generated by treating phosphonium salt (37) prepared according to a publicly-known method with a base such as potassium t-butoxide. Compound (1D) is prepared by allowing the phosphorus ylide to react with aldehyde (38). A cis isomer may be generated depending on reaction conditions, and therefore the cis isomer is isomerized into a trans isomer according to a publicly-known method when necessary.

### (5) Formation of -CH₂CH₂-

Compound (1E) is prepared by hydrogenating compound (1D) in the presence of a catalyst such as palladium on carbon.

### (6) Formation of - (CH₂)₄-

A compound having - (CH₂)₂-CH=CH- is obtained by using phosphonium salt (39) in place of phosphonium salt (37) according to the method in method (4). Compound (1F) is prepared by performing catalytic hydrogenation of the compound obtained.

### (7) Formation of -CH₂CH=CHCH₂-

Compound (1G) is prepared by using phosphonium salt (40) in place of phosphonium salt (37) and aldehyde (41) in place of aldehyde (38) according to the method in method (4). A trans isomer may be generated depending on reaction conditions, and therefore the trans isomer is isomerized into a cis isomer according to a publicly-known method when necessary.

### (8) Formation of -C≡C-

Compound (32) is obtained by allowing halide (33) to react with 2-methyl-3-butyn-2-ol in the presence of a catalyst including dichloropalladium and copper halide, and then performing deprotection under basic conditions. Compound (1H) is prepared by allowing compound (42) to react with halide (32) in the presence of the catalyst including dichloropalladium and copper halide.

### (9) Formation of -CF=CF-

Compound (43) is obtained by treating halide (33) with n-butyllithium and then allowing the treated halide to react with tetrafluoroethylene. Compound (1I) is prepared by treating halide (32) with n-butyllithium and then allowing the treated halide to react with compound (43).

### (10) Formation of -OCH₂-

Compound (44) is obtained by reducing aldehyde (38) with a reducing agent such as sodium borohydride. Bromide (45) is obtained by brominating compound (44) with hydrobromic acid or the like. Compound (1J) is prepared by allowing bromide (45) to react with compound (46) in the presence of a base such as potassium carbonate.

### (11) Formation of -CF₂CF₂-

A compound having -(CF₂)₂- is obtained by fluorinating diketone (-COCO-) with sulfur tetrafluoride, in the presence of a hydrogen fluoride catalyst, according to the method described in J. Am. Chem. Soc., 2001, 123, 5414.

### 2-2. Formation of ring A¹, ring A² and ring A³

A starting material is commercially available or the formation method is well known with regard to a ring such as 1,4-cyclohexylene, 1,4-phenylene, 2-fluoro-1,4-phenylene or 2,6-difluoro-1,4-phenylene. With regard to formation of tetrahydropyran-2,5-diyl, see paragraphs [0084] to [0107] in JP 2013-241397 A. With regard to formation of 1,3-dioxane-2,5-diyl, see paragraphs [0096] to [0119] in JP 2009-132927 A. With regard to formation of pyrimidine-2,5-diyl and pyridine-2,5-diyl, see paragraphs [0086] to [0094] in WO 2010/047260 A.

### 2-3. Formation of connecting groups Sp¹, Sp², Sp³ and Sp⁴

P¹, P², P³ and P⁴ are a polymerizable group. Examples of a method to prepare a compound in which the polymerizable group is bonded with a ring at connecting groups Sp¹, Sp², Sp³ and Sp⁴ are as described below. First, an example in which connecting groups Sp¹, Sp², Sp³ and Sp⁴ are a single bond will be described.

### (1) Formation of a single bond

A method for forming a single bond is as described below in a scheme. In the scheme, MSG¹ is a monovalent organic group having at least one ring. Compounds (1S), (IT), (1U), (1V), (1W) and (1X) are equivalent to compound (1).

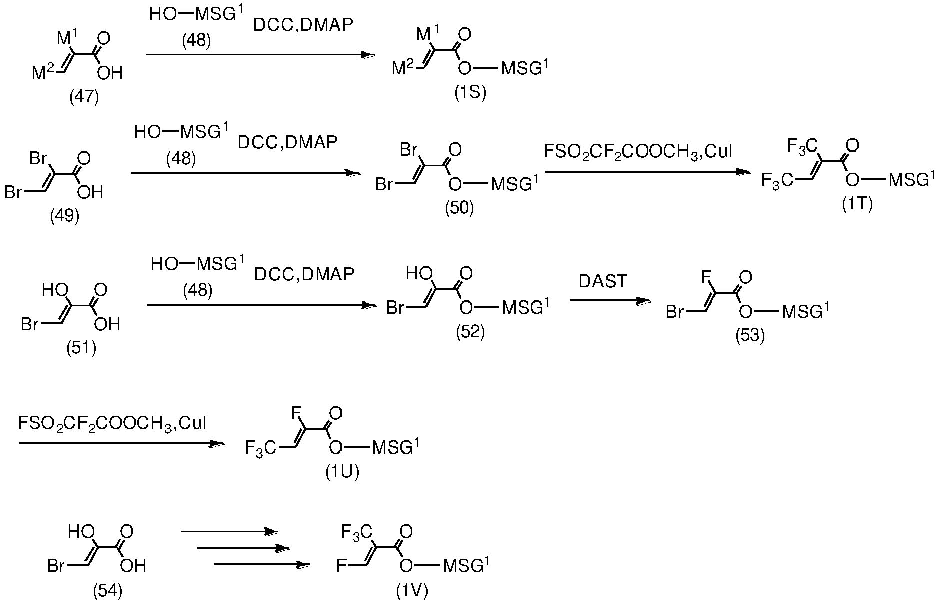

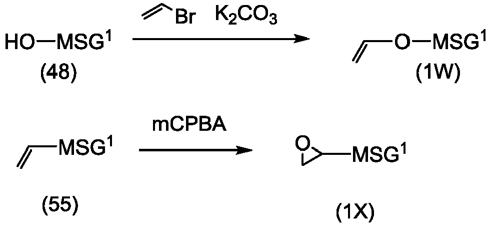

### (2) Formation of M²CH=CM¹-COO-

When neither M¹ nor M² is -CF₃, when M¹ is fluorine and M² is not -CF₃, or when M¹ is -CF₃ and M² is not fluorine, carboxylic acid (47) described in the above scheme is commercially available. Compound (1S) is prepared by dehydrating condensation of carboxylic acid (47) and compound (48) in the presence of DCC and DMAP.

When both M¹ and M² are -CF₃, compound (50) is obtained by dehydrating condensation of carboxylic acid (49) and compound (48) in the presence of DCC and DMAP. Compound (1T) is prepared by allowing compound (50) to react with 2,2-difluoro-2-(fluorosulfonyl)methyl acetate in the presence of a catalyst amount of copper iodide.

When M¹ is fluorine and M² is -CF₃, compound (52) is obtained by dehydrating condensation of carboxylic acid (51) and compound (48) in the presence of DCC and DMAP. Compound (53) is obtained by fluorinating compound (52) with a fluorinating agent such as DAST. Compound (1U) is prepared by allowing compound (53) to react with 2,2-difluoro-2-(fluorosulfonyl)methyl acetate in the presence of a catalyst amount of copper iodide.

When M¹ is -CF₃ and M² is a fluorine, compound (1V) is prepared using carboxylic acid (54) as a starting material according to the method described above.

### (3) Formation of a vinyloxy group

Compound (1W) is prepared by allowing compound (48) to react with vinyl bromide in the presence of potassium carbonate or the like.

### (4) Formation of epoxy

Compound (1X) is prepared by oxidizing vinyl compound (55) prepared according to a publicly-known method with meta-chloroperbenzoic acid (mCPBA) or the like.

A synthesis method of a compound in which connecting groups Sp¹, Sp², Sp³ and Sp⁴ are a single bond is as described above. Next, a method for forming any other connecting group will be described below in a scheme. In the scheme, MSG¹ is a monovalent organic group having at least one ring. Compound (1Y), compound (1Z), compound (1a) and compound (1b) are equivalent to compound (1).

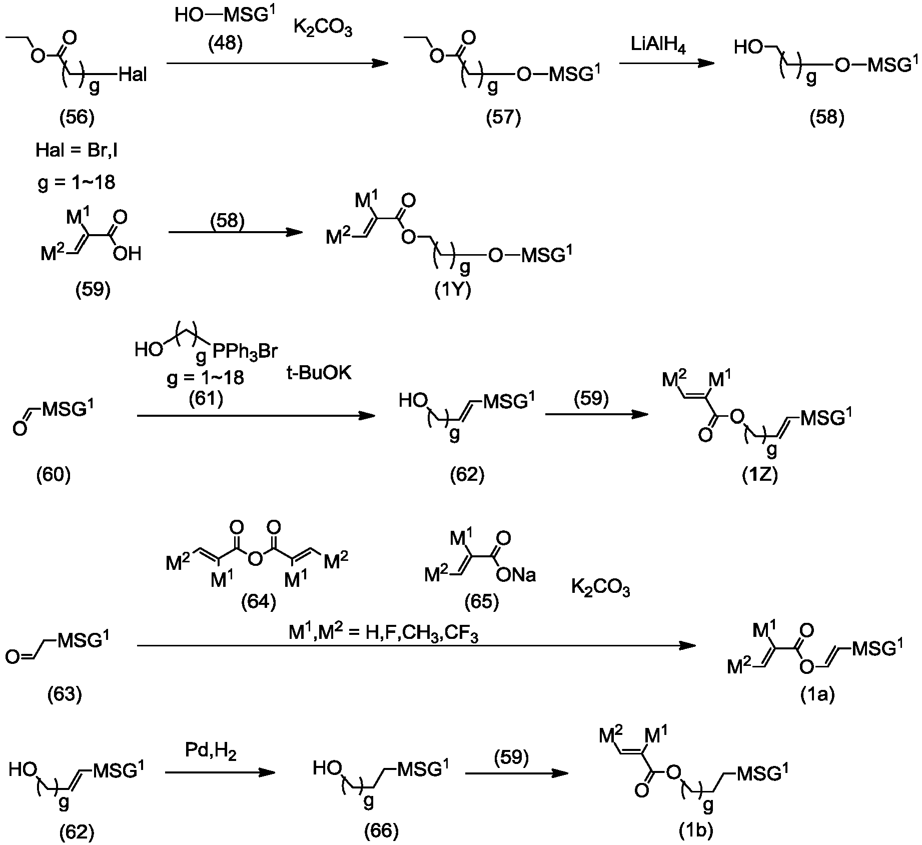

### (5) Formation of -(CH₂)_{g}-O-

Compound (57) is obtained by allowing compound (56) prepared according to a publicly-known method to react with compound (48) in the presence of potassium carbonate or the like. Compound (58) is obtained by reducing compound (57) with a reducing agent such as lithium hydride aluminum. Compound (1Y) is obtained by dehydrating condensation of compound (58) and carboxylic acid (59).

### (6) Formation of - (CH₂)_{g}-CH=CH-

Compound (62) is obtained by allowing phosphorus ylide generated by treating phosphonium salt (61) prepared according to a publicly-known method with a base such as potassium t-butoxide to react with aldehyde (60). Compound (1Z) is obtained by dehydrating condensation of compound (62) and compound (59).

### (7) Formation of -CH=CH-

Compound (1a) is prepared by allowing aldehyde (63) prepared according to a publicly-known method to react with acid anhydride (64) and sodium carboxylate (65) in the presence of potassium carbonate or the like.

### (8) Formation of -(CH₂)g-

Alcohol (66) is prepared by hydrogenating compound (62) in the presence of a catalyst such as palladium on carbon. Compound (1b) is obtained by dehydrating condensation of compound (66) and compound (59) .

### 2-4. Method for introducing a methoxymethyl acrylic group

An example of a method for preparing compound (1) is as described below. Compound (1) is prepared by allowing compound (67) prepared according to a publicly-known method to react with compound (68) prepared according to a publicly-known method in the presence of 1-(3-dimethylaminopropyl)-3-ethylcarbodiimide and triethylamine.

In the compounds, a definition of a symbol of R¹, ring A¹ or the like is identical to a definition of a symbol described in item 1.

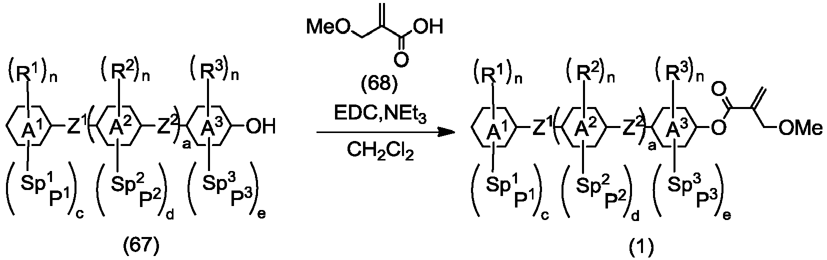

### 3. Polymerizable Composition

A liquid crystal composition of the invention contains compound (1) as component A. The composition preferably further contains a liquid crystal compound selected from components B, C, D and E described below. Component B is compounds (2) to (4) described above. Component C is compounds (5) to (7) described above. Component D is compound (8) described above. Component E is compounds (11) to (19) described later. The composition may contain any other liquid crystal compound different from compounds (2) to (19). When the composition is prepared, components B, C, D and E are preferably selected by taking into account magnitude of the positive or negative dielectric anisotropy or the like. A composition in which components thereof are suitably selected has high maximum temperature, low minimum temperature, small viscosity, suitable optical anisotropy (more specifically, large optical anisotropy or small optical anisotropy), large positive or negative dielectric anisotropy, large specific resistance, stability to heat or ultraviolet light and a suitable elastic constant (more specifically, a large elastic constant or a small elastic constant).

A polymerizable composition is prepared by adding compound (1) to the liquid crystal composition. An additive may be added to the composition when necessary. In such a composition, an addition amount of compound (1), more specifically, component A is in the range of 0.05% by weight to 20% by weight based on the weight of the liquid crystal composition. A further preferred addition amount is in the range of 0.1% by weight to 10% by weight. A most preferred addition amount is in the range of 0.2% by weight to 1% by weight. At least one of other polymerizable compounds different from compound (1) may be further added. On the occasion, a total amount of adding compound (1) and any other polymerizable compound is preferably in the range described above. Physical properties of a polymer to be formed can be adjusted by suitably selecting any other polymerizable compound. Specific examples of any other polymerizable compound include acrylate and methacrylate as described above. Compounds (M-1) to (M-18) is also included in the specific examples.

Component B includes a compound in which two terminal groups are alkyl or the like. Specific examples of preferred component B include compounds (2-1) to (2-11), compounds (3-1) to (3-19) and compounds (4-1) to (4-7) . In a compound of component B, R¹¹ and R¹² are independently alkyl having 1 to 10 carbons or alkenyl having 2 to 10 carbons, and in the alkyl or the alkenyl, at least one piece of -CH₂- may be replaced by -O-, and at least one hydrogen may be replaced by fluorine.

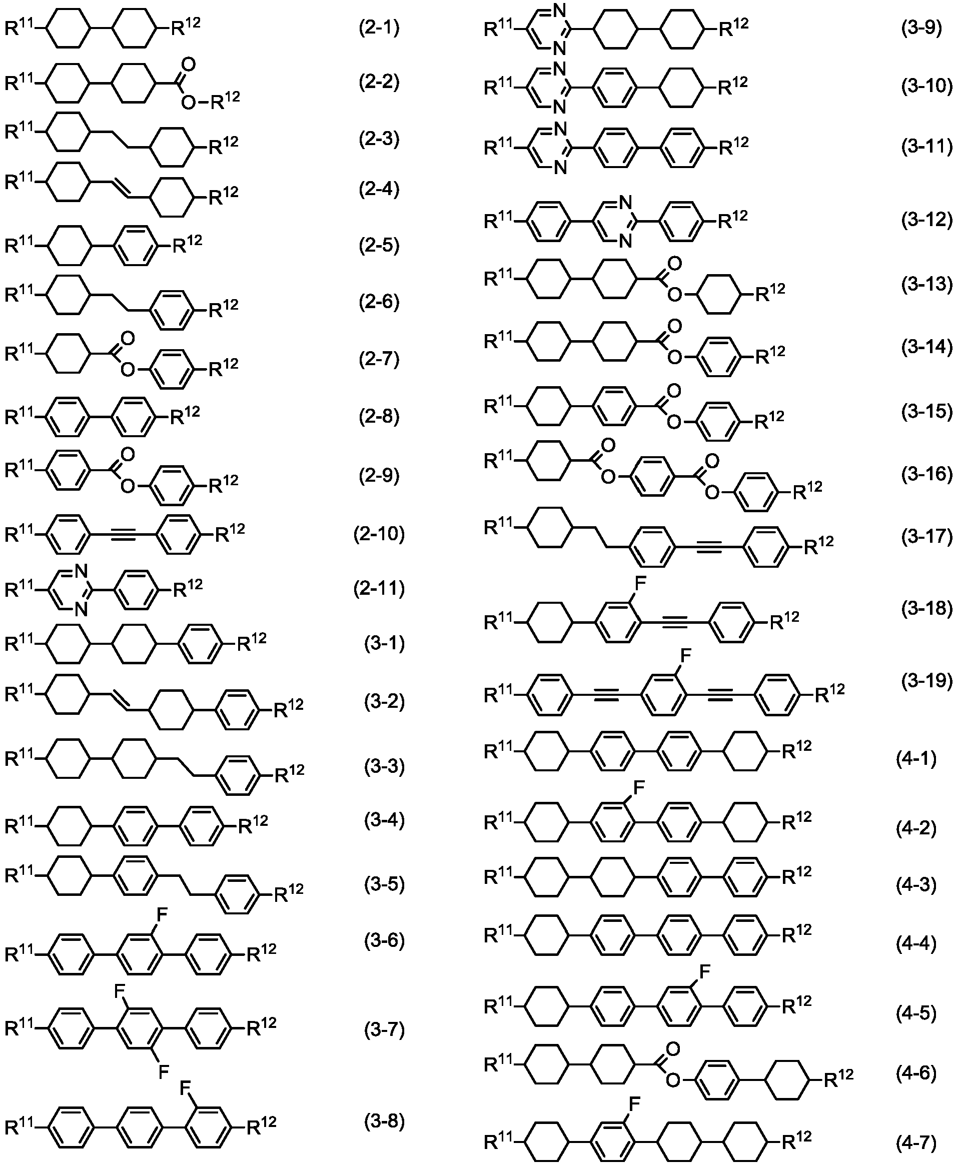

Component B has a small absolute value of dielectric anisotropy, and therefore is a compound close to neutrality. Compound (2) is mainly effective in adjusting viscosity or adjusting optical anisotropy. Compounds (3) and (4) are effective in extending the temperature range of the nematic phase by increasing the maximum temperature, or in adjusting the optical anisotropy.

As a content of component B is increased, the dielectric anisotropy of the composition is decreased, but the viscosity is decreased. Thus, as long as a desired value of threshold voltage of the device is met, the content is preferably as large as possible. Accordingly, when a composition for the PS-IPS mode, the PSA-VAmode or the like is prepared, the content of component B is preferably 30% by weight or more, and further preferably 40% by weight or more, based on the weight of the liquid crystal composition.

Component C is a compound having a halogen-containing group or a fluorine-containing group at a right terminal. Specific examples of preferred component C include compounds (5-1) to (5-16), compounds (6-1) to (6-116) and compounds (7-1) to (7-59). In the compounds, R¹³ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine. X¹¹ is fluorine, chlorine, -OCF₃, -OCHF₂, -CF₃, -CHF₂, -CH₂F, -OCF₂CHF₂ or -OCF₂CHFCF₃.

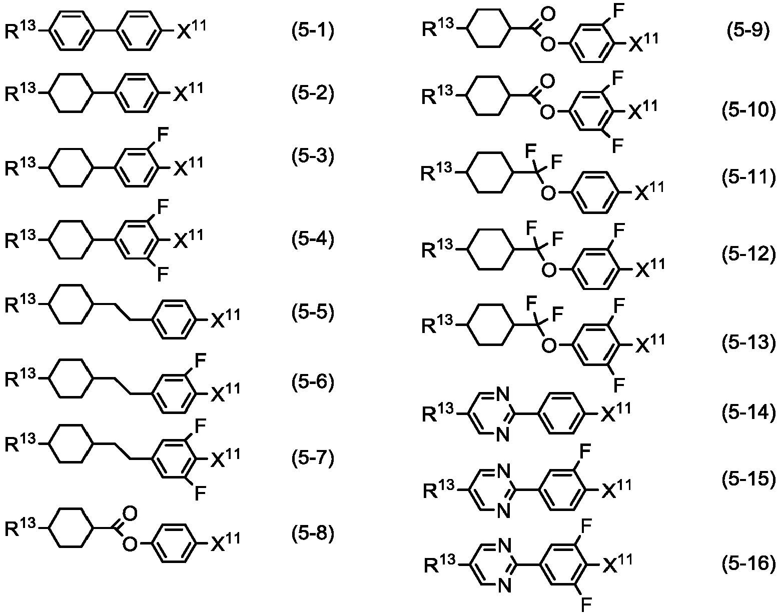

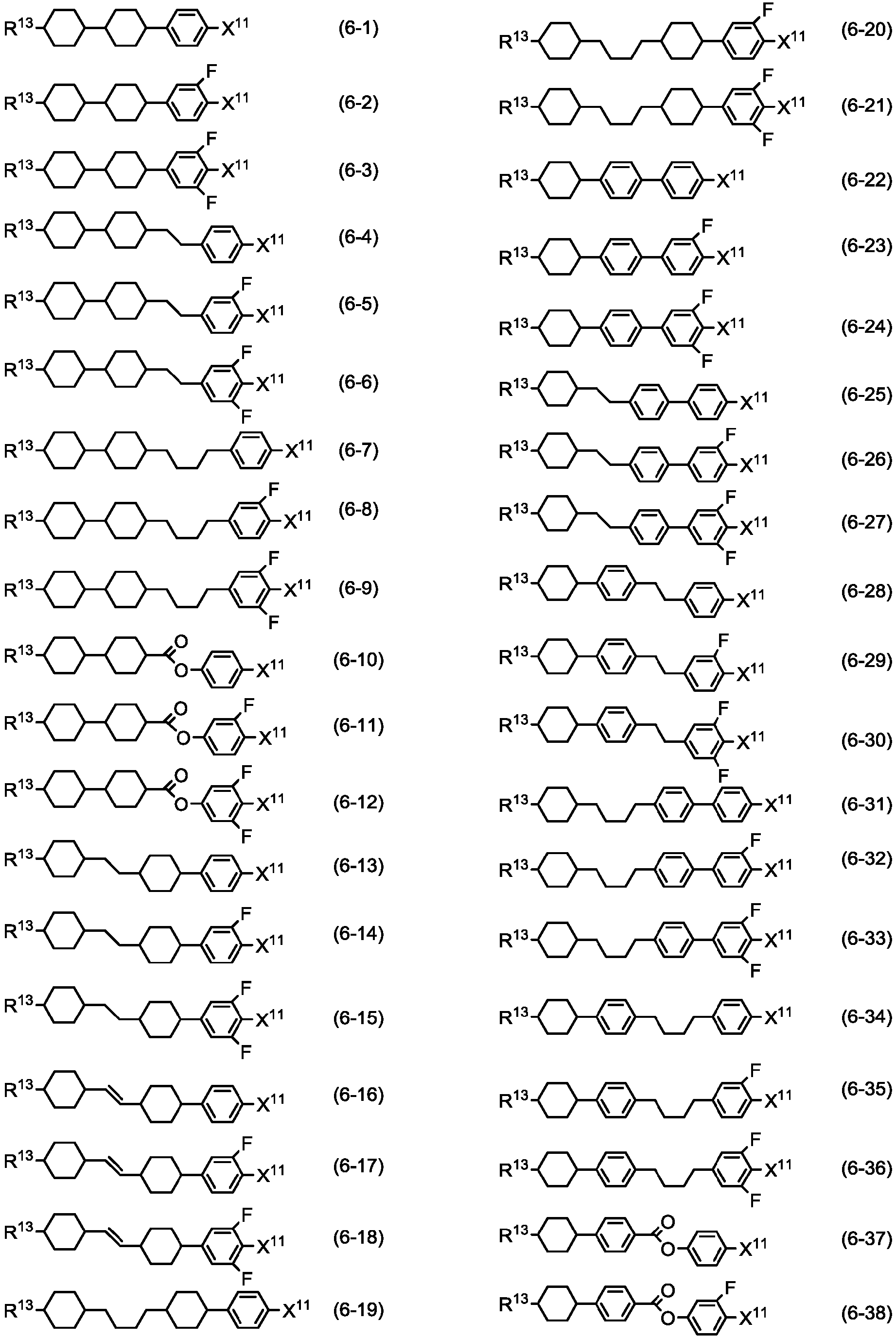

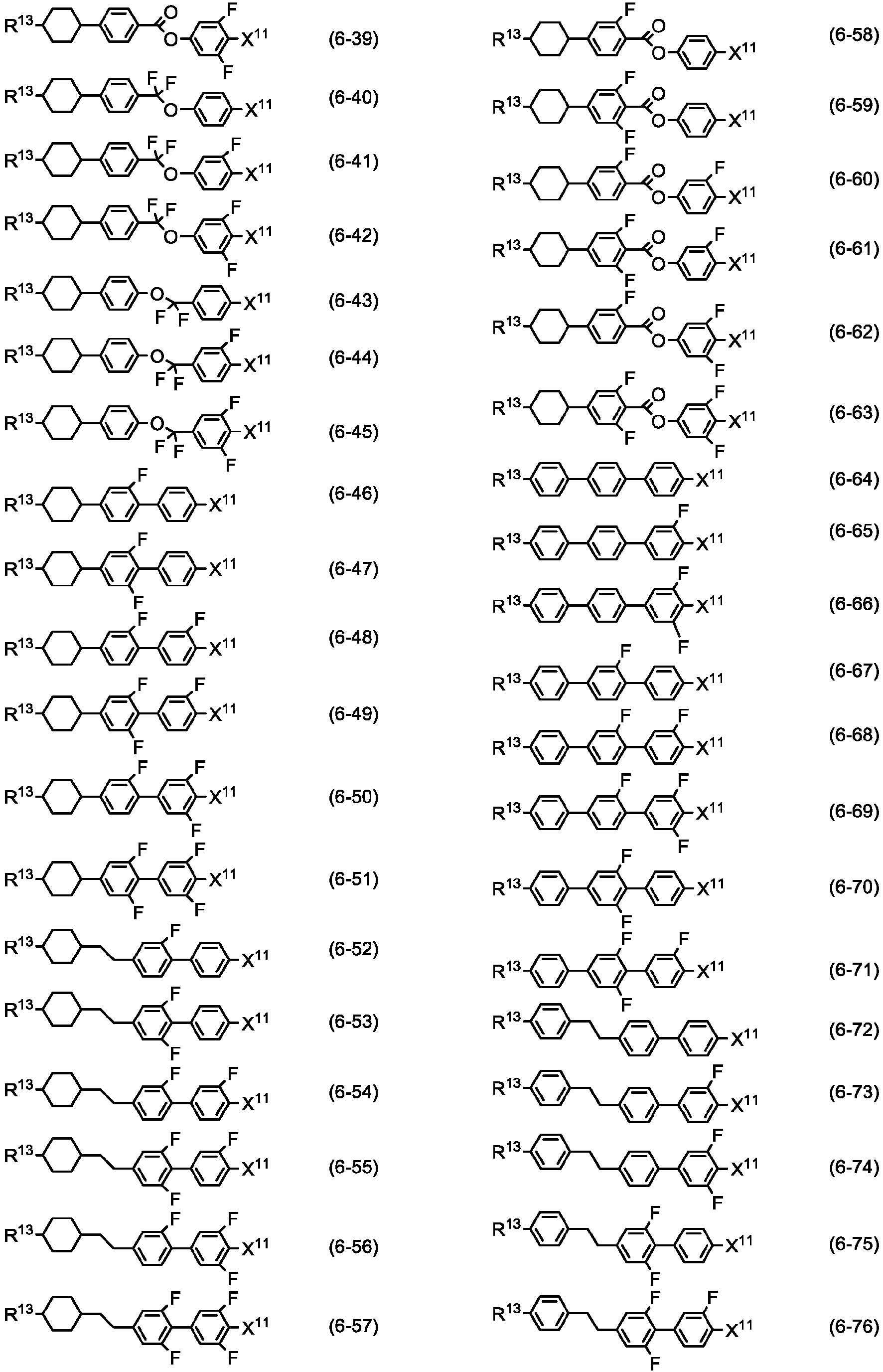

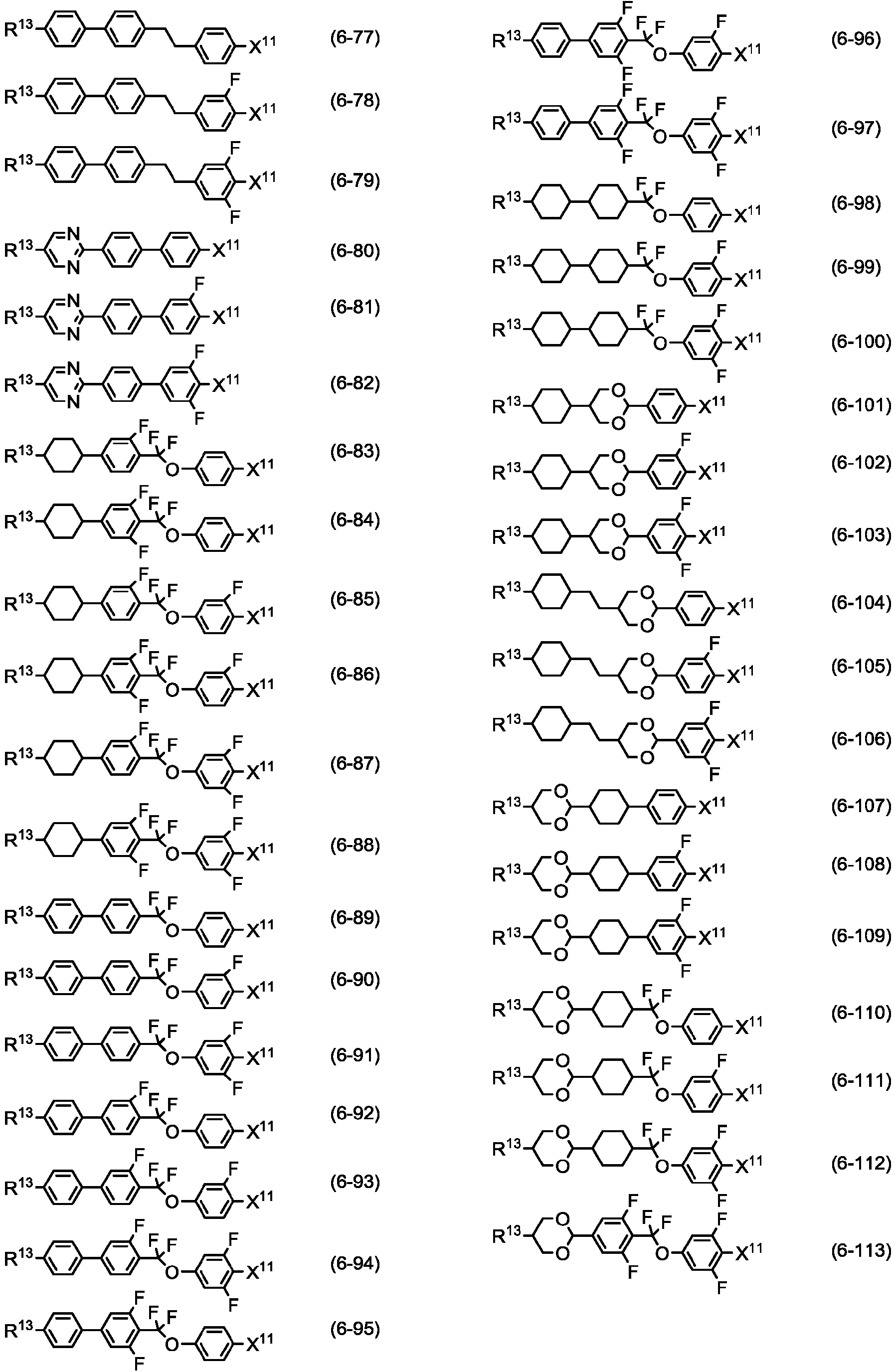

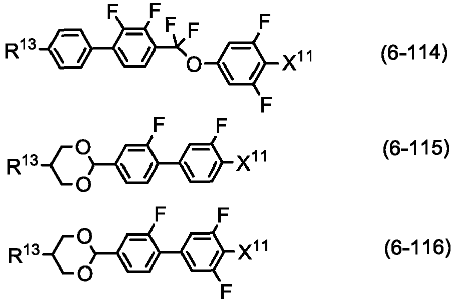

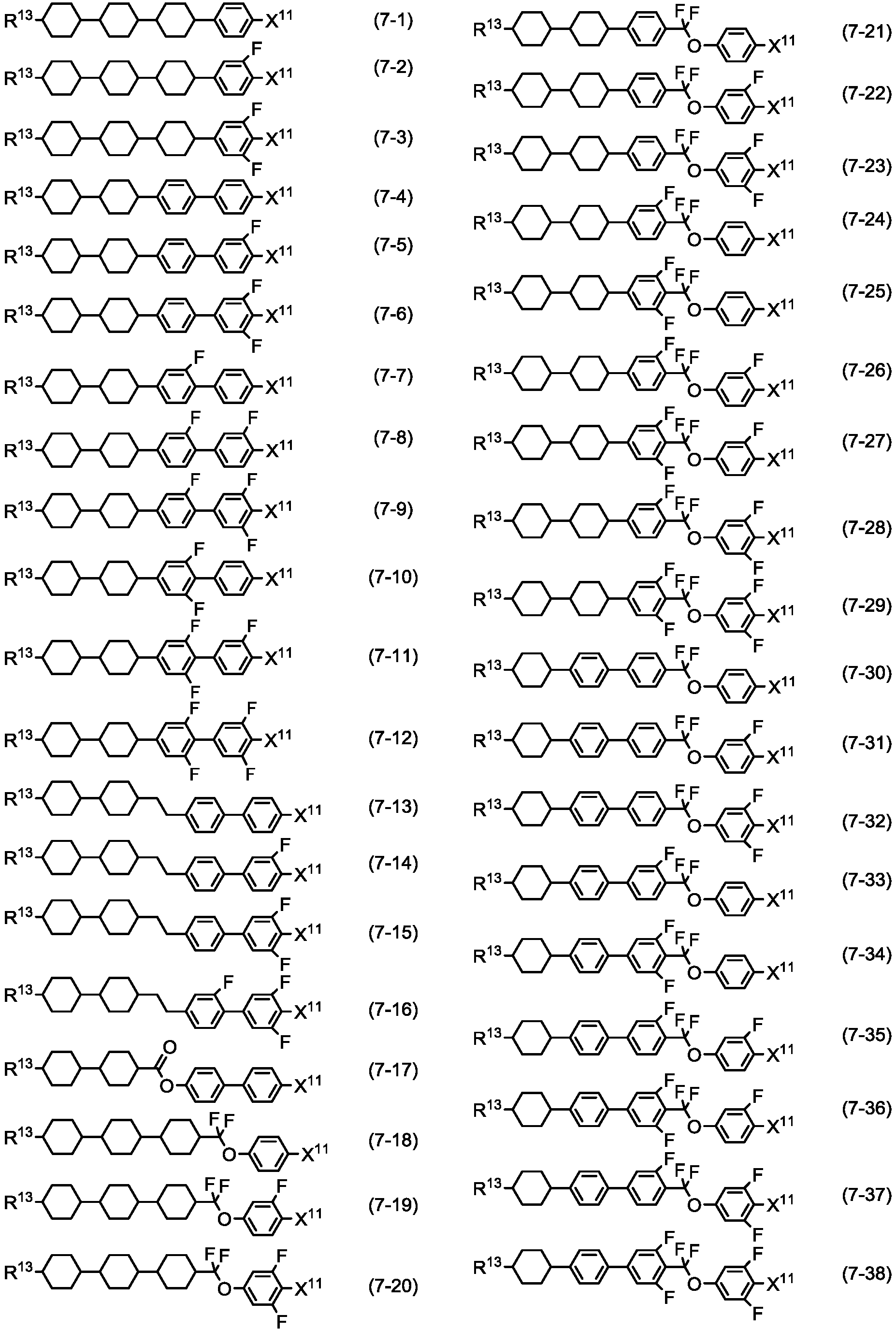

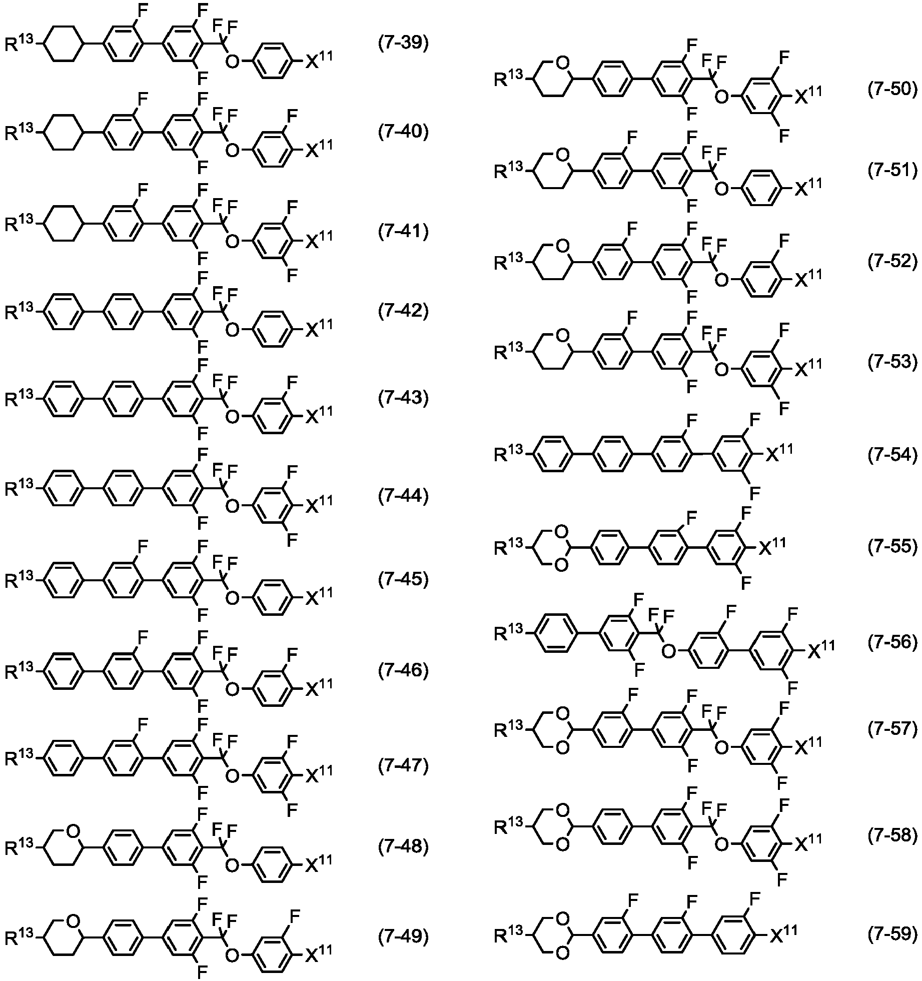

Component C has positive dielectric anisotropy, and significantly good stability to heat and light, and therefore is used when a composition for the IPS mode, the FFS mode, the OCB mode or the like is prepared. A content of component C is suitably in the range of 1% by weight to 99% by weight, preferably in the range of 10% by weight to 97% by weight, and further preferably in the range of 40% by weight to 95% by weight, based on the weight of the liquid crystal composition. When component C is added to a composition having negative dielectric anisotropy, the content of component C is preferably 30% by weight or less. Addition of component C allows adjustment of the elastic constant of the composition and adjustment of a voltage-transmittance curve of the device.

Component D is compound (8) in which a right-terminal group is -C=N or -C≡C-C≡N. Preferred examples of component D include compounds (8-1) to (8-64). In the compounds, R¹⁴ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine. X¹² is -C≡N or -C≡C-C≡N.

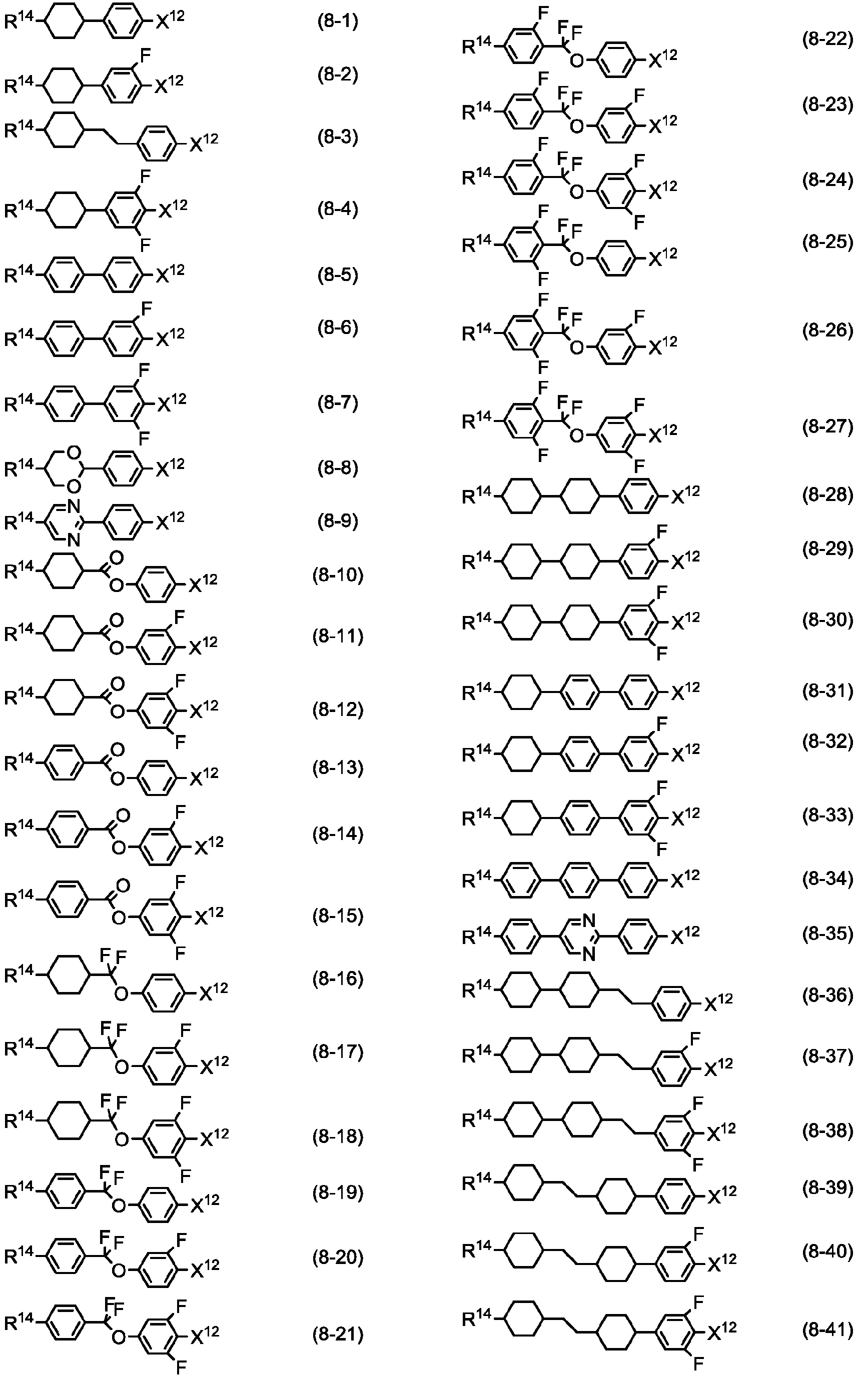

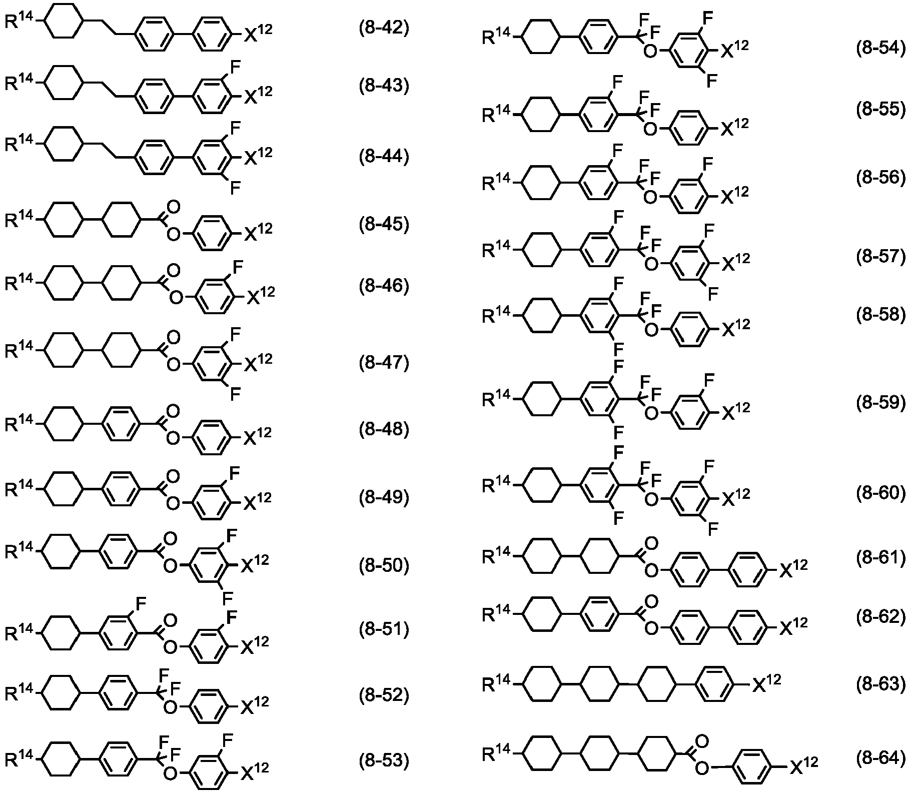

Component D has positive dielectric anisotropy and a value thereof is large, and therefore is used when a composition for the TN mode or the like is prepared. Addition of component D can increase the dielectric anisotropy of the composition. Component D is effective in extending the temperature range of the liquid crystal phase, adjusting the viscosity or adjusting the optical anisotropy. Component D is also useful for adjustment of the voltage-transmittance curve of the device.

When the composition for the PS-TN mode or the like is prepared, a content of component D is suitably in the range of 1% by weight to 99% by weight, preferably in the range of 10% by weight to 97% by weight, and further preferably in the range of 40% by weight to 95% by weight, based on the weight of the liquid crystal composition. When component D is added to a composition having negative dielectric anisotropy, the content of component D is preferably 30% by weight or less. Addition of component D allows adjustment of the elastic constant of the composition and adjustment of the voltage-transmittance curve of the device.

Component E includes compounds (11) to (19). The compounds have phenylene in which hydrogen in lateral positions are replaced by two halogens, such as 2,3-difluoro-1,4-phenylene. Specific examples of preferred component E include compounds (11-1) to (11-9), compounds (12-1) to (12-19), compounds (13-1) and (13-2), compounds (14-1) to (14-3), compounds (15-1) to (15-3), compounds (16-1) to (16-11), compounds (17-1) to (17-3), compounds (18-1) to (18-3) and compound (19-1) . In the compounds, R¹⁵, R¹⁶ and R¹⁷ are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine, and R¹⁷ may be hydrogen or fluorine.

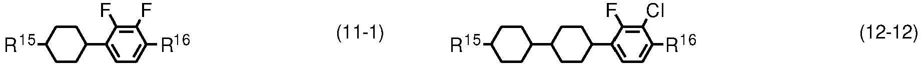

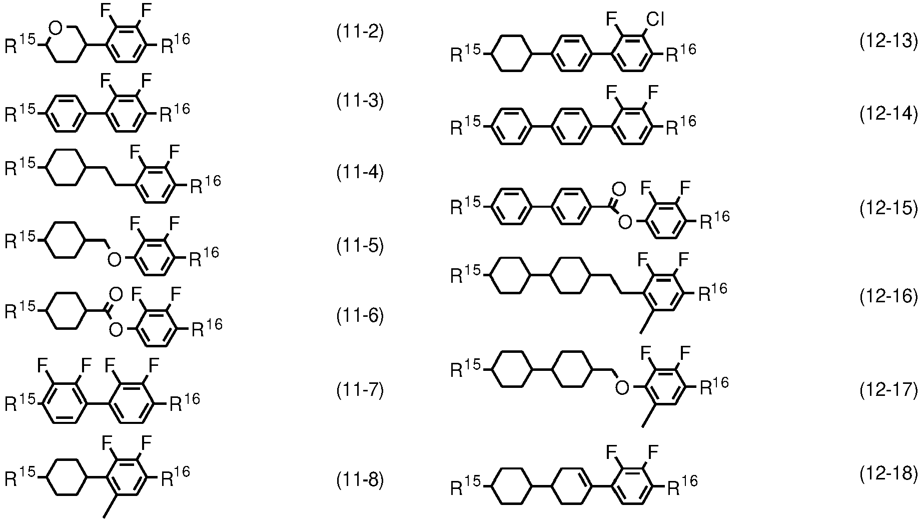

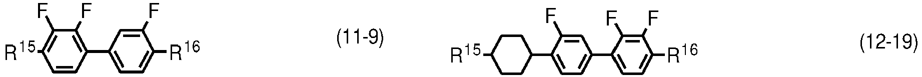

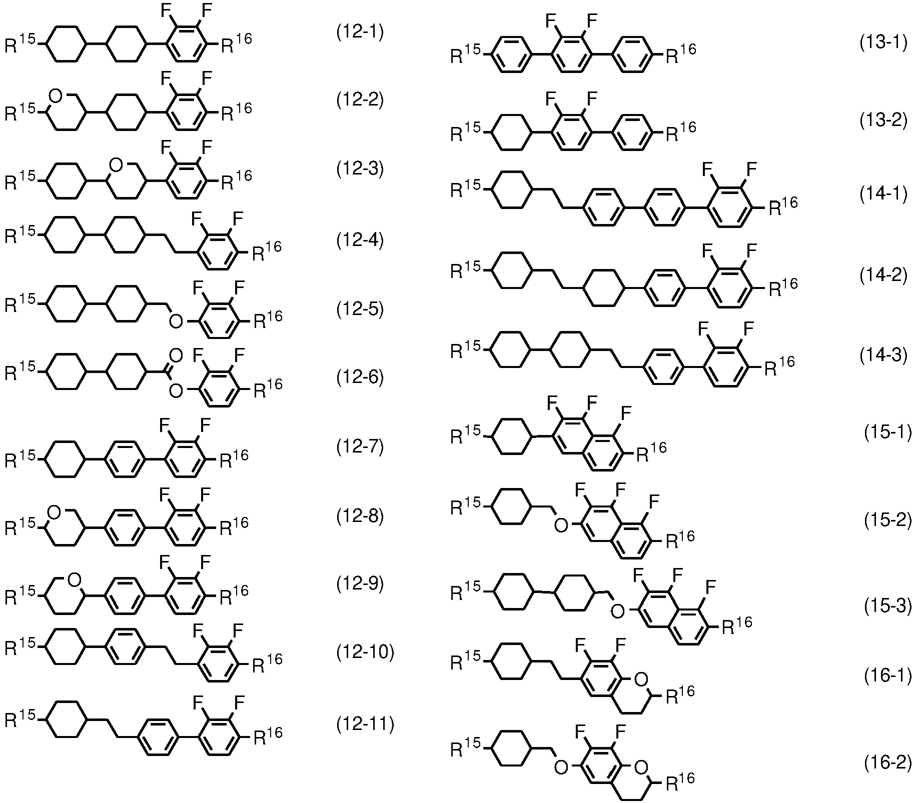

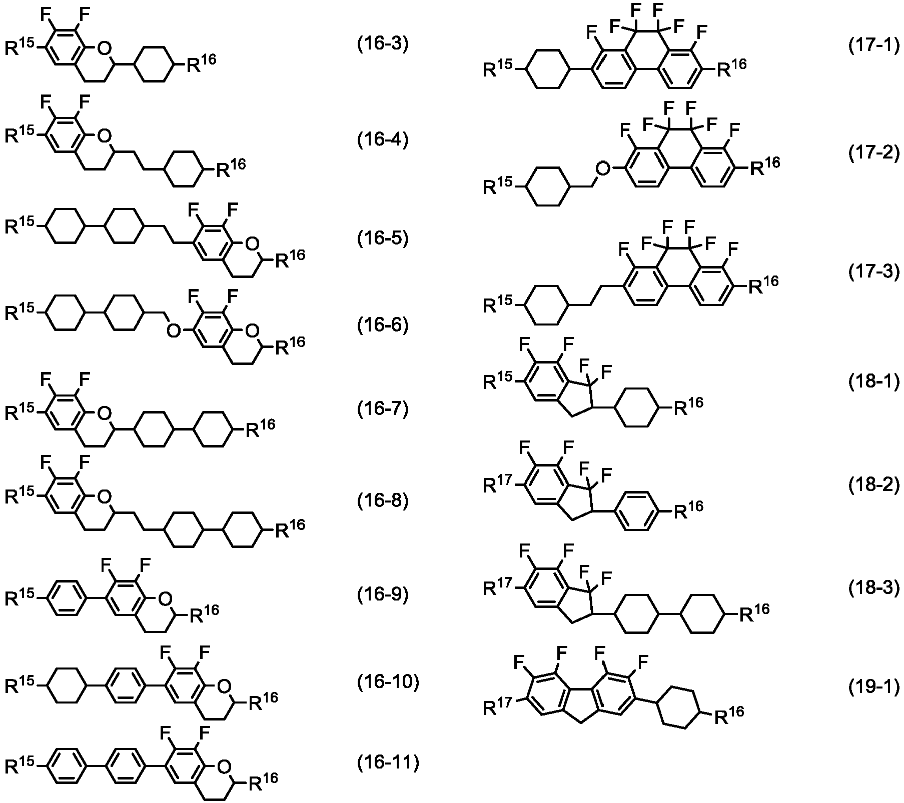

Component E has large negative dielectric anisotropy. Component E is used when a composition for the IPS mode, the VA mode, the PSA mode or the like is prepared. As a content of component E is increased, the dielectric anisotropy of the composition is negatively increased, but the viscosity is increased. Thus, as long as the desired value of threshold voltage of the device is met, the content is preferably as small as possible. When the dielectric anisotropy at a degree of -5 is taken into account, the content is preferably 40% by weight or more in order to allow a sufficient voltage driving.

Among types of component E, compound (11) is a bicyclic compound, and therefore is effective in decreasing the viscosity, adjusting the optical anisotropy or increasing the dielectric anisotropy. Compounds (12) and (13) are a tricyclic compound, and compound (14) is a tetracyclic compound, and therefore compounds (12), (13) and (14) are effective in increasing the maximum temperature, the optical anisotropy or the dielectric anisotropy. Compounds (15) to (19) are effective in increasing the dielectric anisotropy.

When a composition for the IPS mode, the VA mode, the PSA mode or the like is prepared, the content of component E is preferably 40% by weight or more, and further preferably in the range of 50% by weight to 95% by weight, based on the weight of the liquid crystal composition. When component E is added to a composition having positive dielectric anisotropy, the content of component E is preferably 30% by weight or less. Addition of component E allows adjustment of the elastic constant of the composition and adjustment of the voltage-transmittance curve of the device.

A liquid crystal composition satisfying at least one of physical properties such as high stability to heat and light, high maximum temperature, low minimum temperature, small viscosity, suitable optical anisotropy (more specifically, large optical anisotropy or small optical anisotropy), large dielectric anisotropy, large specific resistance and a suitable elastic constant (more specifically, a large elastic constant or a small elastic constant) can be prepared by suitably combining components B, C, D and E with compound (1). The device including such a composition has a wide temperature range in which the device can be used, a short response time, a large voltage holding ratio, low threshold voltage, a large contrast ratio, a small flicker rate and a long service life.

If the device is used for a long period of time, a flicker may be occasionally generated on a display screen. The flicker rate (%) can be represented by a formula (| luminance when applying positive voltage - luminance when applying negative voltage|) / (average luminance) × 100. In a device having the flicker rate in the range of 0% to 1%, a flicker is hardly generated on the display screen even if the device is used for a long period of time. The flicker is associated with image persistence, and is presumed to be generated according to a difference in electric potential between a positive frame and a negative frame in driving at alternating current. The composition containing compound (1) is also useful for a decrease in generation of the flicker.

### 3-2. Additive

A liquid crystal composition is prepared according to a publicly-known method. For example, the component compounds are mixed and dissolved in each other by heating. According to an application, an additive may be added to the composition. Specific examples of the additives include the polymerizable compound, the polymerization initiator, the polymerization inhibitor, the optically active compound, the antioxidant, the ultraviolet light absorber, the light stabilizer, the heat stabilizer, the dye and the antifoaming agent. Such additives are well known to those skilled in the art, and described in literature.

In a liquid crystal display device having the polymer sustained alignment (PSA) mode, the composition contains a polymer. The polymerizable compound is added for the purpose of forming the polymer in the composition. The polymerizable compound is polymerized by irradiation with ultraviolet light while voltage is applied between electrodes, and thus the polymer is formed in the composition. A suitable pretilt is achieved by the method, and therefore the device in which a response time is shortened and the image persistence is improved is prepared.

Specific examples of a preferred polymerizable compound include acrylate, methacrylate, a vinyl compound, a vinyloxy compound, propenyl ether, an epoxy compound (oxirane, oxetane) and vinyl ketone. Further preferred examples include a compound having at least one acryloyloxy, and a compound having at least one methacryloyloxy. Still further preferred examples also include a compound having both acryloyloxy and methacryloyloxy.

Still further preferred examples include compound (16).

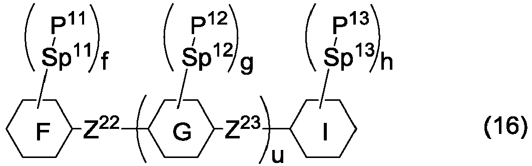

In formula (16), ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen.

Preferred ring F or ring I is cyclohexyl, cyclohexenyl, phenyl, fluorophenyl, difluorophenyl, 1-naphthyl or 2-naphthyl. Further preferred ring F or ring I is cyclohexyl, cyclohexenyl or phenyl. Particularly preferred ring F or ring I is phenyl.

Ring G is 1,4-cyclohexylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, phenanthrene-2,7-diyl, tetrahydropyran-2,5-diyl, 1,3-dioxane-2,5-diyl, pyrimidine-2,5-diyl or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen.

Preferred ring G is 1,4-cyclohexylene, 1,4-cyclohexenylene, 1,4-phenylene, 2-fluoro-1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl or naphthalene-2,7-diyl. Further preferred ring G is 1,4-cyclohexylene, 1,4-cyclohexenylene, 1,4-phenylene or 2-fluoro-1,4-phenylene. Particularly preferred ring G is 1,4-phenylene or 2-fluoro-1,4-phenylene. Most preferred ring G is 1,4-phenylene.

In formula (16), Z²² and Z²³ are independently a single bond or alkylene having 1 to 10 carbons, and in the alkylene, at least one piece of -CH₂- may be replaced by -O-, -CO-, -COO- or -OCO-, and at least one piece of -CH₂CH₂- may be replaced by -CH=CH-, -C(CH₃)=CH-, -CH=C(CH₃)- or -C(CH₃) =C(CH₃) -, and in the groups, at least one hydrogen may be replaced by fluorine or chlorine. Preferred Z²² or Z²³ is a single bond, -CH₂CH₂-, -CH₂O-, -OCH₂-, -COO- or -OCO-. Further preferred Z²² or Z²³ is a single bond.

In compound (16), P¹¹, P¹² and P¹³ are independently a polymerizable group. Preferred P¹¹ to P¹³ are a group selected from the group of polymerizable groups represented by formula (P-2) to formula (P-6). Further preferred P¹¹ to P¹³ are a group represented by formula (P-2) or formula (P-6). Particularly preferred P¹¹ to P¹³ are a group represented by formula (P-2). A preferred group represented by formula (P-2) is acryloyloxy (-OCO-CH=CH₂) or methacryloyloxy (-OCO-C(CH₃)=CH₂). A wavy line in group (P-2) to group (P-6) represents a site subjected to a bonding.

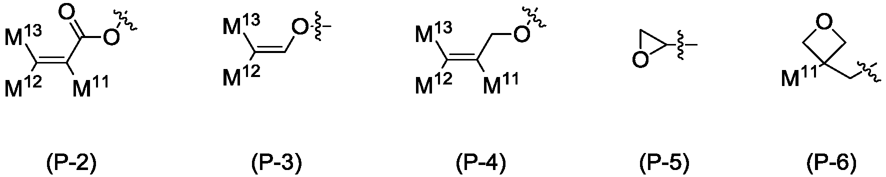

In group (P-2) to group (P-6), M¹¹, M¹² and M¹³ are independently hydrogen, fluorine, alkyl having 1 to 5 carbons, or alkyl having 1 to 5 carbons in which at least one hydrogen is replaced by halogen. Preferred M¹¹, M¹² or M¹³ is hydrogen or methyl for increasing reactivity. Further preferred M¹¹ is hydrogen or methyl, and further preferred M¹² or M¹³ is hydrogen.

In formula (16), Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine. Preferred Sp¹¹, Sp¹² or Sp¹³ is a single bond.

In formula (16), u is 0, 1 or 2. Preferred u is 0 or 1.

Then, f, g and h are independently 0, 1, 2, 3 or 4, and a sum of f, g and h is 1 or more. Preferred f, g or h is 1 or 2. A preferred sum thereof is 2, 3 or 4. A further preferred sum thereof is 2 or 3. A still further preferred sum thereof is 3 or 4.

Still further preferred examples include compounds (16-1-1) to (16-1-5), compounds (16-2-1) to (16-2-5), compound (16-4-1), compound (16-5-1), compound (16-6-1), compound (16-8), compound (16-9), compound (16-10), compound (16-11), compound (16-12), compound (16-13), compound (16-14), compound (16-15) and compound (16-16). In the compounds, R²⁵ to R³¹ are independently hydrogen or methyl; v and x are independently 0 or 1; t and u are independently an integer from 1 to 10; and L³¹ to L³⁶ are independently hydrogen or fluorine, and L³⁷ and L³⁸ are independently hydrogen, fluorine or methyl.

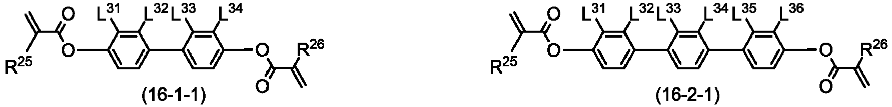

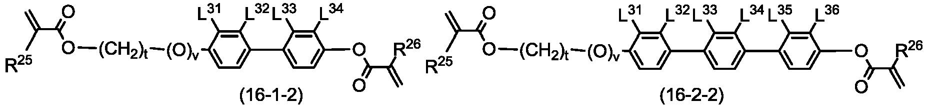

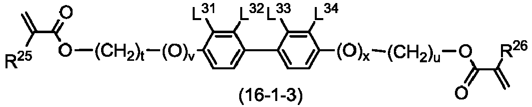

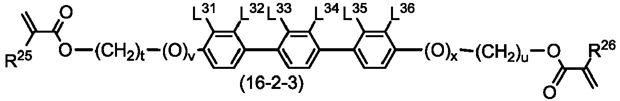

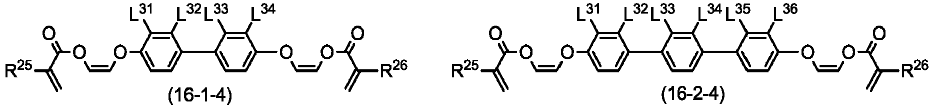

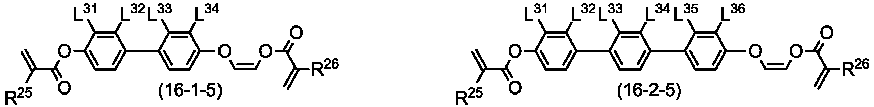

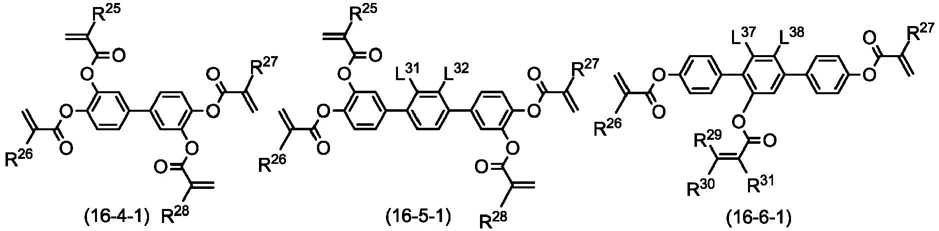

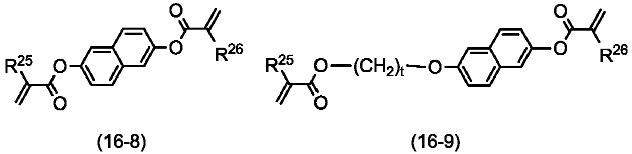

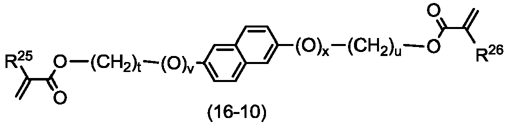

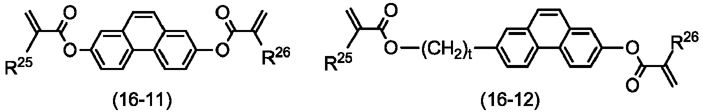

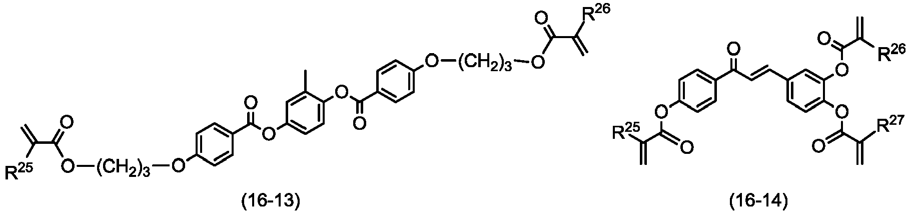

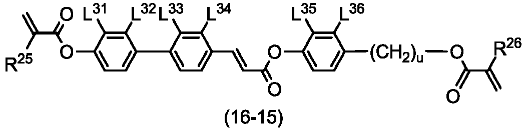

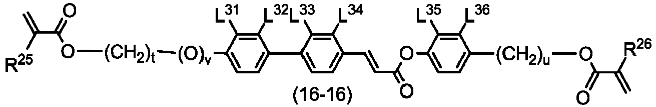

A polymerizable compound can be rapidly polymerized by adding the polymerization initiator. An amount of a remaining polymerizable compound can be decreased by optimizing a reaction condition. Specific examples of a photoradical polymerization initiator include TPO, 1173 and 4265 from Darocur series of BASF SE, and 184, 369, 500, 651, 784, 819, 907, 1300, 1700, 1800, 1850 and 2959 from Irgacure series thereof.

Additional examples of the photoradical polymerization initiator include 4-methoxyphenyl-2,4-bis(trichloromethyl)triazine, 2-(4-butoxystyryl)-5-trichloromethyl-1,3,4-oxadiazole, 9-phenylacridine, 9,10-benzphenazine, a benzophenone-Michler's ketone mixture, a hexaarylbiimidazole-mercaptobenzimidazole mixture, 1-(4-isopropylphenyl)-2-hydroxy-2-methylpropane-1-one, benzyl dimethyl ketal, 2-methyl-1-[4-(methylthio)phenyl]-2-morpholinopropane-1-one, a mixture of 2,4-diethylxanthone and methyl p-dimethylaminobenzoate and a mixture of benzophenone and methyltriethanolamine.

After the photoradical polymerization initiator is added to the liquid crystal composition, polymerization can be performed by irradiation with ultraviolet light while an electric field is applied. However, an unreacted polymerization initiator or a decomposition product of the polymerization initiator may cause a poor display such as the image persistence in the device. In order to prevent such an event, photopolymerization may be performed with no addition of the polymerization initiator. A preferred wavelength of irradiation light is in the range of 150 nanometers to 500 nanometers . A further preferred wavelength is in the range of 250 nanometers to 450 nanometers, and a most preferred wavelength is in the range of 300 nanometers to 400 nanometers.

Upon storing the polymerizable compound, the polymerization inhibitor may be added thereto for preventing polymerization. The polymerizable compound is ordinarily added to the composition without removing the polymerization inhibitor. Specific examples of the polymerization inhibitor include hydroquinone, a hydroquinone derivative such as methylhydroquinone, 4-t-butylcatechol, 4-methoxyphenol and phenothiazine.

The optically active compound is effective in inducing a helical structure in liquid crystal molecules to give a required twist angle, and thereby preventing a reverse twist. A helical pitch can be adjusted by adding the optically active compound thereto. Two or more optically active compounds may be added for the purpose of adjusting temperature dependence of the helical pitch. Specific examples of a preferred optically active compound include compounds (Op-1) to (Op-18) described below. In compound (Op-18), ring J is 1,4-cyclohexylene or 1,4-phenylene, and R²⁸ is alkyl having 1 to 10 carbons. An asterisk (*) represents asymmetrical carbon.

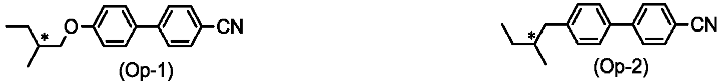

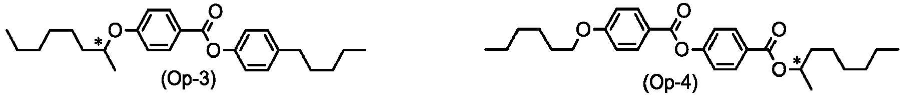

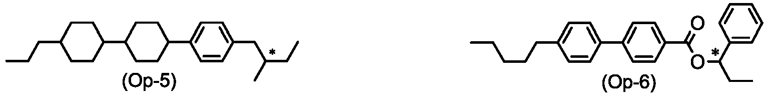

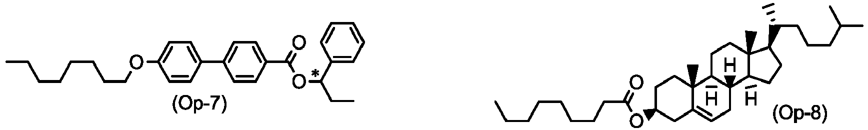

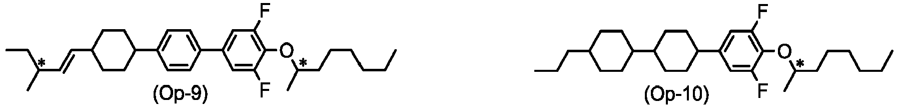

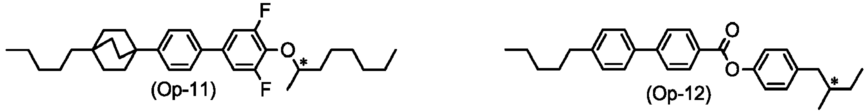

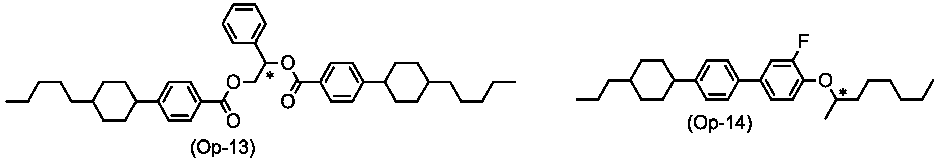

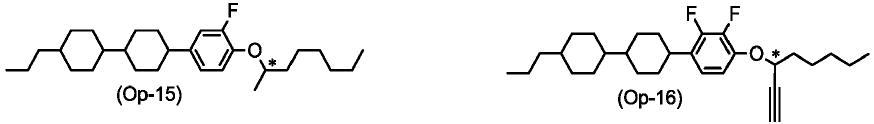

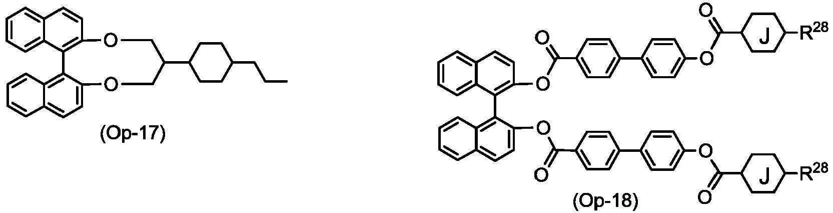

The antioxidant is effective for maintaining the large voltage holding ratio. Specific examples of a preferred antioxidant include compounds (AO-1) and (AO-2) described below; and Irganox 415, Irganox 565, Irganox 1010, Irganox 1035, Irganox 3114 and Irganox 1098 (trade names; BASF SE). The ultraviolet light absorber is effective for preventing a decrease of the maximum temperature. Preferred examples of the ultraviolet light absorbers include a benzophenone derivative, a benzoate derivative and a triazole derivative, and specific examples include compounds (AO-3) and (AO-4) described below; Tinuvin 329, Tinuvin P, Tinuvin 326, Tinuvin 234, Tinuvin 213, Tinuvin 400, Tinuvin 328 and Tinuvin 99-2 (trade names; BASF SE); and 1,4-diazabicyclo[2.2.2]octane (DABCO).

The light stabilizer such as an amine having steric hindrance is preferred for maintaining the large voltage holding ratio. Specific examples of a preferred light stabilizer include compounds (AO-5), (AO-6) and (AO-7) described below; Tinuvin 144, Tinuvin 765, Tinuvin 770DF and Tinuvin 780 (trade names; BASF SE) ; and LA-52, LA-57, LA-77Y and LA-77G (trade names; ADEKA Corporation). The heat stabilizer is also effective for maintaining the large voltage holding ratio, and preferred examples include Irgafos 168 (trade name; BASF SE). A dichroic dye such as an azo dye or an anthraquinone dye is added to the composition to be adapted for a device having a guest host (GH) mode. The antifoaming agent is effective for preventing foam formation. Specific examples of a preferred antifoaming agent include dimethyl silicone oil and methylphenyl silicone oil.

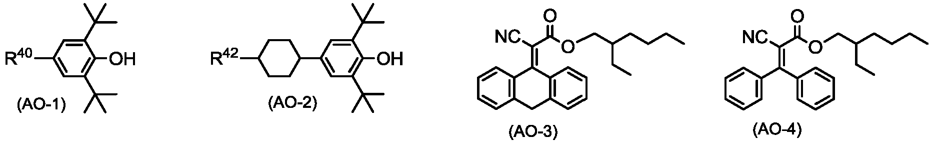

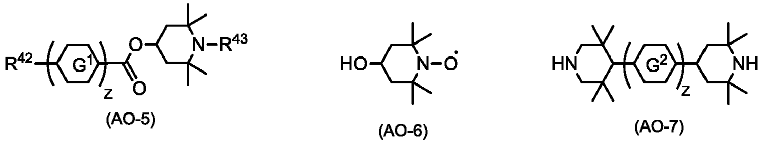

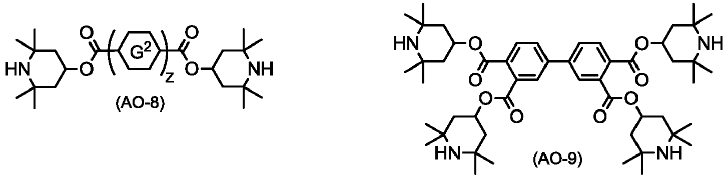

In compound (AO-1), R⁴⁰ is alkyl having 1 to 20 carbons, alkoxy having 1 to 20 carbons, -COOR⁴¹ or -CH₂CH₂COOR⁴¹, in which R⁴¹ is alkyl having 1 to 20 carbons. In compounds (AO-2) and (AO-5), R⁴² is alkyl having 1 to 20 carbons. In compound (AO-5), R⁴³ is hydrogen, methyl or O˙ (oxygen radical); and ring G¹ is 1,4-cyclohexylene or 1,4-phenylene; and in compound (AO-7), ring G² is 1,4-cyclohexylene, 1,4-phenylene or 1,4-phenylene in which at least one hydrogen is replaced by fluorine; and in compounds (AO-5) and (AO-7), z is 1, 2 or 3.

### 4. Liquid crystal display device

An effect of a polymer in a liquid crystal display device is interpreted as described below. "Polymerizable composition" is a mixture of the liquid crystal compound, the polymerizable compound or the like. Liquid crystal molecules are aligned in a direction of an electric field by applying the electric field to the composition. Molecules of the polymerizable compound are also aligned in the same direction according to the alignment. The polymerizable compound is polymerized by irradiating the composition with ultraviolet light in a state described above. As a result, a network of the polymer is formed in the polymerizable composition. The liquid crystal molecules are stabilized while being aligned in the direction of the electric field by an effect of the network. The effect is maintained even when the electric field is eliminated. Accordingly, a response time of the device is shortened.

Polymerization of the polymerizable composition is preferably performed in the display device. One example is as described below. A display device having two glass substrates including a transparent electrode and an alignment film is used. A polymerizable composition having compound (1), the liquid crystal composition, the additive or the like as a component is prepared. The composition is injected into the display device. Compound (1) is polymerized by irradiating the display device with ultraviolet light while the electric field is applied thereto. A liquid crystal composite is formed by the polymerization. A liquid crystal display device having the liquid crystal composite by the above method can be easily prepared. Rubbing treatment of the alignment film may be omitted in the above method. In addition, a method for stabilizing liquid crystal molecules in a state without the electric field may be adopted.

When an amount of addition of the polymerizable compound is in the range of 0.1% by weight to 2% by weight based on the weight of the liquid crystal composition, a liquid crystal display device having the PSA mode is prepared. The device having the PSA mode can be driven by a driving mode such as the active matrix (AM) mode and the passive matrix (PM) mode. Such a device can also be applied to any of a reflective type, a transmissive type and a transflective type. A device having a polymer dispersed mode can also be prepared by increasing the amount of addition of the polymerizable compound.

### Examples

### 1. Example of compound (1)

The invention will be described in greater detail by way of Examples . The Examples include a typical example, and therefore the invention is not limited by the Examples. Compound (1) was prepared according to procedures described below. The thus prepared compound was identified by methods such as an NMR analysis. Physical properties of the compound and a composition and characteristics of a device were measured by methods described below.

NMR analysis: For measurement, DRX-500 made by Bruker BioSpin Corporation was used. In ¹H-NMR measurement, a sample was dissolved in a deuterated solvent such as CDCl₃, and measurement was carried out under conditions of room temperature, 500 MHz and 16 times of accumulation. Tetramethylsilane was used as an internal standard. In ¹⁹F-NMR measurement, CFCl₃ was used as an internal standard, and measurement was carried out under conditions of 24 times of accumulation. In explaining nuclear magnetic resonance spectra obtained, s, d, t, q, quin, sext and m stand for a singlet, a doublet, a triplet, a quartet, a quintet, a sextet and a multiplet, and br being broad, respectively.

Gas chromatographic analysis: For measurement, GC-2010 Gas Chromatograph made by Shimadzu Corporation was used. As a column, a capillary column DB-1 (length 60 m, bore 0.25 mm, film thickness 0.25 µm) made by Agilent Technologies, Inc. was used. As a carrier gas, helium (1 mL/minute) was used. A temperature of a sample vaporizing chamber and a temperature of a detector (FID) were set to 300°C and 300°C, respectively. A sample was dissolved in acetone and prepared to be a 1 weight % solution, and then 1 microliter of the solution obtained was injected into the sample vaporizing chamber. As a recorder, GC Solution System made by Shimadzu Corporation or the like was used.

Gas chromatographic mass analysis: For measurement, QP-2010 Ultra Gas Chromatograph Mass Spectrometer made by Shimadzu Corporation was used. As a column, a capillary column DB-1 (length 60 m, bore 0.25 mm, film thickness 0.25 µm) made by Agilent Technologies, Inc. was used. As a carrier gas, helium (1 mL/minute) was used. A temperature of a sample vaporizing chamber, a temperature of an ion source, ionizing voltage and emission current were set to 300°C, 200°C, 70 eV and 150 uA, respectively. A sample was dissolved in acetone and prepared to be a 1 weight % solution, and then 1 microliter of the solution obtained was injected into the sample vaporizing chamber. As a recorder, GCMS solution system made by Shimadzu Corporation was used.

HPLC Analysis: For measurement, Prominence (LC-20AD; SPD-20A) made by Shimadzu Corporation was used. As a column, YMC-Pack ODS-A (length 150 mm, bore 4.6 mm, particle diameter 5 µm) made by YMC Co., Ltd. was used. As an eluate, acetonitrile and water were appropriately mixed and used. As a detector, a UV detector, an RI detector, a CORONA detector or the like was appropriately used. When the UV detector was used, a detection wavelength was set at 254 nanometers. A sample was dissolved in acetonitrile and prepared to be a 0.1 weight % solution, and then 1 microliter of the solution was injected into a sample chamber. As a recorder, C-R7Aplus made by Shimadzu Corporation was used.

Ultraviolet-Visible Spectrophotometry: For measurement, PharmaSpec UV-1700 made by Shimadzu Corporation was used. A detection wavelength was adjusted in the range of 190 nanometers to 700 nanometers . A sample was dissolved in acetonitrile and prepared to be a 0.01 mmol/L solution, and measurement was carried out by putting the solution in a quartz cell (optical path length: 1 cm).

Sample for measurement: Upon measuring phase structure and a transition temperature (a clearing point, a melting point, a polymerization starting temperature or the like), a compound itself was used as a sample. Upon measuring physical properties such as maximum temperature of a nematic phase, viscosity, optical anisotropy and dielectric anisotropy, a mixture of a compound and a base liquid crystal was used as a sample.

When the dielectric anisotropy of the compound was zero or positive, base liquid crystal (A) described below was used. A proportion of each component was expressed in terms of % by weight.

| | |
|---|---|
| 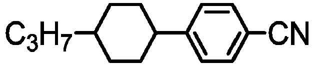 | 24% |
| 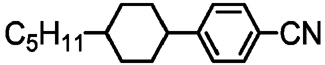 | 36% |
| 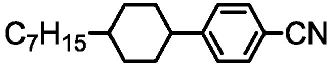 | 25% |
| 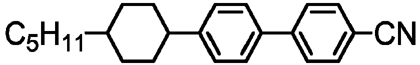 | 15% |

When the dielectric anisotropy of the compound was zero or negative, base liquid crystal (B) described below was used. A proportion of each component was expressed in terms of % by weight.

| | |
|---|---|
| 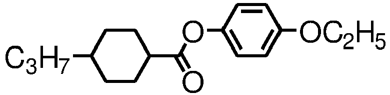 | 17.2% |
| 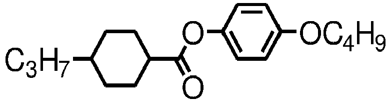 | 27.6% |
| 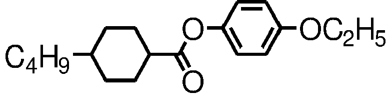 | 20.7% |
| 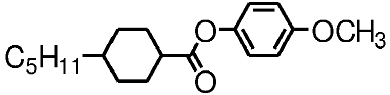 | 20.7% |
| 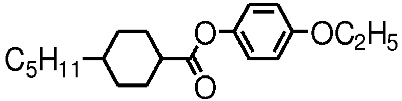 | 13.8% |

Base liquid crystal (C): Base liquid crystal (C) having a fluorine-based compound described below as a component may be occasionally used.

| | |
|---|---|
| 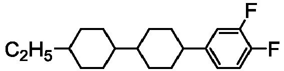 | 16.67% |
| 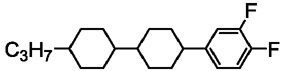 | 16.67% |
| 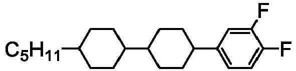 | 16.67% |
| 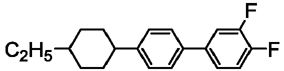 | 6.25% |
| 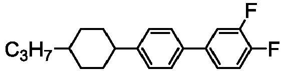 | 6.25% |
| 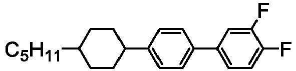 | 12.49% |
| 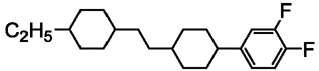 | 12.50% |
| 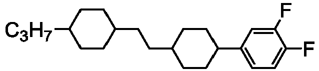 | 6.25% |
| 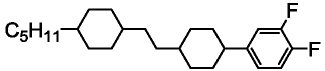 | 6.25% |

Measuring method: Physical properties were measured according to methods described below. Most of the methods are described in the Standard of Japan Electronics and Information Technology Industries Association (JEITA) discussed and established in JEITA (JEITA ED-2521B). A modified method was also applied. No thin film transistor (TFT) was attached to a TN device used for measurement.
(1) Phase structure: A sample was placed on a hot plate in a melting point apparatus (FP-52 Hot Stage made by Mettler-Toledo International Inc.) equipped with a polarizing microscope. A state of phase and a change thereof were observed with the polarizing microscope while the sample was heated at a rate of 3°C per minute, and a kind of the phase was specified.
(2) Transition temperature (°C): For measurement, a differential scanning calorimeter, Diamond DSC System, made by PerkinElmer, Inc., or a high sensitivity differential scanning calorimeter, X-DSC7000, made by SII NanoTechnology Inc. was used. A sample was heated and then cooled at a rate of 3°C per minute, and a starting point of an endothermic peak or an exothermic peak caused by a phase change of the sample was determined by extrapolation, and thus a transition temperature was determined. A melting point and a polymerization starting temperature of a compound were also measured using the apparatus. Temperature at which a compound undergoes transition from a solid to a liquid crystal phase such as the smectic phase and the nematic phase may be occasionally abbreviated as "minimum temperature of the liquid crystal phase." Temperature at which the compound undergoes transition from the liquid crystal phase to liquid may be occasionally abbreviated as "clearing point."
   A crystal was expressed as C. When the crystals were distinguishable into two kinds, each of the crystals was expressed as C₁ or C₂. The smectic phase and the nematic phase were expressed as S and N, respectively. When phases such as smectic A phase, smectic B phase, smectic C phase and smectic F phase were distinguishable, the phases were expressed as S_{A}, S_{B}, Sc and S_{F}, respectively. A liquid (isotropic) was expressed as I. A transition temperature was expressed as "C 50.0 N 100.0 I," for example. The expression indicates that a transition temperature from the crystals to the nematic phase is 50.0°C, and a transition temperature from the nematic phase to the liquid is 100.0°C.
(3) Compatibility of polymerizable compound: Samples in which any one kind of the base liquid crystals and the compound were mixed for a proportion of the compound to be 1.2% by weight, 1.0% by weight, 0.8% by weight, 0.5% by weight, 0.4% by weight or 0.3% by weight were prepared. The samples were put in glass vials and kept in freezers at -20°C or -30°C for a predetermined period of time. Whether a nematic phase of the samples was maintained or crystals (or a smectic phase) precipitated was observed. A condition in which the nematic phase was maintained was used as a measure of compatibility. The proportion of the compound and a temperature in the freezers may be occasionally changed when necessary.
(4) Maximum temperature of nematic phase (T_{NI} or NI; °C) : A sample was placed on a hot plate in a melting point apparatus equipped with a polarizing microscope, and heated at a rate of 1°C per minute. Temperature when part of the sample began to change from a nematic phase to an isotropic liquid was measured. When the sample was a mixture of compound (1) and the base liquid crystal, the maximum temperature was expressed in terms of a symbol T_{NI}. When the sample was a mixture of compound (1) and a compound selected from compound (2) to compound (15), the maximum temperature was expressed as a symbol NI. A maximum temperature of the nematic phase may be occasionally abbreviated as "maximum temperature."
(5) Minimum temperature of nematic phase (Tc; °C): Samples each having a nematic phase were put in glass vials and kept in freezers at temperatures of 0°C, -10°C, -20°C, -30°C and -40°C for 10 days, and then liquid crystal phases were observed. For example, when the sample was maintained in the nematic phase at -20°C and changed to crystals or a smectic phase at -30°C, T_{C} was described as T_{C} < -20°C. A minimum temperature of the nematic phase may be occasionally abbreviated as "minimum temperature."
(6) Viscosity (bulk viscosity; η; measured at 20°C; mPa·s): For measurement, a cone-plate (E type) rotational viscometer made by Tokyo Keiki Inc. was used.
(7) Viscosity (rotational viscosity; γ1; measured at 25°C; mPa·s) : Measurement was carried out according to a method described in M. Imai et al., Molecular Crystals and Liquid Crystals, Vol. 259, p. 37 (1995) . A sample was put in a TN device in which a twist angle was 0 degrees and a distance (cell gap) between two glass substrates was 5 micrometers . Voltage was applied stepwise to the device in the range of 16 V to 19.5 V at an increment of 0.5 V. After a period of 0.2 second with no voltage application, voltage was repeatedly applied under conditions of only one rectangular wave (rectangular pulse; 0.2 second) and no voltage application (2 seconds) . A peak current and a peak time of transient current generated by the applied rectangular waves were measured. A value of rotational viscosity was obtained from the measured values and equation (8) described on page 40 of the paper presented by M. Imai et al. A value of dielectric anisotropy required for the calculation was determined by using the device by which the rotational viscosity was measured according to a method described below.
(8) Optical anisotropy (refractive index anisotropy; measured at 25°C; Δn): Measurement was carried out by an Abbe refractometer with a polarizing plate mounted on an ocular, using light at a wavelength of 589 nanometers . A surface of a main prism was rubbed in one direction, and then a sample was added dropwise onto the main prism. A refractive index (n||) was measured when a direction of polarized light was parallel to a direction of rubbing. A refractive index (n⊥) was measured when the direction of polarized light was perpendicular to the direction of rubbing. A value of optical anisotropy (Δn) was calculated from an equation: Δn = n|| - n⊥.
(9) Dielectric anisotropy (Δε; measured at 25°C) : A sample was put in a TN device in which a distance (cell gap) between two glass substrates was 9 micrometers and a twist angle was 80 degrees. Sine waves (10 V, 1 kHz) were applied to the device, and after 2 seconds, a dielectric constant (ε||) of liquid crystal molecules in a major axis direction was measured. Sine waves (0.5 V, 1 kHz) were applied to the device, and after 2 seconds, a dielectric constant (ε⊥) of liquid crystal molecules in a minor axis direction was measured. A value of dielectric anisotropy was calculated from an equation: Δε = ε|| - ε⊥.
(10) Elastic constant (K; measured at 25°C; pN): For measurement, HP4284A LCR Meter made by Yokogawa-Hewlett-Packard Co. was used. A sample was put in a horizontal alignment device in which a distance (cell gap) between two glass substrates was 20 micrometers. An electric charge of 0 V to 20 V was applied to the device, and electrostatic capacity (C) and applied voltage (V) were measured. The measured values were fitted to equation (2.98) and equation (2.101) on page 75 of "Liquid Crystal Device Handbook" (Ekisho Debaisu Handobukku in Japanese; Nikkan Kogyo Shimbun, Ltd.) and values of K₁₁ and K₃₃ were obtained from equation (2.99) . Next, K₂₂ was calculated using the previously determined values of K₁₁ and K₃₃ in equation (3.18) on page 171. Elastic constant K was expressed in terms of a mean value of the thus determined K₁₁, K₂₂ and K₃₃.
(11) Threshold voltage (Vth; measured at 25°C; V): For measurement, an LCD-5100 luminance meter made by Otsuka Electronics Co., Ltd. was used. A light source was a halogen lamp. A sample was put in a normally white mode TN device in which a distance (cell gap) between two glass substrates was 0.45/Δn (µm) and a twist angle was 80 degrees. A voltage (32 Hz, rectangular waves) to be applied to the device was stepwise increased from 0 V to 10 V at an increment of 0.02 V. On the occasion, the device was irradiated with light from a direction perpendicular to the device, and an amount of light transmitted through the device was measured. A voltage-transmittance curve was prepared, in which the maximum amount of light corresponds to 100% transmittance and the minimum amount of light corresponds to 0% transmittance. A threshold voltage is expressed in terms of a voltage at 90% transmittance.
(12) Voltage holding ratio (VHR-1; measured at 25°C; %): A TN device used for measurement had a polyimide alignment film, and a distance (cell gap) between two glass substrates was 5 micrometers. A sample was put in the device, and then the device was sealed with an ultraviolet-curable adhesive. The device was charged by applying a pulse voltage (60 microseconds at 5 V) at 25°C. A decaying voltage was measured for 16.7 milliseconds with a high-speed voltmeter, and area A between a voltage curve and a horizontal axis in a unit cycle was determined. Area B is an area without decay. A voltage holding ratio is expressed in terms of a percentage of area A to area B.
(13) Voltage holding ratio (VHR-2; measured at 80°C; %) : A voltage holding ratio was measured by a method described above except that the voltage holding ratio was measured at 80°C in place of 25°C. The results were expressed in terms of a symbol VHR-2.
(14) Specific resistance (ρ; measured at 25°C; Ω cm) : Into a vessel equipped with electrodes, 1.0 milliliter of sample was injected. DC voltage (10 V) was applied to the vessel, and DC current after 10 seconds was measured. Specific resistance was calculated from the following equation: (specific resistance) = {(voltage) × (electric capacity of a vessel)} / {(direct current) × (dielectric constant of vacuum)}.
(15a) Response time (τ; measured at 25°C; ms)
   Positive dielectric anisotropy: For measurement, an LCD-5100 luminance meter made by Otsuka Electronics Co., Ltd. was used. A light source was a halogen lamp. A low-pass filter was set to 5 kHz. A sample was put in a normally white mode TN device in which a distance (cell gap) between two glass substrates was 5.0 micrometers and a twist angle was 80 degrees. A voltage (rectangular waves; 60 Hz, 5 V, 0.5 second) was applied to the device. On the occasion, the device was irradiated with light from a direction perpendicular to the device, and an amount of light transmitted through the device was measured. The maximum amount of light corresponds to 100% transmittance, and the minimum amount of light corresponds to 0% transmittance. A rise time (τr; millisecond) was expressed in terms of time required for a change from 90% transmittance to 10% transmittance. A fall time (τf; millisecond) was expressed in terms of time required for a change from 10% transmittance to 90% transmittance. A response time was expressed by a sum of the rise time and the fall time thus obtained.
(15b) Response time (τ; measured at 25°C; ms)
   Negative dielectric anisotropy: For measurement, an LCD-5100 luminance meter made by Otsuka Electronics Co., Ltd. was used. A light source was a halogen lamp. A low-pass filter was set to 5 kHz. A sample was put in a normally black mode PVA device in which a distance (cell gap) between two glass substrates was 3.2 micrometers and a rubbing direction was anti-parallel. The device was sealed with an ultraviolet-curable adhesive. Voltage having a degree of slightly exceeding threshold voltage was applied to the device for 1 minute, and then the device was irradiated with ultraviolet light of 23.5 mW/cm² for 8 minutes while voltage of 5.6V was applied to the device. A voltage (rectangular waves; 60 Hz, 10 V, 0.5 second) was applied to the device. On the occasion, the device was irradiated with light from a direction perpendicular to the device, and an amount of light transmitted through the device was measured. The maximum amount of light corresponds to 100% transmittance, and the minimum amount of light corresponds to 0% transmittance. A response time was expressed in terms of time required for a change from 90% transmittance to 10% transmittance (fall time; millisecond).
(16) Flicker rate (measured at 25°C; %): For measurement, 3298F Multimedia Display Tester made by Yokogawa Electric Corporation was used. A light source was LED. A sample was put in a normally black mode FFS device in which a distance (cell gap) between two glass substrates was 3.5 micrometers and a rubbing direction was anti-parallel. The device was sealed with an ultraviolet-curable adhesive. Voltage was applied to the device, and a voltage having a maximum amount of light transmitted through the device was measured. A flicker rate displayed thereon was read by bringing a sensor unit close to the device while voltage was applied to the device.

Raw material: Solmix (registered trade name) A-11 is a mixture of ethanol (85.5%), methanol (13.4%) and isopropanol (1.1%), and was purchased from Japan Alcohol Trading Co., Ltd.

### Synthesis Example 1

### Synthesis of compound (No. 1-1-14)

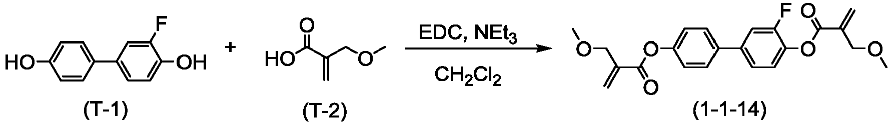

### First step

Under a nitrogen atmosphere, compound (T-1) (3 g, 14.69 mmol) prepared according to a publicly-known method and dichloromethane (300 mL) were put in a reaction vessel, and the resulting mixture was cooled down to 0°C. Compound (T-2) (5.63 g, 48.48 mmol) prepared according to a publicly-known method, 1-(3-dimethylaminopropyl)-3-ethylcarbodiimide (9.29 g, 48.48 mmol) and triethylamine (9.01 mL, 64.64 mmol) were added thereto, and the resulting mixture was heated to room temperature. The resulting reaction mixture was poured into water, and an aqueous layer was subjected to extraction with ethyl acetate. Combined organic layers were washed with water, and dried over anhydrous magnesium sulfate. The solution was concentrated under reduced pressure, and the residue was purified by silica gel column chromatography (dichloromethane / ethyl acetate = 19 / 1 in a volume ratio) and recrystallization to obtain compound (1-1-14) (4.13 g, yield: 70%).
¹H-NMR (ppm; CDCl₃): δ 7.81 - 7.76 (m, 3H), 7.60 (d, J = 8.3 Hz, 1H), 7.45 (t, J = 8.4 Hz, 1H), 7.30 (d, J = 8.4 Hz, 1H), 6.54 (s, 1H), 6.48 (s, 1H), 6.15 (s, 1H), 6.08 (s, 1H), 4.21 (s, 4H), 3.36 (s, 6H).
Transition temperature: C 105.3 I.

### Synthesis Example 2

### Synthesis of compound (No. 1-1-35)

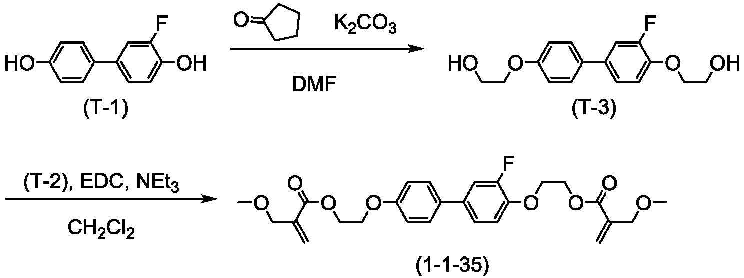

### First step

Under a nitrogen atmosphere, compound (T-1) (8 g, 39.18 mmol), ethylene carbonate (7.59 g, 86.19 mmol), potassium carbonate (16.24 g, 117.53 mmol) and dimethylformamide (100 mL) were put in a reaction vessel, and the resulting mixture was heated and refluxed. The resulting reaction mixture was poured into water, and an aqueous layer was subjected to extraction with ethyl acetate. Combined organic layers were washed with water, and dried over anhydrous magnesium sulfate. The solution was concentrated under reduced pressure, and the residue was purified by silica gel column chromatography (toluene / ethyl acetate = 4 / 1 in a volume ratio) to obtain compound (T-3) (3.8 g, yield: 33%).

### Second step

Under a nitrogen atmosphere, compound (T-3) (3.8 g, 13.0 mmol) prepared according to a publicly-known method and dichloromethane (300 mL) were put in a reaction vessel, and the resulting mixture was cooled down to 0°C. Compound (T-2) (3.32 g, 28.6 mmol) prepared according to a publicly-known method, 1-(3-dimethylaminopropyl)-3-ethylcarbodiimide (5.48 g, 28.6 mmol) and triethylamine (5.98 mL, 42.9 mmol) were added thereto, and the resulting mixture was heated to room temperature. The resulting reaction mixture was poured into water, and an aqueous layer was subjected to extraction with ethyl acetate. Combined organic layers were washed with water, and dried over anhydrous magnesium sulfate. The solution was concentrated under reduced pressure, and the residue was purified by silica gel column chromatography (dichloromethane / ethyl acetate = 19 / 1 in a volume ratio) and recrystallization to obtain compound (1-1-35) (4.5 g, yield: 71%).
¹H-NMR (ppm; CDCl₃) : δ 7.45 (d, J = 6.7 Hz, 2H), 7.29 - 7.23 (m, 2H), 7.04 (t, J = 8.7 Hz, 1H), 6.97 (d, J = 8.7 Hz, 2H), 6.38 (s, 2H), 5.90 (s, 2H), 4.56 - 4.53 (m, 4H), 4.35 - 4.33 (m, 2H), 4.28 - 4.26 (m, 2H), 4.15 - 4.14 (m, 4H), 3.38 (s, 6H).
Transition temperature: C 71.5 I.

### Synthesis Example 3

### Synthesis of compound (No. 1-1-46)

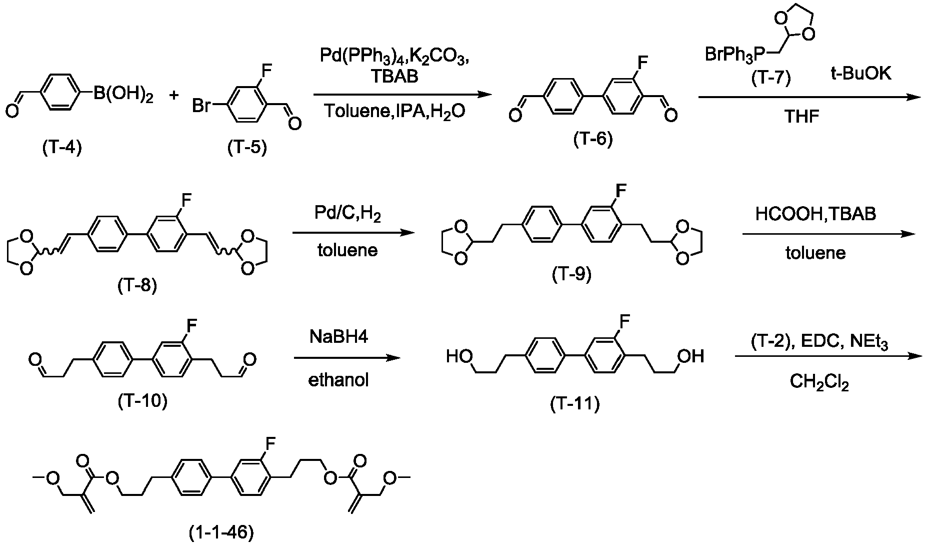

### First step

Under a nitrogen atmosphere, compound (T-4) (22.2 g, 147.78 mmol), compound (T-5) (25 g, 123.15 mmol), tetrakis(triphenylphosphine)palladium (4.27 g, 3.69 mmol), potassium carbonate (34.0 g, 246.29 mmol), tetrabutylammonium bromide (TBAB) (11.91 g, 36.94 mmol), toluene (100 mL), IPA (100 mL) and pure water (50 mL) were put in a reaction vessel, and the resulting mixture was heated and refluxed. The resulting reaction mixture was poured into water, and an aqueous layer was subjected to extraction with toluene. Combined organic layers were washed with water, and dried over anhydrous magnesium sulfate. The solution was concentrated under reduced pressure, and the residue was purified by silica gel column chromatography (heptane / ethyl acetate = 9 / 1 in a volume ratio) to obtain compound (T-6) (14.8 g, yield: 53%).

### Second step:

A THF (200 mL) solution of compound (T-7) (28.22 g, 65.73 mmol) was cooled down to -70°C, potassium t-butoxide (5.41 g, 48.2 mmol) was added dropwise, and the resulting mixture was stirred for 1 hour. A THF (100 mL) solution of compound (T-6) (5 g, 21.91 mmol) was added dropwise thereto, and the resulting mixture was returned to room temperature while being stirred. The resulting reaction mixture was poured into water, ordinary post-treatment was performed, and the residue was purified by silica gel chromatography (toluene) to obtain compound (T-8) (8.4 g, quantitative).

### Third step

Compound (T-8) (8.4 g, 22.80 mmol), Pd/C (0.84 g) and toluene (200 mL) were put in a reaction vessel, and the resulting mixture was stirred for 10 hours under a hydrogen atmosphere. The resulting reaction mixture was poured into water, and an aqueous layer was subjected to extraction with toluene. Combined organic layers were washed with water, and dried over anhydrous magnesium sulfate. The solution was concentrated under reduced pressure, and the residue was purified by silica gel chromatography (toluene) to obtain compound (T-9) (8.5 g; yield: 93%).

### Fourth step:

Then, 87% of formic acid (85 mL) was added to a toluene (85 mL) solution of compound (T-9) (8.5 g, 22.82 mmol) and TBAB (0.22 g, 0.68 mmol), and the resulting mixture was stirred at room temperature. Ordinary post-treatment was performed to obtain compound (T-10) (5.54 g, 19.23 mmol, yield: 84%).

### Fifth step:

An ethanol (100 mL) solution of sodium borohydride (0.73 g, 19.48 mmol) was cooled down to 0°C, an ethanol (10 mL) solution of compound (T-10) (5.54 g, 19.48 mmol) was added dropwise, and the resulting mixture was heated to room temperature. The resulting reaction mixture was poured into an aqueous solution of ammonium chloride, ordinary post-treatment was performed, and purification by silica gel chromatography (toluene / ethyl acetate = 2 / 1 in a volume ratio) was performed to obtain compound (T-11) (3.7 g, yield: 66%).

### Sixth step

Under a nitrogen atmosphere, compound (T-11) (3.7 g, 12.83 mmol) and dichloromethane (300 mL) were put in a reaction vessel, and the resulting mixture was cooled down to 0 °C. Compound (T-2) (4.92 g, 42.34 mmol) prepared according to a publicly-known method, 1-(3-dimethylaminopropyl)-3-ethylcarbodiimide (8.12 g, 42.34 mmol) and triethylamine (7.87 mL, 56.46 mmol) were added thereto, and the resulting mixture was heated to room temperature. The resulting reaction mixture was poured into water, and an aqueous layer was subjected to extraction with ethyl acetate. Combined organic layers were washed with water, and dried over anhydrous magnesium sulfate. The solution was concentrated under reduced pressure, and the residue was purified by silica gel column chromatography (dichloromethane / ethyl acetate = 9 / 1 in a volume ratio) and recrystallization to obtain compound (1-1-46) (2.4 g, yield: 38%).
¹H-NMR (ppm; CDCl₃) : δ 7.48 (d, J = 8.2 Hz, 2H), 7.29 - 7.21 (m, 5H), 6.30 (s, 2H), 5.86 - 5.85 (m, 2H), 4.24 - 4.21 (m, 4H), 4.14 (m, 4H), 3.41 (s, 6H), 2.80 - 2.74 (m, 4H), 2.08 - 2.03 (m, 4H).
Transition temperature: C -0.7 C 12.1 I.

### Synthesis Example 4

### Synthesis of compound (No. 1-2-41)

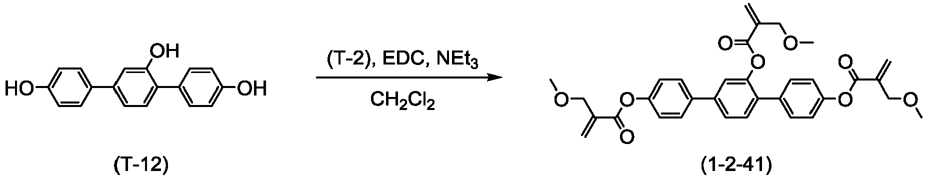

### First step

Under a nitrogen atmosphere, compound (T-12) (5.2 g, 18.68 mmol) prepared according to a publicly-known method and dichloromethane (300 mL) were put in a reaction vessel, and the resulting mixture was cooled down to 0°C. Compound (T-2) (10.41 g, 89.69 mmol) prepared according to a publicly-known method, 1-(3-dimethylaminopropyl)-3-ethylcarbodiimide (17.19 g, 89.69 mmol) and triethylamine (17.19 mL, 123.32 mmol) were added thereto, and the resulting mixture was heated to room temperature. The resulting reaction mixture was poured into water, and an aqueous layer was subjected to extraction with ethyl acetate. Combined organic layers were washed with water, and dried over anhydrous magnesium sulfate. The solution was concentrated under reduced pressure, and the residue was purified by silica gel column chromatography (dichloromethane / ethyl acetate = 19 / 1 in a volume ratio) and recrystallization to obtain compound (1-2-41) (4.6 g, yield: 43%).
¹H-NMR (ppm; CDCl₃) : δ 7.82 (d, J = 8.5 Hz, 2H), 7.74 (m, 1H), 7.63 - 7.57 (m, 4H), 7.34 - 7.27 (m, 4H), 6.50 (d, J = 6.9 Hz, 2H), 6.38 (s, 1H), 6.07 (d, J = 4.3 Hz, 2H), 5.98 (s, 1H), 4.25 - 4.24 (m, 4H), 4.11 (s, 2H), 3.42 - 3.34 (m, 9H).
Transition temperature: C 90.8 I.

### Synthesis Example 5

### Synthesis of compound (No. 1-2-47)

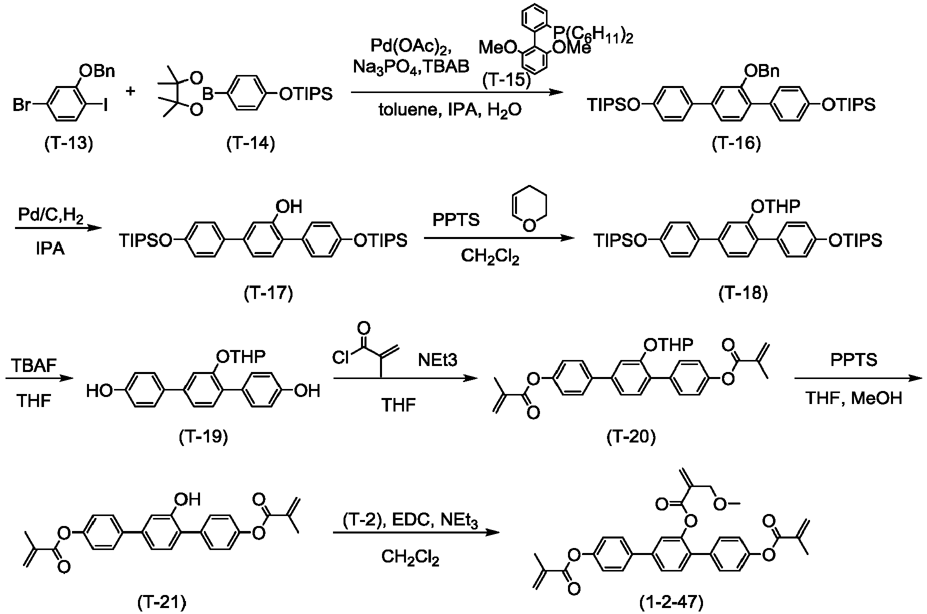

### First step

Under a nitrogen atmosphere, compound (T-13) (10.0 g, 35.91 mmol), compound (T-14) (32.72 g, 86.91 mmol), palladium acetate (0.044 g, 0.20 mmol), compound (T-15) (2-dicyclohexylphosphino-2',6'-dimethoxybiphenyl) (0.16 g, 0.01 mmol), trisodium phosphate (60.01 g, 0.40 mmol), TBAB (6.37 g, 19.75 mmol), toluene (50 mL), IPA (50 mL) and pure water (50 mL) were put in a reaction vessel, and the resulting mixture was heated and refluxed. The resulting reaction mixture was poured into water, and an aqueous layer was subjected to extraction with toluene. Combined organic layers were washed with water, and dried over anhydrous magnesium sulfate. The solution was concentrated under reduced pressure, and the residue was purified by silica gel column chromatography (toluene / ethyl acetate = 9 / 1 in a volume ratio) and recrystallization to obtain compound (T-16) (21.2 g, yield: 79%).

### Second step

Compound (T-16) (21.2 g, 31.13 mmol), Pd/C (1.06 g) and IPA (200 mL) were put in a reaction vessel, and the resulting mixture was stirred for 10 hours under a hydrogen atmosphere. The resulting reaction mixture was poured into water, and an aqueous layer was subjected to extraction with toluene. Combined organic layers were washed with water, and dried over anhydrous magnesium sulfate. The solution was concentrated under reduced pressure, and the residue was purified by silica gel chromatography (toluene / ethyl acetate = 10 / 1 in a volume ratio) to obtain compound (T-17) (16.2 g; 88%).

### Third step

Compound (T-17) (20 g, 33.84 mmol), pyridinium p-toluenesulfonate (2 g, 7.96 mmol), 3,4-dihydro-2H-pyran (5.69 g, 67.68 mmol) and dichloromethane (100 mL) were put in a reaction vessel, and the resulting mixture was stirred at room temperature. The resulting reaction mixture was poured into sodium hydrogencarbonate water, and an aqueous layer was subjected to extraction with toluene. Combined organic layers were washed with water, and dried over anhydrous magnesium sulfate. The solution was concentrated under reduced pressure, and the residue was purified by silica gel column chromatography (toluene / ethyl acetate = 9 / 1 in a volume ratio) to obtain compound (T-18) (18.65 g, yield: 82%).

### Fourth step

Compound (T-18) (18 g, 26.66 mmol) and THF (90 mL) were put in a reaction vessel, and the resulting mixture was cooled down to 0°C while being stirred. Tetrabutylammonium fluoride (TBAF) (20.9 g) was added dropwise, and the resulting mixture was stirred for 3 hours while raising temperature to room temperature. The resulting reaction mixture was poured into water, and an aqueous layer was subjected to extraction with ethyl acetate. Combined organic layers were washed with water, and dried over anhydrous magnesium sulfate. The solution was concentrated under reduced pressure, and the residue was purified by silica gel chromatography (toluene : ethyl acetate = 2 : 1 in a volume ratio) to obtain compound (T-19) (10.58 g, quantitative).

### Fifth step

Compound (T-19) (7.5 g, 20.69 mmol), triethylamine (6.28 g, 62.08 mmol) and THF (250 mL) were put in a reaction vessel, and the resulting mixture was cooled down to 0°C while being stirred. Methacryloyl chloride (5.40 g, 51.73 mmol) was added dropwise thereto, and the resulting mixture was stirred all night at room temperature. The resulting reaction mixture was poured into water, and an aqueous layer was subjected to extraction with toluene. Combined organic layers were washed with water, and dried over anhydrous magnesium sulfate. The solution was concentrated under reduced pressure, and the residue was purified by silica gel chromatography (toluene : ethyl acetate = 9 : 1 in a volume ratio) to obtain compound (T-20) (9.1 g, yield: 88%).

### Sixth step

Compound (T-20) (9 g, 18.05 mmol), pyridinium p-toluenesulfonate (0.9g, 3.61 mmol), THF (90 mL) and methanol (90 mL) were put in a reaction vessel, and the resulting mixture was heated and stirred at 40°C. The resulting reaction mixture was poured into water, and an aqueous layer was subjected to extraction with ethyl acetate. Combined organic layers were washed with water, and dried over anhydrous magnesium sulfate. The solution was concentrated under reduced pressure, and the residue was purified by silica gel column chromatography (dichloromethane / ethyl acetate = 9 / 1 in a volume ratio) to obtain compound (T-21) (6.3 g, yield: 84%).

### Seventh step

Under a nitrogen atmosphere, compound (T-21) (2.7 g, 6.51 mmol) and dichloromethane (300 mL) were put in a reaction vessel, and the resulting mixture was cooled down to 0°C. Compound (T-2) (1.21 g, 10.42 mmol) prepared according to a publicly-known method, 1-(3-dimethylaminopropyl)-3-ethylcarbodiimide (2.0 g, 10.42 mmol) and triethylamine (2.0 ml, 14.33 mmol) were added thereto, and the resulting mixture was heated to room temperature. The resulting reaction mixture was poured into water, and an aqueous layer was subjected to extraction with dichloromethane. Combined organic layers were washed with water, and dried over anhydrous magnesium sulfate. The solution was concentrated under reduced pressure, and the residue was purified by silica gel column chromatography (toluene / ethyl acetate = 19 / 1 in a volume ratio) and recrystallization to obtain compound (1-2-47) (2.7 g, yield: 81%).
¹H-NMR (ppm; CDCl₃): δ 7.82 - 7.81 (m, 2H), 7.74 - 7.73 (m, 1H), 7.63 - 7.56 (m, 4H), 7.33 - 7.26 (m, 4H), 6.39 (s, 1H), 6.33 (d, J = 6.9 Hz, 2H), 5.98 (s, 1H), 5.88 - 5.86 (m, 2H), 4.11 (s, 2H), 3.34 (s, 3H), 2.85 (s, 6H).
Transition temperature: C 68.2 C 79.9 I.

### Synthesis Example 6

### Synthesis of compound (No. 1-2-94)

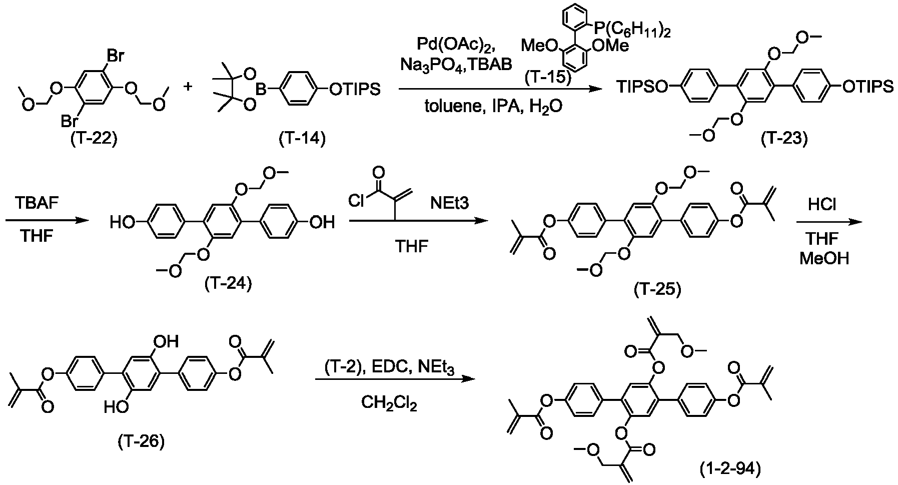

### First step

Under a nitrogen atmosphere, compound (T-22) (10.0 g, 28.09 mmol), compound (T-14) (22.2 g, 59.00 mmol), palladium acetate (0.03 g, 0.14 mmol), compound (T-15) (2-dicyclohexylphosphino-2',6'-dimethoxybiphenyl) (0.12 g, 0.28 mmol), trisodium phosphate (32.0 g, 84.3 mmol), TBAB (4.5 g, 14.04 mmol), toluene (50 mL), IPA (50 mL) and pure water (50 mL) were put in a reaction vessel, and the resulting mixture was heated and refluxed. The resulting reaction mixture was poured into water, and an aqueous layer was subjected to extraction with toluene. Combined organic layers were washed with water, and dried over anhydrous magnesium sulfate. The solution was concentrated under reduced pressure, and the residue was purified by silica gel column chromatography (toluene in a volume ratio) and recrystallization to obtain compound (T-23) (19.1 g, yield: 98%) .

### Second step

Compound (T-23) (5.0 g, 7.19 mmol) and THF (50 mL) were put in a reaction vessel, and the resulting mixture was cooled down to 0 °C. TBAF (28.8 mL, 28.8 mmol) was added dropwise thereto, and the resulting mixture was stirred for 2 hours. The resulting reaction mixture was poured into water, and an aqueous layer was subjected to extraction with ethyl acetate. An organic layer was washed with water, and dried over anhydrous magnesium sulfate. The solution was concentrated under reduced pressure, and the residue was purified by silica gel chromatography (toluene / ethyl acetate = 5 / 1 in a volume ratio) to obtain compound (T-24) (2.5 g; 91%).

### Third step

Compound (T-25) (4.3 g, yield: 45%) was obtained by using compound (T-24) (7 g, 18.3 mmol) as a raw material in a manner similar to the technique in the fifth step in Synthesis Example 5.

### Fourth step

Compound (T-25) (4.3 g, 8.3 mmol), 6 N hydrochloric acid (24.9 mL), THF (43 mL) and methanol (43 mL) were put in a reaction vessel, and the resulting mixture was heated and stirred at 50°C for 3 hours. The resulting reaction mixture was poured into water, and an aqueous layer was subjected to extraction with ethyl acetate. The resulting organic layer was washed with water, and dried over anhydrous magnesium sulfate . The solution was concentrated under reduced pressure to obtain compound (T-26) (3.3 g, yield: 92%) .

### Fifth step

Compound (1-2-94) (0.5 g, yield: 11%) was obtained by using compound (T-26) (3.3 g, 7.7 mmol) as a raw material in a manner similar to the technique in the seventh step in Synthesis Example 5.
¹H-NMR (ppm; CDCl₃): δ 7.51 - 7.50 (m, 4H), 7.31 - 7.29 (m, 2H), 7.20 - 7.18 (m, 4H), 6.42 (s, 2H), 6.39 (s, 2H), 5.99 (s, 2H), 5.80 (s, 2H), 4.12 (s, 4H), 3.39 (s, 6H), 2.10 (s, 6H) .
Transition temperature: C 160.2 I.

### Comparative Example 1

### Comparison of polymerization starting temperature

Compound (S-1) described below was selected as a comparative compound. The compound was prepared according to a publicly-known method. 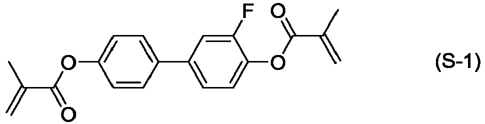 ¹H-NMR (ppm; CDCl₃): δ 7.57 (d, J = 8.6 Hz, 2H), 7.39 - 7.34 (m, 2H), 7.24 - 7.19 (m, 3H), 6.40 (d, J = 18.6 Hz, 2H), 5.80 (dt, J = 17.3 Hz, J = 1.6 Hz, 2H), 2.10 (s, 3H), 2.08 (s, 3H).
Transition temperature: C 183.6 I.

**Table 1. Polymerization starting temperature**

| **Structure** | **Polymerization starting temperature (°C)** |
|---|---|
| 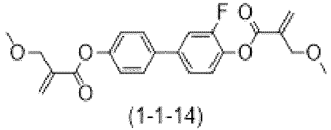 | 111.5 |
| 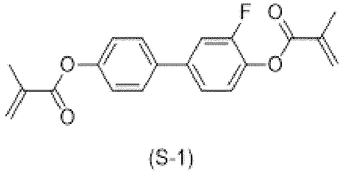 | 144.7 |

Polymerization starting temperatures of compound (1-1-14) and comparative compound (S-1) were summarized in Table 1. Table 1 shows that compound (1-1-14) has low polymerization starting temperature and high reactivity.

### Comparative Example 2

### Comparison of polymerization starting temperature

Compound (S-2) described below was selected as a comparative compound. The compound was prepared according to a publicly-known method. 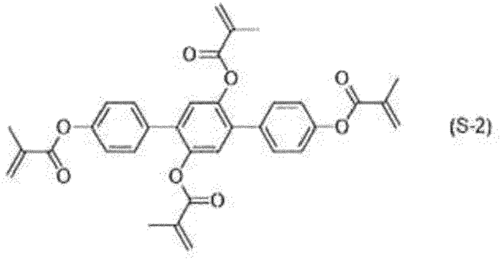 ¹H-NMR (ppm; CDCl₃): δ 7.50 - 7.48 (m, 4H), 7.27 - 7.26 (m, 2H), 7.18 - 7.16 (m, 4H), 6.37 (s, 2H), 6.19 (s, 2H), 5.77 (s, 2H), 5.66 (s, 2H), 2.08 (s, 6H), 1.93 (s, 6H).
Transition temperature: C 212.7 I.

**Table 2. Polymerization starting temperature**

| **Structure** | **Polymerization starting temperature (°C)** |
|---|---|
| 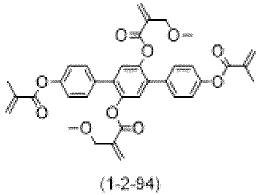 | 162.3 |
| 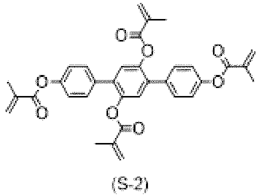 | 281.3 |

Polymerization starting temperatures of compound (1-2-94) and comparative compound (S-2) were summarized in Table 2. Table 2 shows that compound (1-2-94) has low polymerization starting temperature and high reactivity.

### 2. Synthesis of compound (1)

Compound (1) was prepared according to "2. Synthesis of compound (1) " and Synthesis Examples described above. Specific examples of such a compound include compounds (1-1-1) to (1-1-150), compounds (1-2-1) to (1-2-118) and compounds (1-3-1) to (1-3-36) as described below.

| No. | | No. | |
|---|---|---|---|
| 1-1-1 | 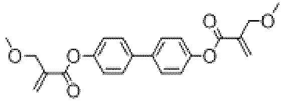 | 1-1-11 | 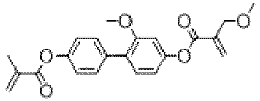 |
| 1-1-2 | 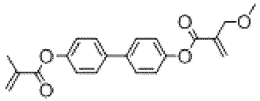 | 1-1-12 | 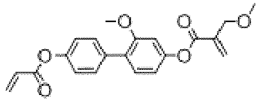 |
| 1-1-3 | 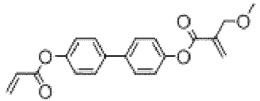 | 1-1-13 | 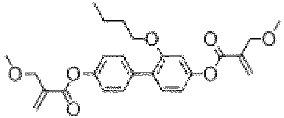 |
| 1-1-4 | 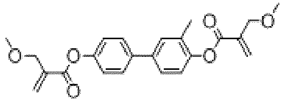 | 1-1-14 | 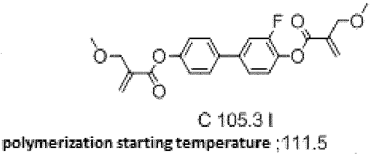 |
| 1-1-5 | 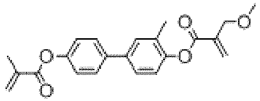 | 1-1-15 | 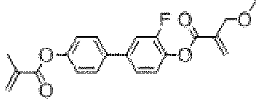 |
| 1-1-6 | 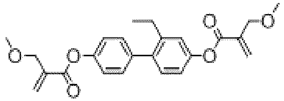 | 1-1-16 | 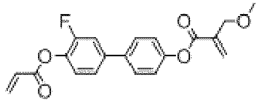 |
| 1-1-7 | 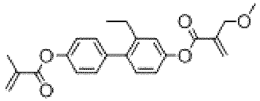 | 1-1-17 | 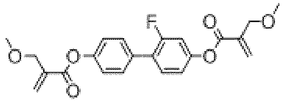 |
| 1-1-8 | 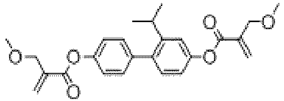 | 1-1-18 | 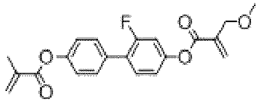 |
| 1-1-9 | 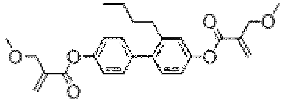 | 1-1-19 | 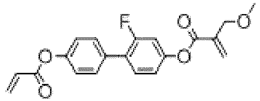 |
| 1-1-10 | 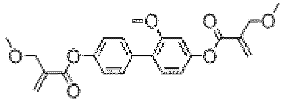 | 1-1-20 | 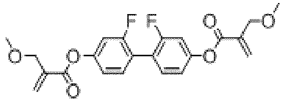 |
| 1-1-21 | 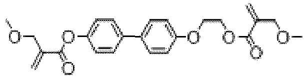 | 1-1-31 | 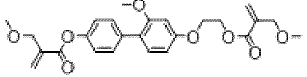 |
| 1-1-22 | 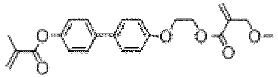 | 1-1-32 | 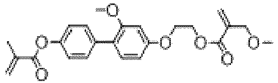 |
| 1-1-23 | 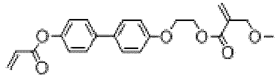 | 1-1-33 | 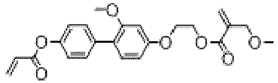 |
| 1-1-24 | 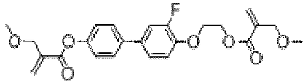 | 1-1-34 | 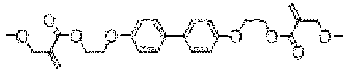 |
| 1-1-25 | 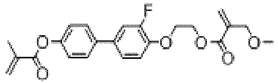 | 1-1-35 | 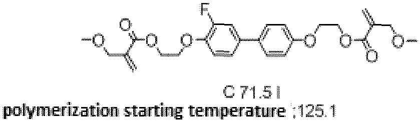 |
| 1-1-26 | 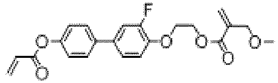 | 1-1-36 | 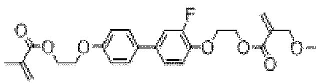 |
| 1-1-27 | 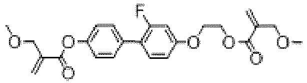 | 1-1-37 | 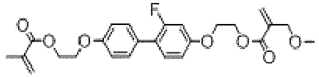 |
| 1-1-28 | 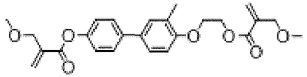 | 1-1-38 | 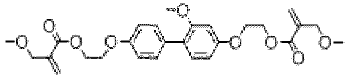 |
| 1-1-29 | 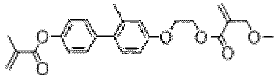 | 1-1-39 | 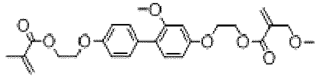 |
| 1-1-30 | 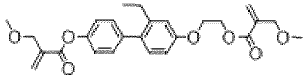 | 1-1-40 | 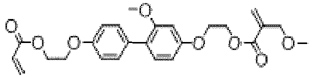 |
| 1-1-41 | 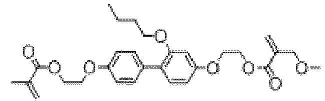 | 1-1-51 | 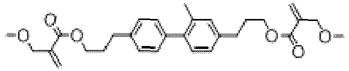 |
| 1-1-42 | 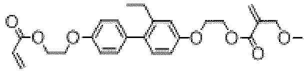 | 1-1-52 | 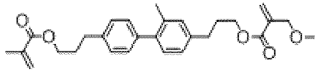 |
| 1-1-43 | 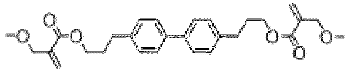 | 1-1-53 | 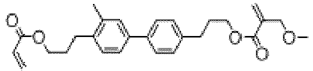 |
| 1-1-44 | 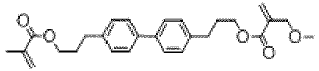 | 1-1-54 | 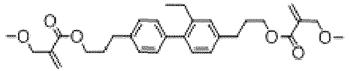 |
| 1-1-45 | 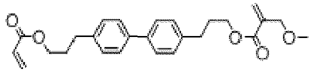 | 1-1-55 | 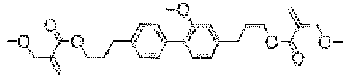 |
| 1-1-46 | 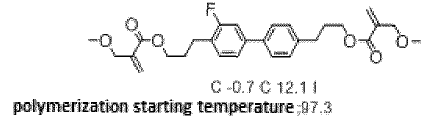 | 1-1-56 | 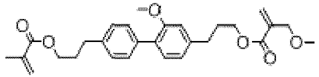 |
| 1-1-47 | 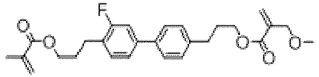 | 1-1-57 | 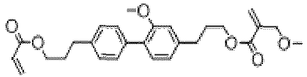 |
| 1-1-48 | 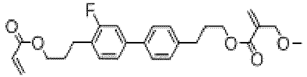 | 1-1-58 | 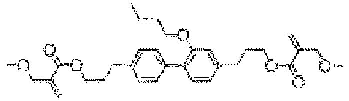 |
| 1-1-49 | 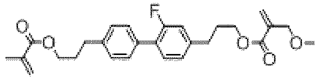 | 1-1-59 | 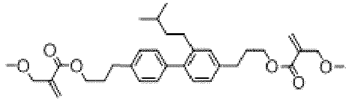 |
| 1-1-50 | 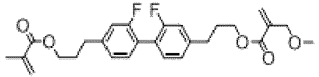 | 1-1-60 | 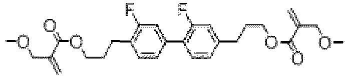 |
| 1-1-61 | 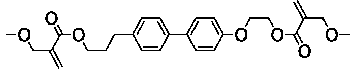 | 1-1-71 | 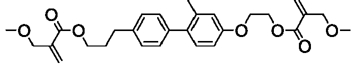 |
| 1-1-62 | 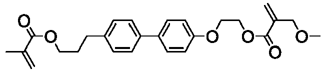 | 1-1-72 | 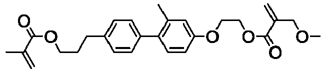 |
| 1-1-63 | 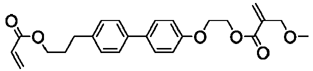 | 1-1-73 | 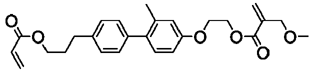 |
| 1-1-64 | 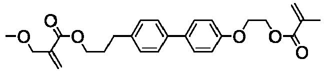 | 1-1-74 | 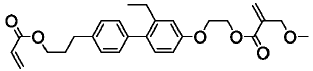 |
| 1-1-65 | 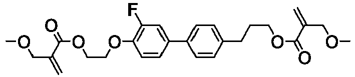 | 1-1-75 | 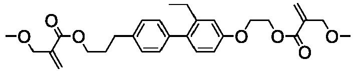 |
| 1-1-66 | 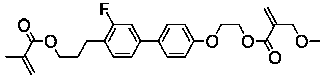 | 1-1-76 | 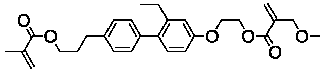 |
| 1-1-67 | 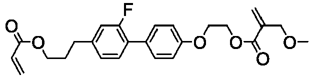 | 1-1-77 | 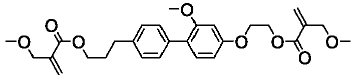 |
| 1-1-68 | 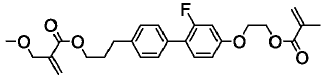 | 1-1-78 | 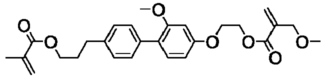 |
| 1-1-69 | 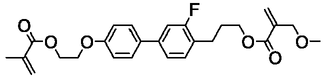 | 1-1-79 | 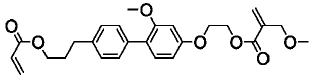 |
| 1-1-70 | 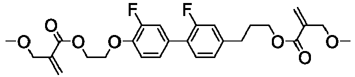 | 1-1-80 | 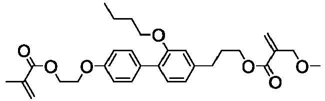 |
| 1-1-81 | 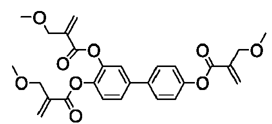 | 1-1-91 | 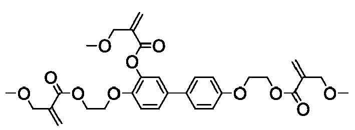 |
| 1-1-82 | 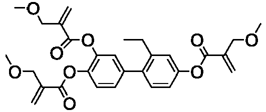 | 1-1-92 | 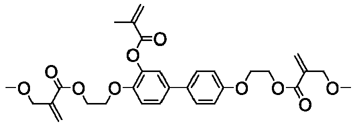 |
| 1-1-83 | 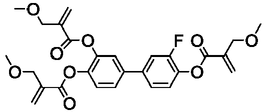 | 1-1-93 | 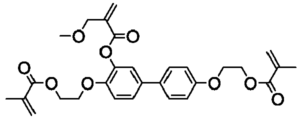 |
| 1-1-84 | 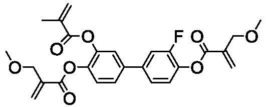 | 1-1-94 | 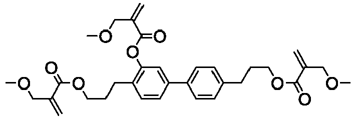 |
| 1-1-85 | 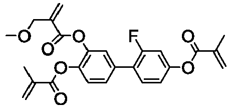 | 1-1-95 | 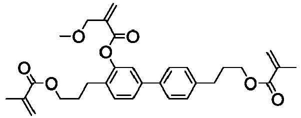 |
| 1-1-86 | 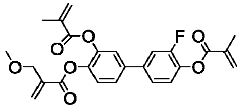 | 1-1-96 | 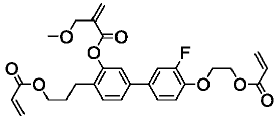 |
| 1-1-87 | 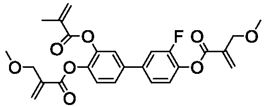 | 1-1-97 | 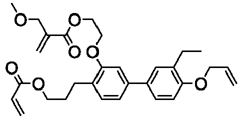 |
| 1-1-88 | 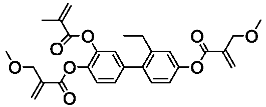 | 1-1-98 | 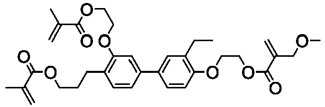 |
| 1-1-89 | 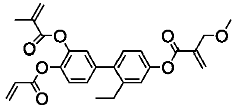 | 1-1-99 | 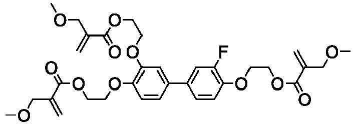 |
| 1-1-90 | 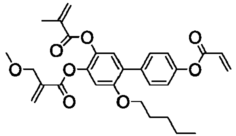 | 1-1-100 | 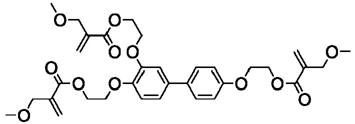 |
| 1-1-101 | 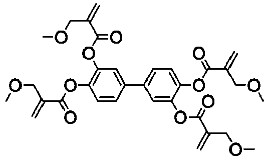 | 1-1-109 | 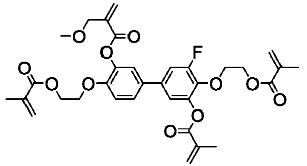 |
| 1-1-102 | 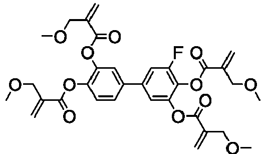 | 1-1-110 | 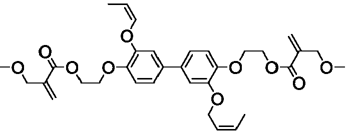 |
| 1-1-103 | 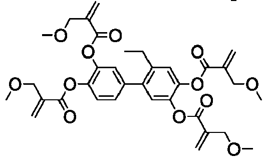 | 1-1-111 | 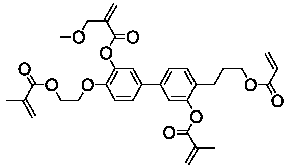 |
| 1-1-104 | 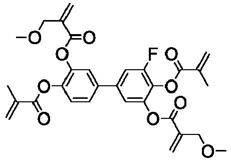 | 1-1-112 | 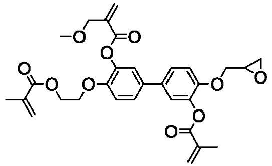 |
| 1-1-105 | 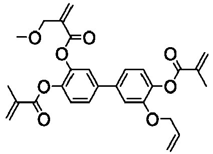 | 1-1-113 | 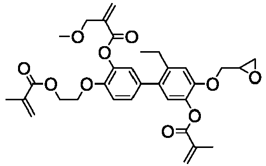 |
| 1-1-106 | 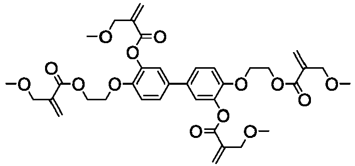 | 1-1-114 | 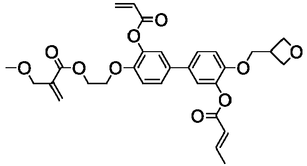 |
| 1-1-107 | 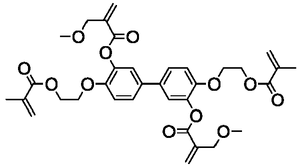 | 1-1-115 | 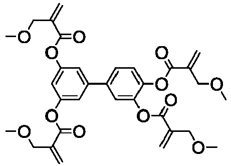 |
| 1-1-108 | 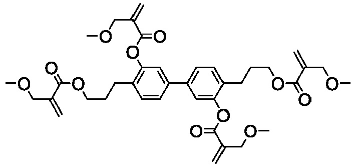 | 1-1-116 | 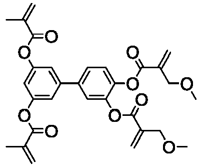 |
| 1-1-117 | 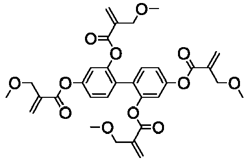 | 1-1-124 | 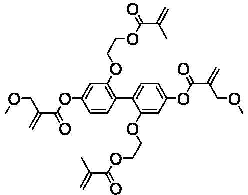 |
| 1-1-118 | 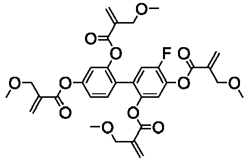 | 1-1-125 | 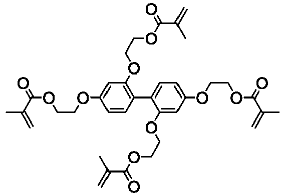 |
| 1-1-119 | 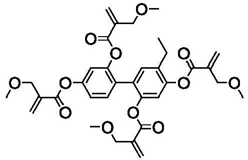 | 1-1-126 | 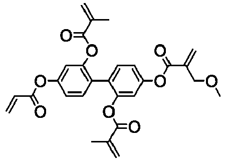 |
| 1-1-120 | 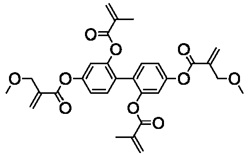 | 1-1-127 | 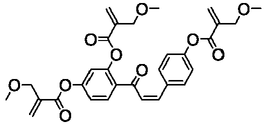 |
| 1-1-121 | 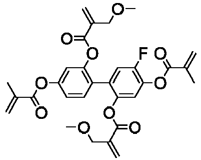 | 1-1-128 | 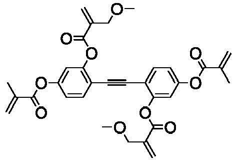 |
| 1-1-122 | 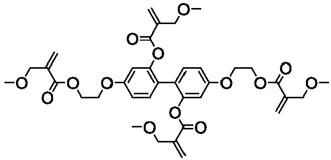 | 1-1-129 | 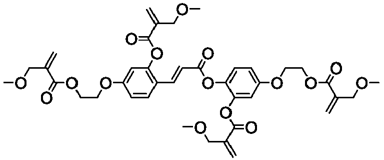 |
| 1-1-123 | 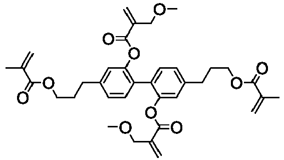 | 1-1-130 | 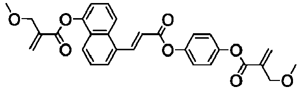 |
| 1-1-131 | 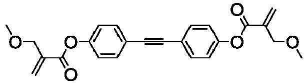 | 1-1-141 | 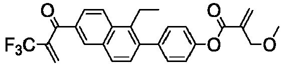 |
| 1-1-132 | 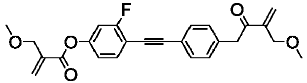 | 1-1-142 | 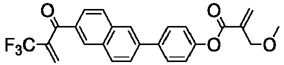 |
| 1-1-133 | 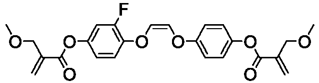 | 1-1-143 | 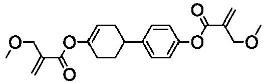 |
| 1-1-134 | 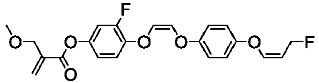 | 1-1-144 | 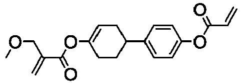 |
| 1-1-135 | 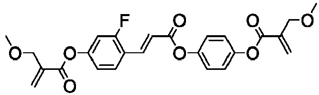 | 1-1-145 | 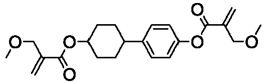 |
| 1-1-136 | 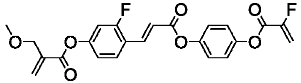 | 1-1-146 | 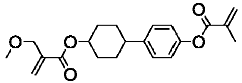 |
| 1-1-137 | 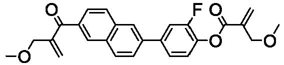 | 1-1-147 | 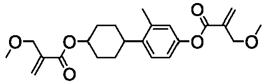 |
| 1-1-138 | 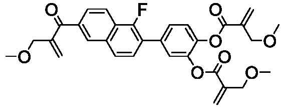 | 1-1-148 | 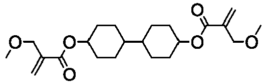 |
| 1-1-139 | 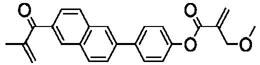 | 1-1-149 | 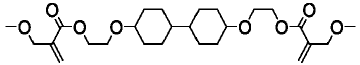 |
| 1-1-140 | 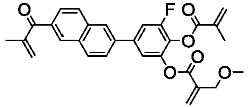 | 1-1-150 | 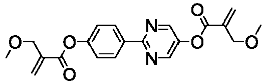 |
| 1-2-1 | 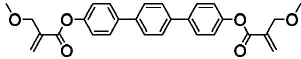 | 1-2-11 | 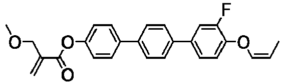 |
| 1-2-2 | 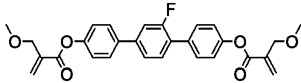 | 1-2-12 | 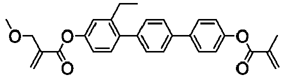 |
| 1-2-3 | 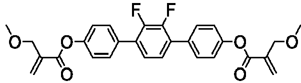 | 1-2-13 | 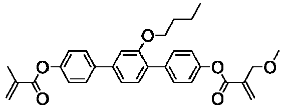 |
| 1-2-4 | 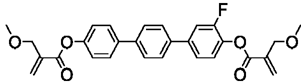 | 1-2-14 | 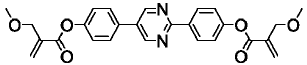 |
| 1-2-5 | 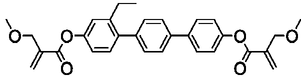 | 1-2-15 | 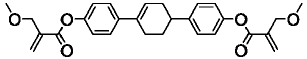 |
| 1-2-6 | 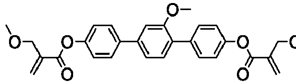 | 1-2-16 | 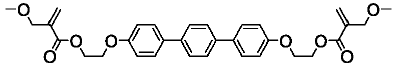 |
| 1-2-7 | 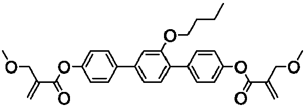 | 1-2-17 | 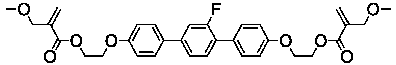 |
| 1-2-8 | 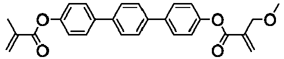 | 1-2-18 | 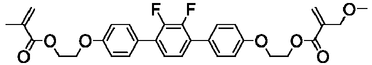 |
| 1-2-9 | 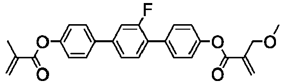 | 1-2-19 | 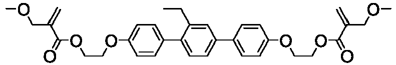 |
| 1-2-10 | 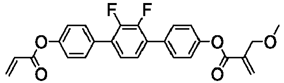 | 1-2-20 | 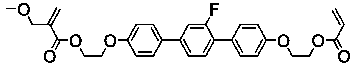 |
| 1-2-21 | 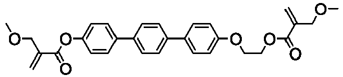 | 1-2-31 | 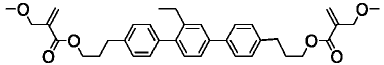 |
| 1-2-22 | 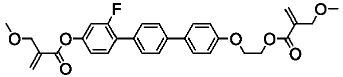 | 1-2-32 | 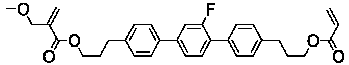 |
| 1-2-23 | 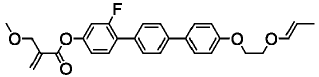 | 1-2-33 | 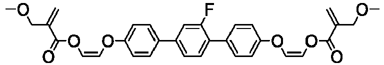 |
| 1-2-24 | 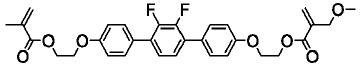 | 1-2-34 | 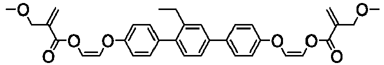 |
| 1-2-25 | 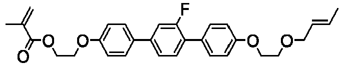 | 1-2-35 | 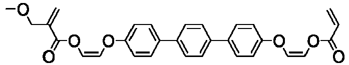 |
| 1-2-26 | 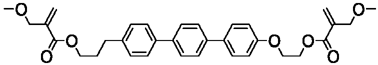 | 1-2-36 | 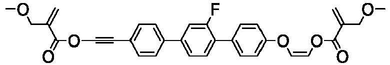 |
| 1-2-27 | 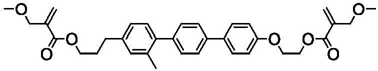 | 1-2-37 | 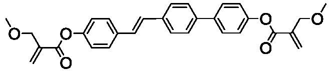 |
| 1-2-28 | 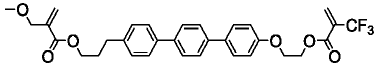 | 1-2-38 | 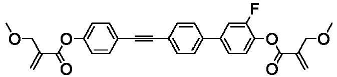 |
| 1-2-29 | 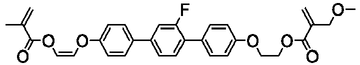 | 1-2-39 | 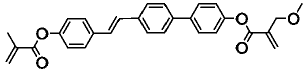 |
| 1-2-30 | 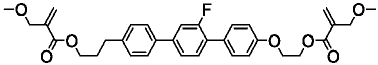 | 1-2-40 | 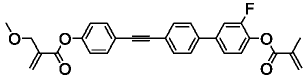 |
| 1-2-41 | 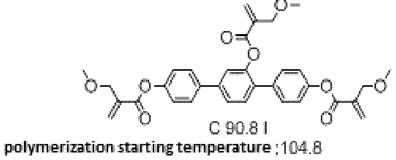 | 1-2-50 | 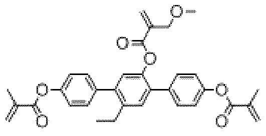 |
| 1-2-42 | 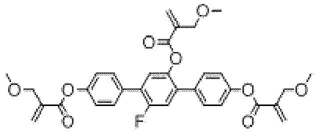 | 1-2-51 | 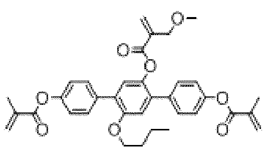 |
| 1-2-43 | 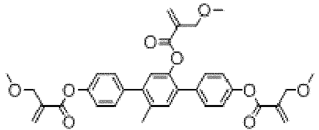 | 1-2-52 | 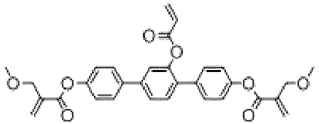 |
| 1-2-44 | 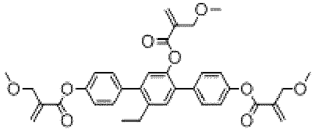 | 1-2-53 | 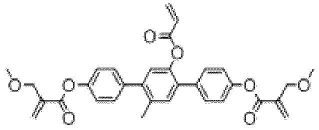 |
| 1-2-45 | 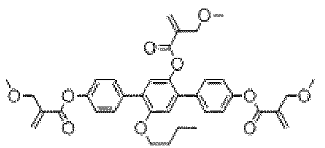 | 1-2-54 | 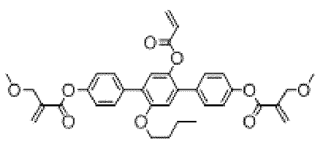 |
| 1-2-46 | 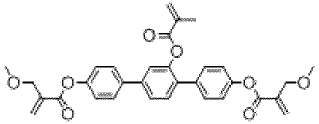 | 1-2-55 | 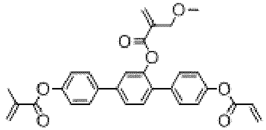 |
| 1-2-47 | 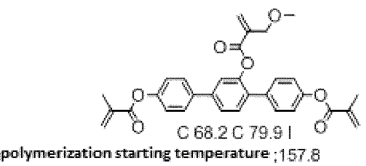 | 1-2-56 | 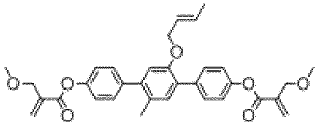 |
| 1-2-48 | 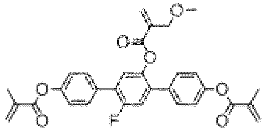 | 1-2-57 | 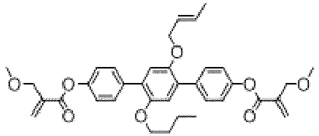 |
| 1-2-49 | 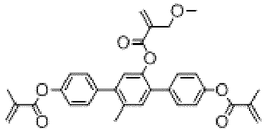 | 1-2-58 | 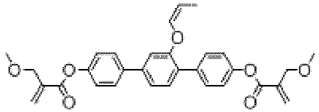 |
| 1-2-59 | 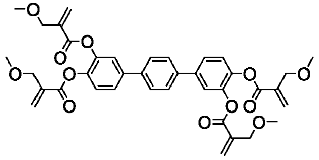 | 1-2-67 | 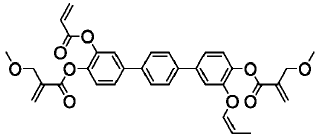 |
| 1-2-60 | 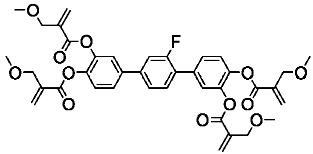 | 1-2-68 | 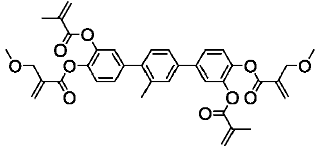 |
| 1-2-61 | 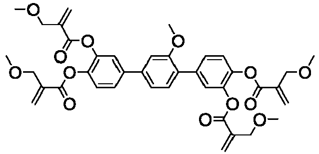 | 1-2-69 | 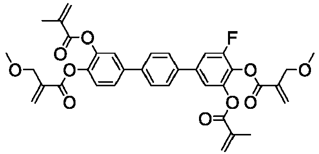 |
| 1-2-62 | 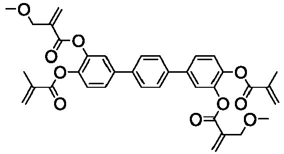 | 1-2-70 | 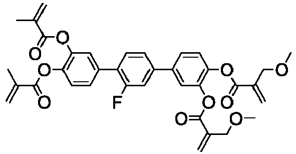 |
| 1-2-63 | 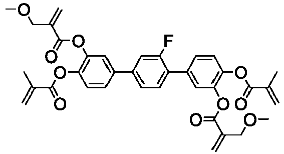 | 1-2-71 | 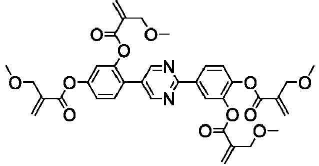 |
| 1-2-64 | 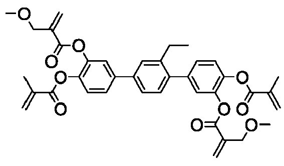 | 1-2-72 | 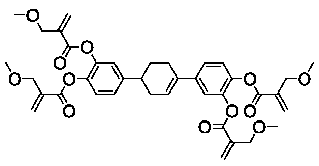 |
| 1-2-65 | 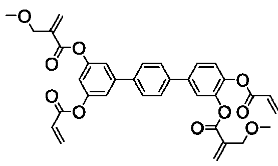 | 1-2-73 | 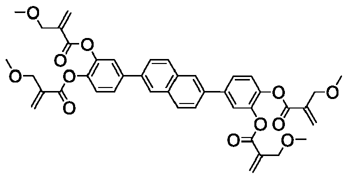 |
| 1-2-66 | 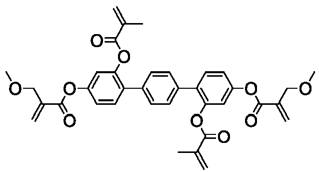 | 1-2-74 | 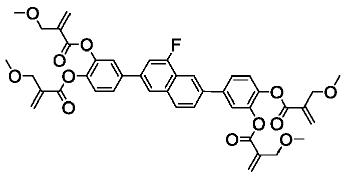 |
| 1-2-75 | 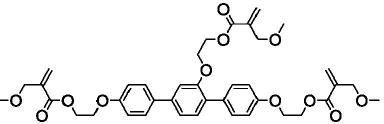 | 1-2-83 | 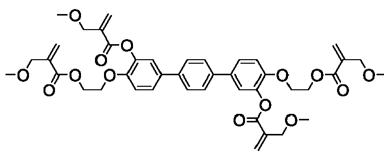 |
| 1-2-76 | 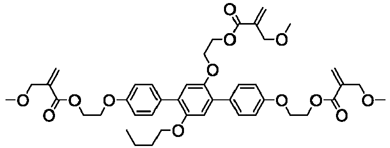 | 1-2-84 | 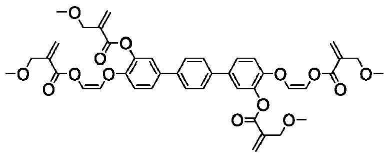 |
| 1-2-77 | 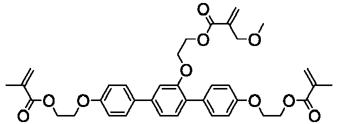 | 1-2-85 | 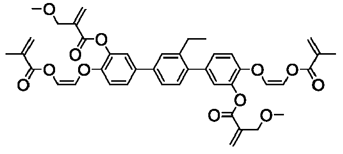 |
| 1-2-78 | 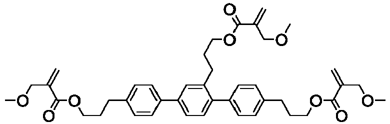 | 1-2-86 | 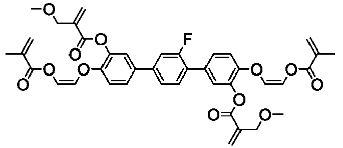 |
| 1-2-79 | 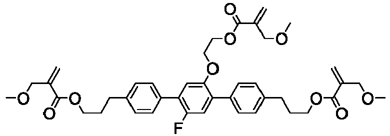 | 1-2-87 | 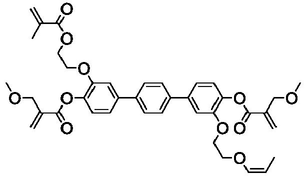 |
| 1-2-80 | 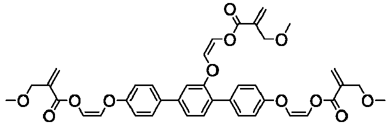 | 1-2-88 | 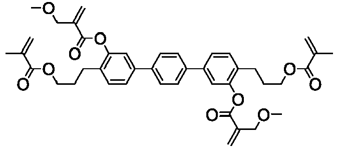 |
| 1-2-81 | 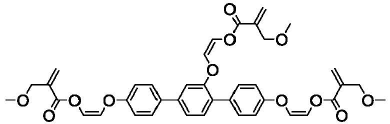 | 1-2-89 | 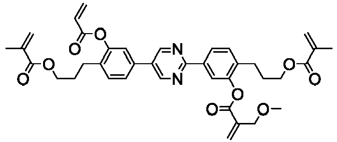 |
| 1-2-82 | 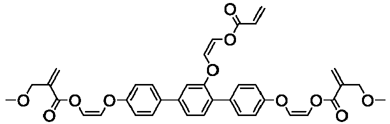 | 1-2-90 | 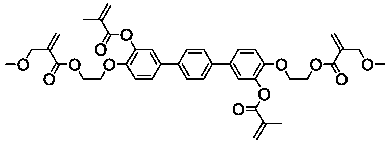 |
| 1-2-91 | 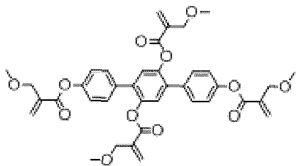 | 1-2-98 | 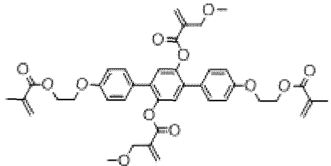 |
| 1-2-92 | 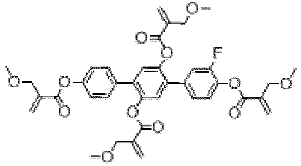 | 1-2-99 | 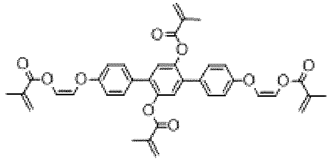 |
| 1-2-93 | 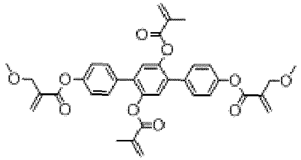 | 1-2-100 | 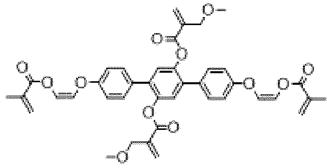 |
| 1-2-94 | 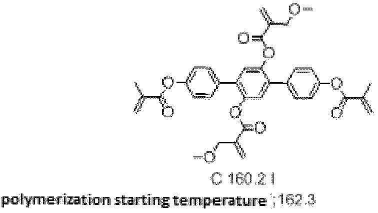 | 1-2-101 | 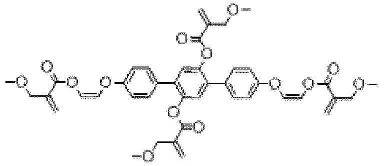 |
| 1-2-95 | 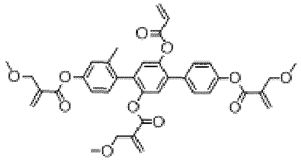 | 1-2-102 | 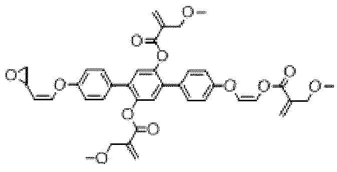 |
| 1-2-96 | 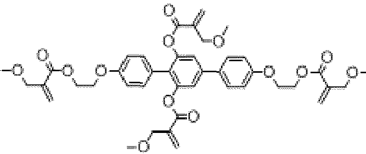 | 1-2-103 | 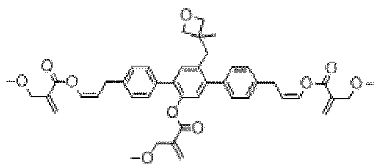 |
| 1-2-97 | 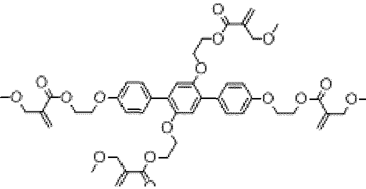 | 1-2-104 | 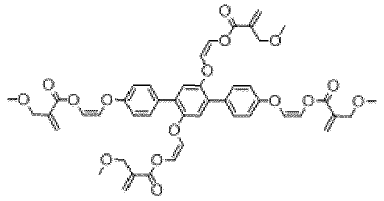 |
| **1-2-105** | 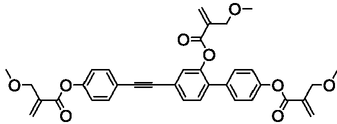 | **1-2-112** | 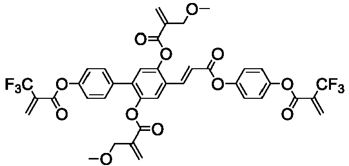 |
| **1-2-106** | 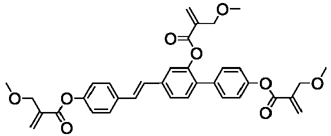 | **1-2-113** | 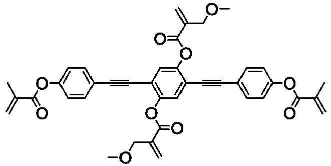 |
| **1-2-107** | 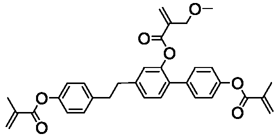 | **1-2-114** | 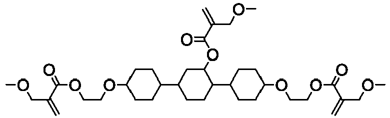 |
| **1-2-108** | 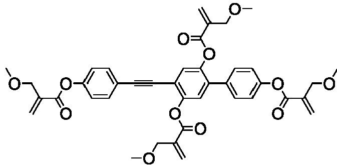 | **1-2-115** | 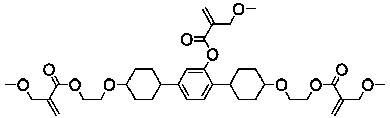 |
| **1-2-109** | 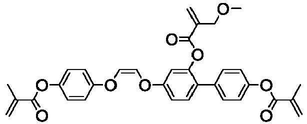 | **1-2-116** - | 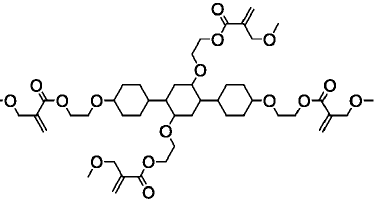 |
| **1-2-110** | 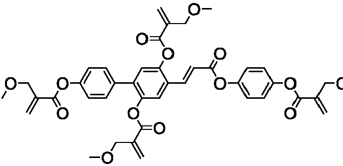 | **1-2-117** | 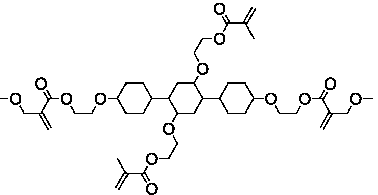 |
| **1-2-111** | 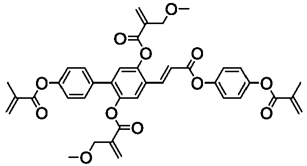 | **1-2-118** | 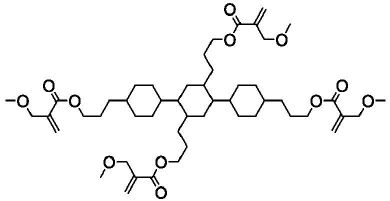 |
| \ **1-3-1** | 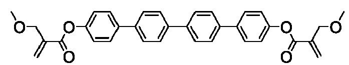 | **1-3-11** | 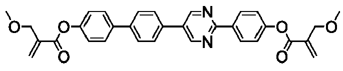 |
| **1-3-2** | 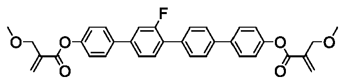 | **1-3-12** | 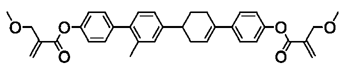 |
| **1-3-3** | 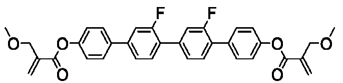 | **1-3-13** | 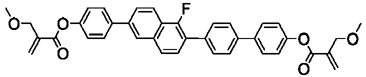 |
| **1-3-4** | 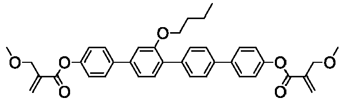 | **1-3-14** | 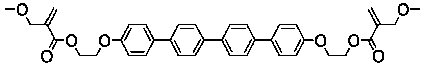 |
| **1-3-5 \ I** | 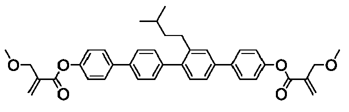 | **1-3-15** | 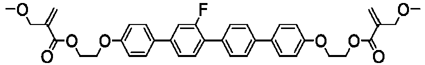 |
| **1-3-6** | 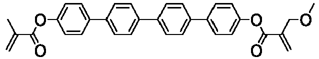 | **1-3-16** | 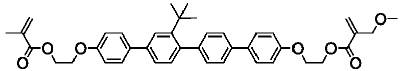 |
| **1-3-7** | 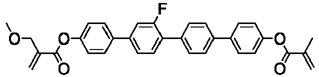 | **1-3-17** | 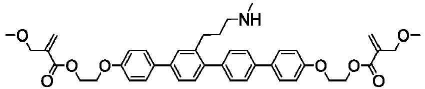 |
| **1-3-8** | 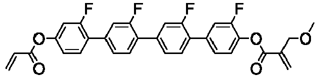 | **1-3-18** | 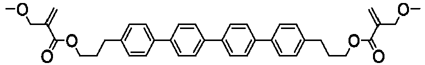 |
| **1-3-9** | 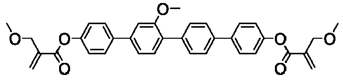 | **1-3-19** | 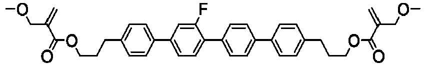 |
| **1-3-10** | 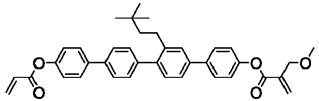 | **1-3-20** | 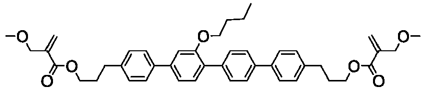 |
| **1-3-21** | 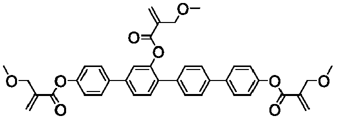 | **1-3-29** | 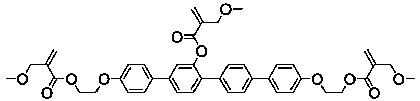 |
| **1-3-22** | 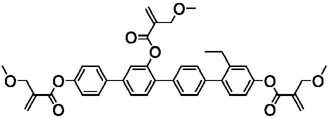 | **1-3-30** | 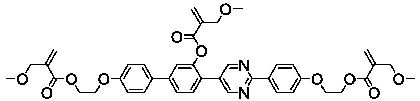 |
| **1-3-23** | 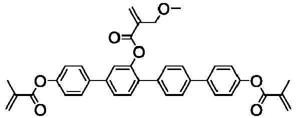 | **1-3-31** | 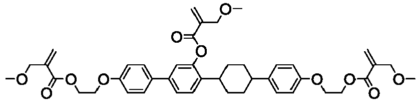 |
| **1-3-24** | 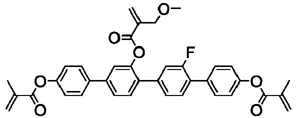 | **1-3-32** | 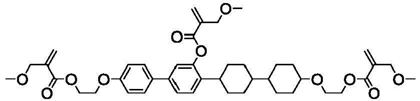 |
| **1-3-25** | 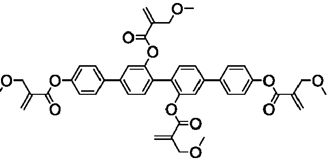 | **1-3-33** | 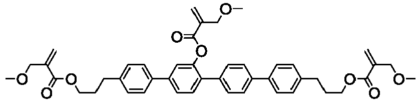 |
| **1-3-26** | 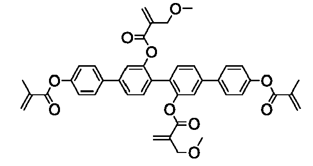 | **1-3-34** | 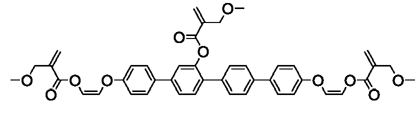 |
| **1-3-27** | 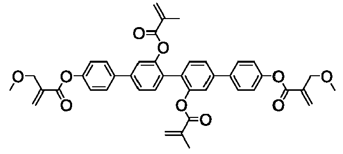 | **1-3-35** | 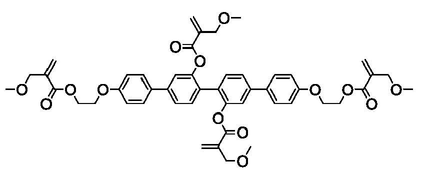 |
| **1-3-28** | 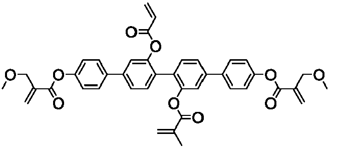 | **1-3-36** | 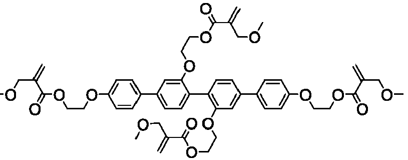 |

### 2. Examples of composition

The invention will be described in greater detail by way of Examples . The Examples include a typical example, and therefore the invention is not limited by the Examples. For example, in addition to compositions in Use Examples, the invention includes a mixture of a composition in Use Example 1 and a composition in Use Example 2. The invention also includes a mixture prepared by mixing at least two compositions in Use Examples. The compounds in Use Examples were represented using symbols according to definitions in Table 3 described below. In Table 3, a configuration of 1,4-cyclohexylene is trans. A parenthesized number next to a symbolized compound in Use Examples represents a chemical formula to which the compound belongs. A symbol (-) means a liquid crystal compound different from compounds (1) to (15). A proportion (percentage) of the liquid crystal compound is expressed in terms of weight percent (% by weight) based on the weight of the liquid crystal composition containing no additive. Values of physical properties of the composition are summarized in a last part. The physical properties were measured according to the methods described above, and measured values are directly described (without extrapolation).

**Table 2 Methods for descriptions of compounds using symbols**

| R-(A₁)-Z₁-.....-Zₙ-(Aₙ)-R' | | | |
|---|---|---|---|
| 1) Left-termirial group R- | Symbol | 4) Ri ng structure -Aₙ- | Symbol |
| CₙH₂ₙ₊₁⁻ | n- | 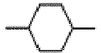 | H |
| CₙH₂ₙ₊₁O- | nO- | | |
| CₘH₂ₘ₊₁OCₙH₂ₙ- | mOn- | 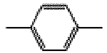 | B |
| CH₂=CH- | V- | | |
| CₙH₂ₙ₊₁-CH=CH- | nV- | | |
| CH₂=CH-CₙH₂ₙ- | Vn- | 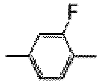 | B(F) |
| CₘH₂ₘ₊₁-CH=CH-CₙH₂ₙ- | mVn- | | |
| CF₂=CH- | VFF- | | |
| CF₂=CH-CₙH₂ₙ- | VFFn- | 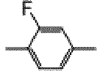 | B(2F) |
| 2) Right-terminal group -R' | Symbol | | |
| -CₙH₂ₙ₊₁ | -n | 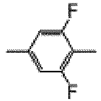 | B(F,F) |
| -OCₙH₂ₙ₊₁ | -On | | |
| -COOCH₃ | -EMe | | |
| -CH=CH₂ | -V | | |
| -CH=CH-CₙH₂ₙ₊₁ | -Vn | 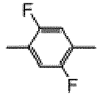 | B(2F,5F) |
| -CₙH₂ₙ-CH=CH₂ | -nV | | |
| -CₘH₂ₘ-CH=CH-CₙH₂ₙ₊₁ | -mVn | | |
| -CH=CF₂ | -VFF | 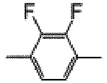 | B(2F,3F) |
| -F | -F | | |
| -Cl | -CL | | |
| -OCF₃ | -OCF3 | 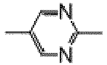 | Py |
| -OCF₂H | -OCF2H | | |
| -CF₃ | -CF3 | | |
| -OCH=CH-CF₃ -C≡N | -OVCF3 -C | 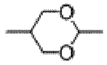 | G |
| 3) Bonding group -Zₙ- | Symbol | | |
| -CₙH₂ₙ- | n | 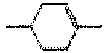 | ch |
| -COO- | E | | |
| -CH=CH- | V | | |
| -CH₂O- | 1O | | |
| -OCH₂- | O1 | | |
| -CF₂O- | X | | |
| -C≡C- | T | | |
| 5) Examples of description | | | |
| Example 1 3-HB-CL | | Example 2 5-HHBB(F,F)-F | |
| 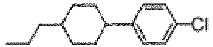 | | 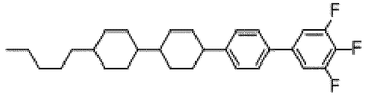 | |
| Example 3 3-HB-O2 | | Example 4 3-HBB(F,F)-F | |
| 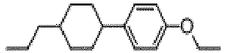 | | 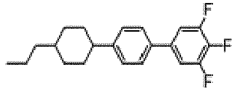 | |

### Use Example 1

| | | |
|---|---|---|
| 1-BB-3 | (2-8) | 6% |
| 1-BB-5 | (2-8) | 8% |
| 2-BTB-1 | (2-10) | 3% |
| 3-HHB-1 | (3-1) | 8% |
| 3-HHB-O1 | (3-1) | 5% |
| 3-HHB-3 | (3-1) | 14% |
| 3-HHB-F | (6-1) | 4% |
| 2-HHB(F)-F | (6-2) | 7% |
| 3-HHB(F)-F | (6-2) | 7% |
| 5-HHB(F)-F | (6-2) | 7% |
| 3-HHB(F,F)-F | (6-3) | 5% |
| 3-HHEB-F | (6-10) | 4% |
| 5-HHEB-F | (6-10) | 4% |
| 2-HB-C | (8-1) | 6% |
| 3-HB-C | (8-1) | 12% |

Compound (1-1-14) was added to the composition described above at a proportion of 0.3% by weight.

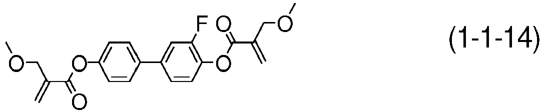

NI = 96.0°C; η = 17.2 mPa ·s; Δn = 0.108; Δε = 4.9.

### Use Example 2

| | | |
|---|---|---|
| 3-HH-4 | (2-1) | 14% |
| 7-HB-1 | (2-5) | 3% |
| 5-HB-O2 | (2-5) | 5% |
| 5-HBB(F)B-2 | (4-5) | 5% |
| 5-HBB(F)B-3 | (4-5) | 5% |
| 3-HB-CL | (5-2) | 13% |
| 3-HHB(F,F)-F | (6-3) | 3% |
| 3-HBB(F,F)-F | (6-24) | 28% |
| 5-HBB(F,F)-F | (6-24) | 24% |

Compound (1-2-41) was added to the composition described above at a proportion of 0.3% by weight.

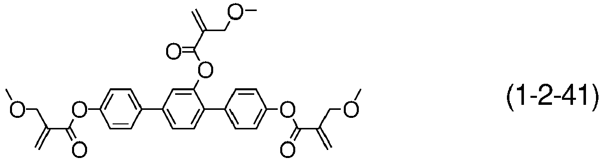

NI = 70.4°C; η = 18.4 mPa ·s; Δn = 0.112; Δε = 5.5.

### Use Example 3

| | | |
|---|---|---|
| 1V2-HH-1 | (2-1) | 3% |
| 1V2-HH-3 | (2-1) | 4% |
| 7-HB (F,F)-F | (5-4) | 3% |
| 2-HHB (F) -F | (6-2) | 9% |
| 3-HHB(F)-F | (6-2) | 10% |
| 5-HHB(F)-F | (6-2) | 10% |
| 2-HBB-F | (6-22) | 4% |
| 3-HBB-F | (6-22) | 3% |
| 5-HBB-F | (6-22) | 3% |
| 2-HBB(F)-F | (6-23) | 9% |
| 3-HBB(F)-F | (6-23) | 9% |
| 5-HBB(F)-F | (6-23) | 16% |
| 3-HBB(F,F)-F | (6-24) | 6% |
| 5-HBB (F,F)-F | (6-24) | 11% |

Compound (1-2-47) was added to the composition described above at a proportion of 0.2% by weight.

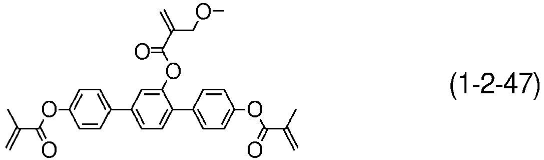

NI = 84.0°C; η = 25.4 mPa ·s; Δn = 0.111; Δε = 5.8.

### Use Example 4

| | | |
|---|---|---|
| 2-HH-3 | (2-1) | 5% |
| 3-HH-4 | (2-1) | 12% |
| 1O1-HBBH-5 | (4-1) | 3% |
| 5-HB-CL | (5-2) | 15% |
| 3-HHB-F | (6-1) | 4% |
| 3-HHB-CL | (6-1) | 4% |
| 4-HHB-CL | (6-1) | 4% |
| 3-HHB (F)-F | (6-2) | 9% |
| 4-HHB(F)-F | (6-2) | 9% |
| 5-HHB(F)-F | (6-2) | 10% |
| 7-HHB(F)-F | (6-2) | 8% |
| 5-HBB(F)-F | (6-23) | 3% |
| 3-HHBB(F,F)-F | (7-6) | 2% |
| 4-HHBB(F,F)-F | (7-6) | 3% |
| 5-HHBB(F,F)-F | (7-6) | 3% |
| 3-HH2BB(F,F)-F | (7-15) | 3% |
| 4-HH2BB (F,F) -F | (7-15) | 3% |

Compound (1-1-35) was added to the composition described above at a proportion of 0.3% by weight.

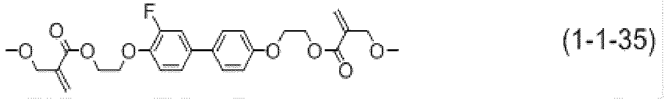

NI = 113.3°C; η = 17.8 mPa ·s; Δn = 0.089; Δε = 3.6.

### Use Example 5

| | | |
|---|---|---|
| V-HBB-2 | (3-4) | 10% |
| 1O1-HBBH-4 | (4-1) | 5% |
| 1O1-HBBH-5 | (4-1) | 3% |
| 3-HHB(F,F)-F | (6-3) | 8% |
| 3-H2HB(F,F)-F | (6-15) | 8% |
| 4-H2HB(F,F)-F | (6-15) | 8% |
| 5-H2HB(F,F)-F | (6-15) | 9% |
| 3-HBB(F,F)-F | (6-24) | 11% |
| 5-HBB (F,F)-F | (6-24) | 18% |
| 3-H2BB(F,F)-F | (6-27) | 12% |
| 5-HHBB(F,F)-F | (7-6) | 3% |
| 3-HH2BB(F,F)-F | (7-15) | 3% |
| 5-HHEBB-F | (7-17) | 2% |

Compound (1-1-46) was added to the composition described above at a proportion of 0.4% by weight.

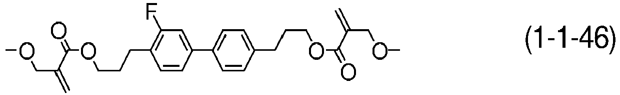

NI = 106.5°C; η = 32.4 mPa ·s; Δn = 0.122; Δε = 8.2.

### Use Example 6

| | | |
|---|---|---|
| 5-HBBH-3 | (4-1) | 3% |
| 3-HB(F)BH-3 | (4-2) | 3% |
| 5-HB-F | (5-2) | 12% |
| 6-HB-F | (5-2) | 9% |
| 7-HB-F | (5-2) | 7% |
| 2-HHB-OCF3 | (6-1) | 8% |
| 3-HHB-OCF3 | (6-1) | 6% |
| 4-HHB-OCF3 | (6-1) | 7% |
| 5-HHB-OCF3 | (6-1) | 6% |
| 3-HHB (F,F)-OCF2H | (6-3) | 3% |
| 3-HHB(F,F)-OCF3 | (6-3) | 4% |
| 3-HH2B-OCF3 | (6-4) | 5% |
| 5-HH2B-OCF3 | (6-4) | 4% |
| 3-HH2B(F)-F | (6-5) | 3% |
| 3-HBB(F)-F | (6-23) | 10% |
| 5-HBB(F)-F | (6-23) | 10% |

Compound (1-2-60) was added to the composition described above at a proportion of 0.3% by weight.

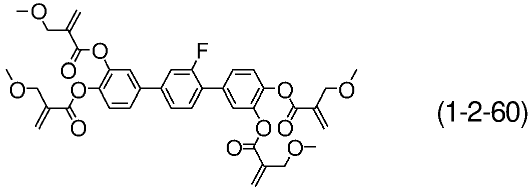

NI = 85.8°C; η = 14.3 mPa ·s; Δn = 0.092; Δε = 4.4.

### Use Example 7

| | | |
|---|---|---|
| 3-HH-4 | (2-1) | 4% |
| 2-HH-5 | (2-1) | 6% |
| 5-B(F)BB-2 | (3-8) | 5% |
| 5-HB-CL | (5-2) | 9% |
| 3-HHB(F,F)-F | (6-3) | 8% |
| 3-HHEB(F,F)-F | (6-12) | 9% |
| 4-HHEB(F,F)-F | (6-12) | 4% |
| 5-HHEB(F,F)-F | (6-12) | 3% |
| 3-HBB(F,F)-F | (6-24) | 19% |
| 5-HBB (F,F)-F | (6-24) | 16% |
| 2-HBEB(F,F)-F | (6-39) | 3% |
| 3-HBEB(F,F)-F | (6-39) | 5% |
| 5-HBEB(F,F)-F | (6-39) | 4% |
| 3-HHBB(F,F)-F | (7-6) | 5% |

Compound (1-1-1) was added to the composition described above at a proportion of 0.3% by weight.

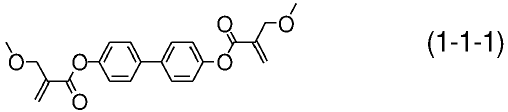

NI = 77.3°C; η = 22.9 mPa ·s; Δn = 0.109; Δε = 8.5.

### Use Example 8

| | | |
|---|---|---|
| V2-HHB-1 | (3-1) | 6% |
| 3-HB-CL | (5-2) | 5% |
| 5-HB-CL | (5-2) | 4% |
| 3-HHB-OCF3 | (6-1) | 7% |
| V-HHB (F) -F | (6-2) | 3% |
| 5-HHB(F)-F | (6-2) | 5% |
| 3-H2HB-OCF3 | (6-13) | 5% |
| 5-H2HB(F,F)-F | (6-15) | 5% |
| 5-H4HB-OCF3 | (6-19) | 15% |
| 3-H4HB(F,F)-CF3 | (6-21) | 8% |
| 5-H4HB(F,F)-CF3 | (6-21) | 10% |
| 5-H4HB(F,F)-F | (6-21) | 7% |
| 2-H2BB(F)-F | (6-26) | 5% |
| 3-H2BB(F)-F | (6-26) | 8% |
| 3-HBEB(F,F)-F | (6-39) | 7% |

Compound (1-1-10) was added to the composition described above at a proportion of 0.3% by weight.

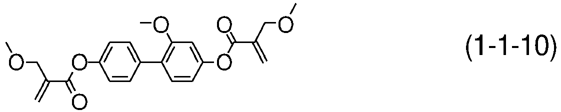

NI = 74.8°C; η = 25.6 mPa ·s; Δn = 0.099; Δε = 8.4.

### Use Example 9

| | | |
|---|---|---|
| 3-HH-4 | (2-1) | 8% |
| 3-HH-5 | (2-1) | 5% |
| 3-HB-O2 | (2-5) | 17% |
| 3-HHB-1 | (3-1) | 8% |
| 3-HHB-O1 | (3-1) | 5% |
| 5-HB-CL | (5-2) | 14% |
| 7-HB(F,F)-F | (5-4) | 3% |
| 2-HHB (F) -F | (6-2) | 7% |
| 3-HHB(F)-F | (6-2) | 6% |
| 5-HHB(F)-F | (6-2) | 6% |
| 3-HHB(F,F)-F | (6-3) | 8% |
| 3-H2HB(F,F)-F | (6-15) | 6% |
| 4-H2HB(F,F)-F | (6-15) | 7% |

Compound (1-2-2) was added to the composition described above at a proportion of 0.3% by weight.

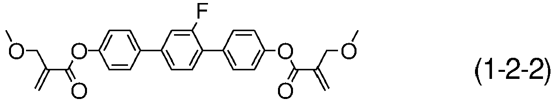

NI = 71.4°C; η = 14.9 mPa ·s; Δn = 0.074; Δε = 3.0.

### Industrial Applicability

A liquid crystal compound of the invention has good physical properties. A liquid crystal composition containing the compound can be widely applied to a liquid crystal display device used in a personal computer, a television and so forth.

## Claims

1. A compound, represented by formula (1): 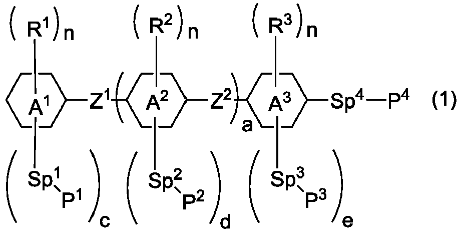 wherein, in formula (1),
R¹, R² and R³ are independently alkyl having 1 to 10 carbons, and in the alkyl, at least one piece of -CH₂- may be replaced by -O- or -NH-;
n is independently 0, 1, 2, 3 or 4;
ring A¹ is cyclohexyl, cyclohexenyl, phenyl, 1-naphthyl, 2-naphthyl, tetrahydropyran-2-yl, tetrahydropyran-3-yl, 1,3-dioxane-2-yl, 1,3-dioxane-3-yl, pyrimidine-2-yl, pyrimidine-5-yl, pyridine-2-yl or pyridine-3-yl, ring A² and ring A³ are independently 1,4-cyclohexylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, tetrahydropyran-2,5-diyl, 1,3-dioxane-2,5-diyl, pyrimidine-2,5-diyl or pyridine-2,5-diyl, and
in the rings, at least one hydrogen may be replaced by fluorine or chlorine;
Z¹ and Z² are independently a single bond or alkylene having 1 to 10 carbons, and in the alkylene, at least one piece of -CH₂- may be replaced by -O-, -COO-, -OCO- or -OCOO-, and at least one piece of -(CH₂)₂- may be replaced by -CH=CH- or -C≡C-, and in the groups, at least one hydrogen may be replaced by fluorine or chlorine;
Sp¹, Sp², Sp³ and Sp⁴ are independently a single bond or alkylene having 1 to 10 carbons, and in the alkylene, at least one piece of -CH₂-may be replaced by -O-, -COO-, -OCO- or -OCOO-, and at least one piece of -(CH₂)₂- may be replaced by -CH=CH- or -C≡C-, and in the groups, at least one hydrogen may be replaced by fluorine or chlorine;
a is 0, 1, 2, 3 or 4;
c, d and e are independently 0, 1, 2, 3 or 4, and a sum of c, d and e is 1, 2, 3 or 4; and
P¹, P², P³ and P⁴ are a polymerizable group, and at least one of P¹, P², P³ and P⁴ is a polymerizable group represented by formula (P-1).

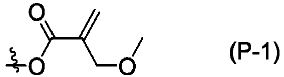

2. The compound according to claim 1,
wherein, in formula (1),
ring A¹ is cyclohexyl, cyclohexenyl, phenyl, 1-naphthyl, 2-naphthyl, tetrahydropyran-2-yl, tetrahydropyran-3-yl, 1,3-dioxane-2-yl or 1,3-dioxane-3-yl, ring A² and ring A³ are independently 1,4-cyclohexylene, 1,4-cyclohexenylene, 1,4-phenylene, naphthalene-1,5-diyl, naphthalene-2,6-diyl, tetrahydropyran-2,5-diyl or 1,3-dioxane-2,5-diyl, and
in the rings, at least one hydrogen may be replaced by fluorine or chlorine.

3. The compound according to claim 1,
wherein, in formula (1),
Sp¹, Sp², Sp³ and Sp⁴ are independently a single bond or alkylene having 1 to 7 carbons, and in the alkylene, at least one piece of -CH₂-may be replaced by -O-, and at least one piece of - (CH₂) ₂- may be replaced by -CH=CH-, and in the groups, at least one hydrogen may be replaced by fluorine or chlorine.

4. The compound according to any one of claims 1 to 3, represented by any one of formula (1-1) to formula (1-3): 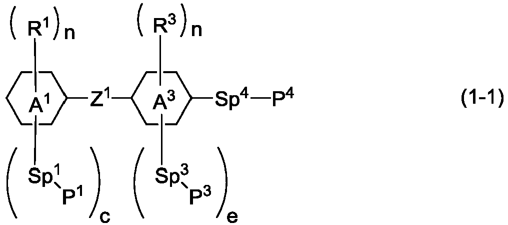 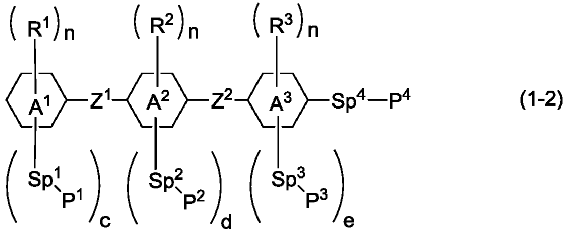 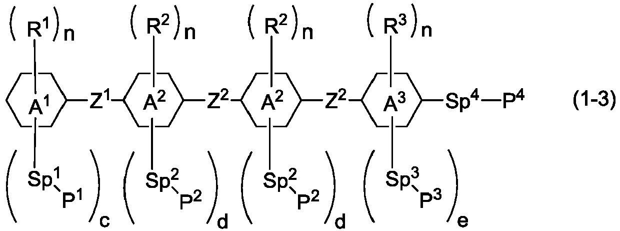 wherein, in formula (1-1) to formula (1-3),
R¹, R² and R³ are independently alkyl having 1 to 7 carbons, and in the alkyl, at least one piece of -CH₂- may be replaced by -O-;
n is independently 0, 1, 2, 3 or 4;
ring A¹ is cyclohexyl, cyclohexenyl, phenyl, 1-naphthyl, 2-naphthyl, tetrahydropyran-2-yl, tetrahydropyran-3-yl, 1,3-dioxane-2-yl or 1,3-dioxane-3-yl, ring A² and ring A³ are independently 1,4-cyclohexylene, 1,4-cyclohexenylene, 1,4-phenylene, naphthalene-1,5-diyl, naphthalene-2,6-diyl, tetrahydropyran-2,5-diyl or 1,3-dioxane-2,5-diyl, and
in the rings, at least one hydrogen may be replaced by fluorine or chlorine;
Z¹ and Z² are independently a single bond, alkylene having 1 to 4 carbons, -CH₂O-, -OCH₂-, -COO-, -OCO-, -CH₂CH₂-, -CH=CH-, -C≡C-, -CH=CH-COO-, -OCO-CH=CH-, -CO-CH=CH- or -CH=CH-CO-;
Sp¹, Sp², Sp³ and Sp⁴ are independently a single bond or alkylene having 1 to 7 carbons, and in the alkylene, at least one piece of -CH₂-may be replaced by -O-, and at least one piece of - (CH₂) ₂- may be replaced by -CH=CH-, and in the groups, at least one hydrogen may be replaced by fluorine or chlorine;
c, d and e are independently 0, 1, 2, 3 or 4, and a sum of c, d and e is 1, 2, 3 or 4; and
P¹, P², P³ and P⁴ are independently a group selected from the group of polymerizable groups represented by formula (P-1)to formula (P-6), and at least one of P¹, P², P³ and P⁴ is a polymerizable group represented by formula (P-1); 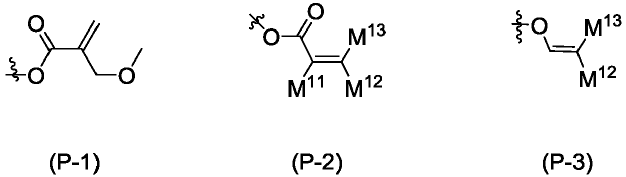 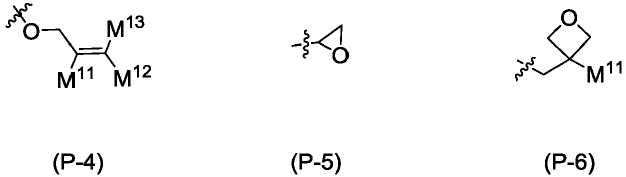 wherein, in formula (P-2) to formula (P-6), M¹¹, M¹² and M¹³ are independently hydrogen, fluorine, alkyl having 1 to 5 carbons, or alkyl having 1 to 5 carbons in which at least one hydrogen is replaced by halogen.

5. The compound according to any one of claims 1 to 4, wherein, in formula (1-1) to formula (1-3),
ring A¹ is cyclohexyl or phenyl, ring A² and ring A³ are independently 1,4-cyclohexylene or 1,4-phenylene, and
in the rings, at least one hydrogen may be replaced by fluorine or chlorine.

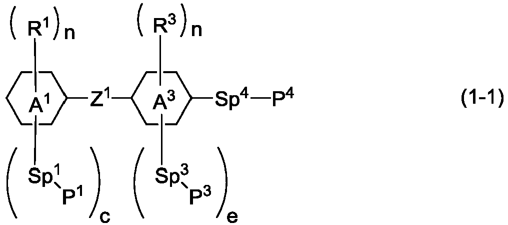

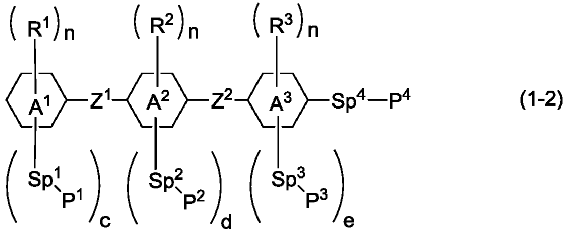

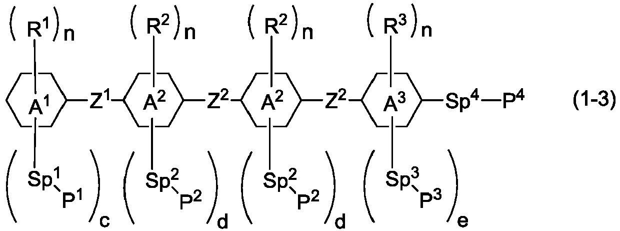

6. The compound according to any one of claims 1 to 5, represented by any one of formula (1-4) to formula (1-12): 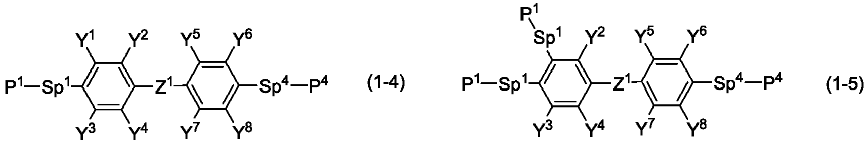 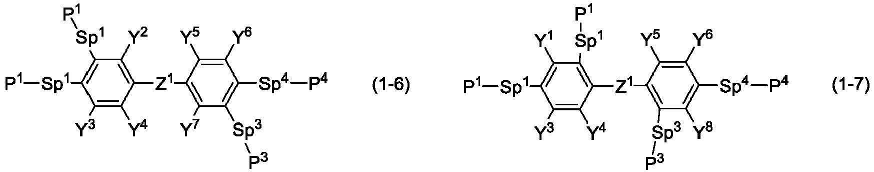 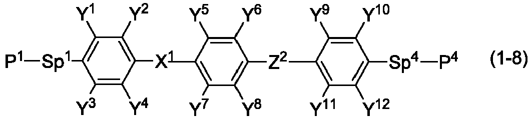 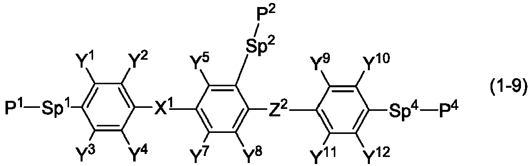 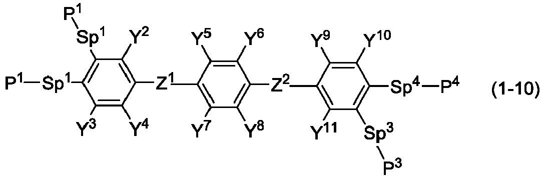 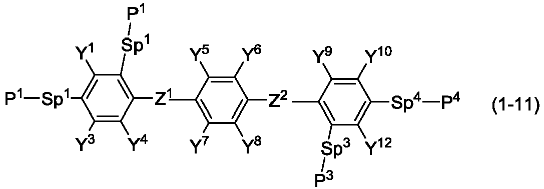 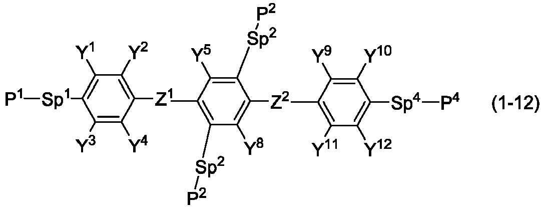 wherein, in formula (1-4) to formula (1-12),
Y¹, Y², Y³, Y⁴, Y⁵, Y⁶, Y⁷, Y⁸, Y⁹, Y¹⁰, Y¹¹ and Y¹² are independently hydrogen, fluorine, methyl, ethyl, propyl, butyl, methoxy, ethoxy, propyloxy or butoxy;
Sp¹, Sp², Sp³ and Sp⁴ are independently a single bond or alkylene having 1 to 5 carbons, and in the alkylene, at least one piece of -CH₂-may be replaced by -O-, and at least one piece of - (CH₂) ₂- may be replaced by -CH=CH-;
Z¹ and Z² are independently a single bond, alkylene having 1 to 4 carbons, -CH₂O-, -OCH₂-, -COO-, -OCO-, -CH₂CH₂-, -CH=CH-, -CH=CH-COO- or -OCO-CH=CH-; and
P¹, P², P³ and P⁴ are independently a group selected from the group of polymerizable groups represented by formula (P-1) to formula (P-4), and at least one of P¹, P², P³ and P⁴ is a polymerizable group represented by formula (P-1); 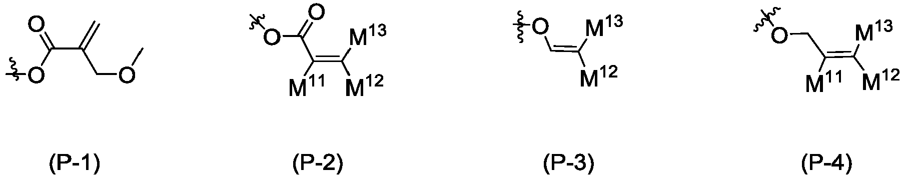 wherein, in formula (P-2) to formula (P-4), M¹¹, M¹² and M¹³ are independently hydrogen, fluorine, alkyl having 1 to 5 carbons, or alkyl having 1 to 5 carbons in which at least one hydrogen is replaced by halogen.

7. The compound according to any one of claims 1 to 6, represented by any one of formula (1-13) to formula (1-24): 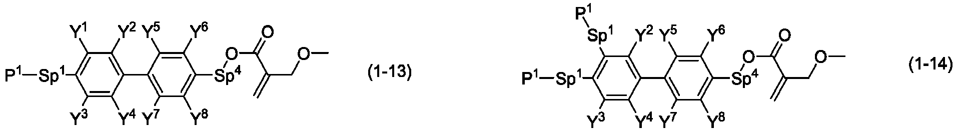 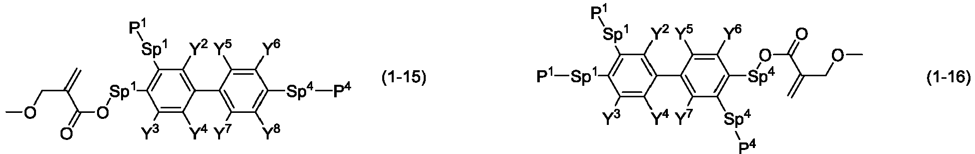 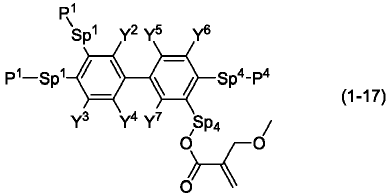 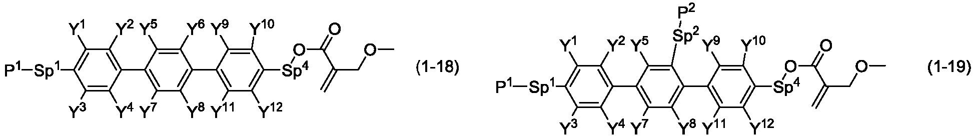 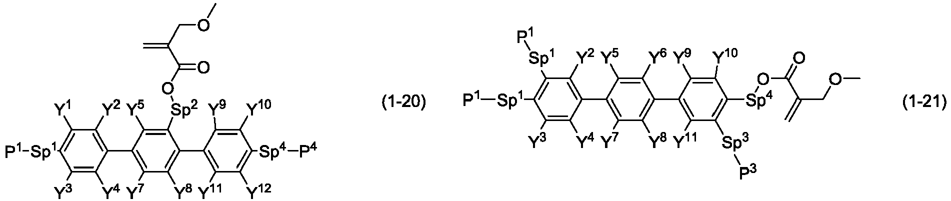 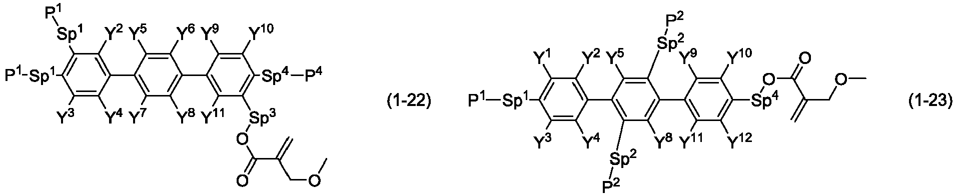 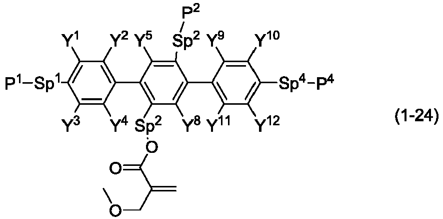 wherein, in formula (1-13) to formula (1-24),
Y¹, Y², Y³, Y⁴, Y⁵, Y⁶, Y⁷, Y⁸, Y⁹, Y¹⁰, Y¹¹ and Y¹² are independently hydrogen, fluorine, methyl, ethyl, propyl, butyl, methoxy, ethoxy, propyloxy or butoxy;
Sp¹, Sp², Sp³ and Sp⁴ are independently a single bond or alkylene having 1 to 5 carbons, and in the alkylene, at least one piece of -CH₂-may be replaced by -O-, and at least one piece of - (CH₂) ₂- may be replaced by -CH=CH-; and
P¹, P², P³ and P⁴ are independently acryloyloxy, methacryloyloxy, or a polymerizable group represented by formula (P-1).

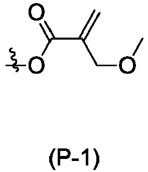

8. A liquid crystal composition, containing at least one compound according to any one of claims 1 to 7 as component A.

9. The liquid crystal composition according to claim 8, further containing at least one compound selected from the group of compounds represented by formulas (2) to (4) as component B: 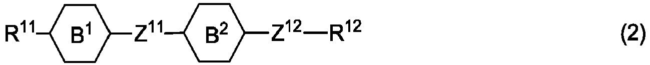 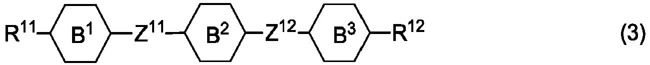 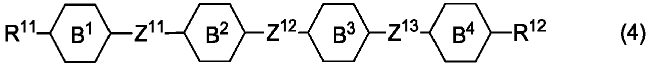 wherein, in formula (2) to formula (4),
R¹¹ and R¹² are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
ring B¹, ring B², ring B³ and ring B⁴ are independently 1,4-cyclohexylene, 1,4-phenylene, 2-fluoro-1,4-phenylene, 2,5-difluoro-1,4-phenylene or pyrimidine-2,5-diyl; and
Z¹¹, Z¹² and Z¹³ are independently a single bond, -COO-, -CH₂CH₂-, -CH=CH- or -C≡C-.

10. The liquid crystal composition according to claim 8 or 9, further containing at least one compound selected from the group of compounds represented by formulas (5) to (7) as component C: 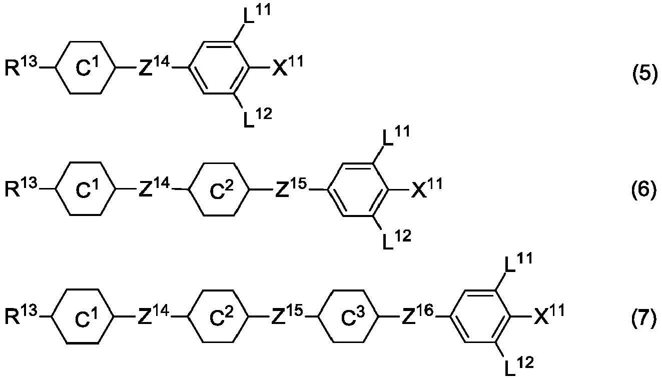 wherein, in formula (5) to formula (7),
R¹³ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
X¹¹ is fluorine, chlorine, -OCF₃, -OCHF₂, -CF₃, -CHF₂, -CH₂F, -OCF₂CHF₂ or -OCF₂CHFCF₃;
ring C¹, ring C² and ring C³ are independently 1, 4-cyclohexylene, 1, 4-phenylene, 1, 4-phenylene in which at least one hydrogen is replaced by fluorine, tetrahydropyran-2,5-diyl, 1,3-dioxane-2,5-diyl or pyrimidine-2,5-diyl;
Z¹⁴, Z¹⁵ and Z¹⁶ are independently a single bond, -CH₂CH₂-, -CH=CH-, -C≡C-, -COO-, -CH₂O- or -(CH₂)₄-; and
L¹¹ and L¹² are independently hydrogen or fluorine.

11. The liquid crystal composition according to any one of claims 8 to 10, further containing at least one compound selected from the group of compounds represented by formula (8) as component D: 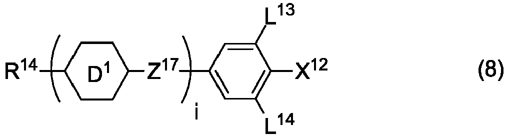 wherein, in formula (8),
R¹⁴ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
X¹² is -C≡N or -C≡C-C≡N;
ring D¹ is 1,4-cyclohexylene, 1,4-phenylene, 1,4-phenylene in which at least one hydrogen is replaced by fluorine, tetrahydropyran-2,5-diyl, 1,3-dioxane-2,5-diyl or pyrimidine-2,5-diyl;
Z¹⁷ is a single bond, -CH₂CH₂-, -C≡C-, -COO- or -CH₂O-;
L¹³ and L¹⁴ are independently hydrogen or fluorine; and
i is 1, 2, 3 or 4.

12. The liquid crystal composition according to any one of claims 8 to 11, further containing at least one compound selected from the group of polymerizable compounds represented by formula (16) as component F: 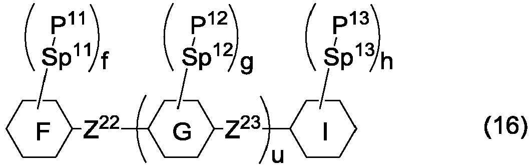 wherein, in formula (16),
ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
ring G is 1,4-cyclohexylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, phenanthrene-2,7-diyl, tetrahydropyran-2,5-diyl, 1,3-dioxane-2,5-diyl, pyrimidine-2,5-diyl or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
Z²² and Z²³ are independently a single bond or alkylene having 1 to 10 carbons, and in the alkylene, at least one piece of -CH₂- may be replaced by -O-, -CO-, -COO- or -OCO-, and at least one piece of -CH₂CH₂- may be replaced by -CH=CH-, -C(CH₃)=CH-, -CH=C(CH₃)- or -C(CH₃) =C(CH₃)-, and in the groups, at least one hydrogen may be replaced by fluorine or chlorine;
Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine;
u is 0, 1 or 2;
f, g and h are independently 0, 1, 2, 3 or 4, and a sum of f, g and h is 1 or more; and
P¹¹, P¹² and P¹³ are independently a group selected from the group of polymerizable groups represented by formula (P-2) to formula (P-6) ; 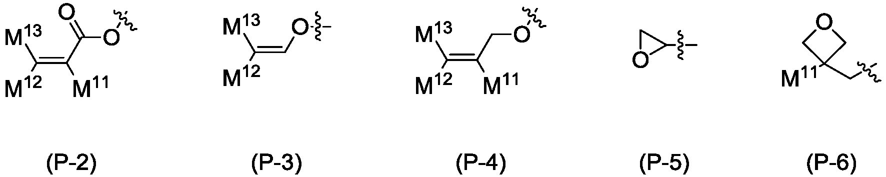 wherein, in formula (P-2) to formula (P-6), M¹¹, M¹² and M¹³ are independently hydrogen, fluorine, alkyl having 1 to 5 carbons, or alkyl having 1 to 5 carbons in which at least one hydrogen is replaced by halogen.

13. The liquid crystal composition according to claim 12, wherein component F is at least one compound selected from the group of polymerizable compounds represented by formula (16-1) to formula (16-7) : 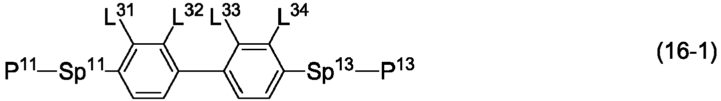 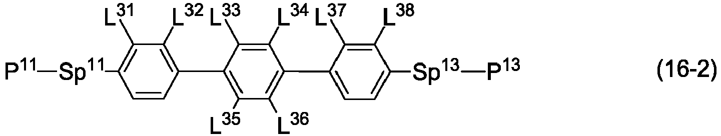 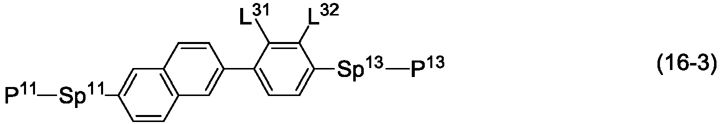 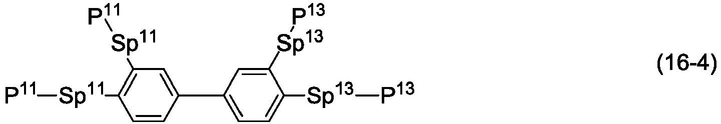 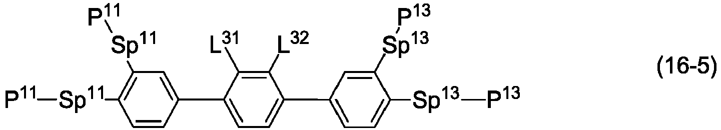 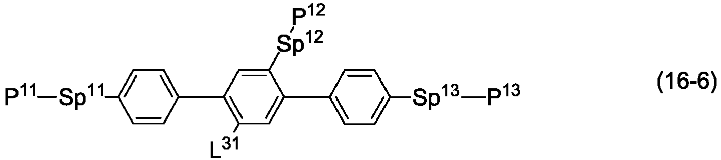 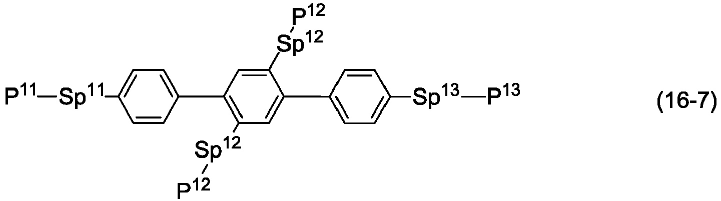 wherein, in formula (16-1) to formula (16-7), P¹¹, P¹² and P¹³ are independently a group selected from the group of polymerizable groups represented by formula (P-2) to formula (P-4), in which M¹¹, M¹² and M¹³ are independently hydrogen, fluorine, alkyl having 1 to 5 carbons, or alkyl having 1 to 5 carbons in which at least one hydrogen is replaced by halogen; 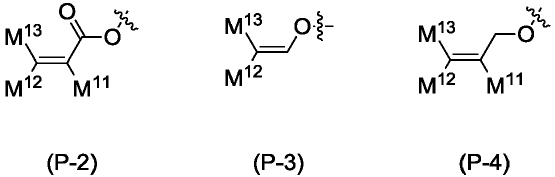 wherein, L³¹, L³², L³³, L³⁴, L³⁵, L³⁶, L³⁷ and L³⁸ are independently hydrogen, fluorine or methyl; and Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine.

14. The liquid crystal composition according to any one of claims 8 to 13, further containing at least one of polymerizable compounds different from the compounds represented by formula (1) and formula (16), a polymerization initiator, a polymerization inhibitor, an optically active compound, an antioxidant, an ultraviolet light absorber, a light stabilizer, a heat stabilizer and an antifoaming agent.

15. A liquid crystal display device, including at least one liquid crystal composition according to any one of claims 8 to 14.
